# EUROPEAN PATENT SPECIFICATION

(11) **EP 2 797 417 B1**
(45) Date of publication and mention of the grant of the patent: **31.07.2019**
(21) Application number: 12813362.6
(22) Date of filing: 21.12.2012
(51) Int. Cl.: A01N 37/40, A01N 43/16, A01N 43/42, A01N 37/46, A01N 43/60, A01N 43/82, A01N 43/90, A01N 45/00

(54) **SELECTIVE INHIBITION OF PHOSPHOENOLPYRUVATE CARBOXYLASES OF C4 PLANTS**
SELEKTIVE HEMMUNG VON PHOSPHOENOLPYRUVAT-CARBOXYLASEN VON C4 PFLANZEN
INHIBITION SÉLECTIVE DES CARBOXYLASES DU PHOSPHOÉNOLPYRUVATE DES PLANTES C4

(30) Priority: 22.12.2011 EP 11195367
(43) Date of publication of application: 05.11.2014
(73) Proprietor: Heinrich Heine Universität Düsseldorf, 40225 Düsseldorf (DE)
(72) Inventor: GROTH, Georg, 52428 Jülich (DE); PAULUS, Judith Katharina, 40223 Düsseldorf (DE); SCHLIEPER, Daniel, 40219 Düsseldorf (DE); WESTHOFF, Peter, 41468 Neuss (DE)
(74) Representative: Herzog, Fiesser & Partner Patentanwälte PartG mbB
(86) International application number: PCT/EP2012/076648
(87) International publication number: WO 2013/093007

(56) References cited:
- WO-A1-99/54495
- JENKINS C L D: "EFFECTS OF THE PHOSPHOENOLPYRUVATE CARBOXYLASE INHIBITOR 3,3-DICHLORO-2-(DIHYDROXYPHOSPHINOYLMETHYL )PROPENOATE IN PHOTOSYNTHESIS", PLANT PHYSIOLOGY, AMERICAN SOCIETY OF PLANT PHYSIOLOGISTS, ROCKVILLE, MD, US, vol. 89, 1 January 1989 (1989-01-01), pages 1231-1237, XP001153349, ISSN: 0032-0889
- R Harold Brown ET AL: "Assessing the Degree of C4 Photosynthesis in C3-C4 Species Using an Inhibitor of Phosphoenolpyruvate Carboxylase", Plant Physiol., 1 January 1991 (1991-01-01), pages 985-989, XP055025771, Retrieved from the Internet: URL:http://www.plantphysiol.org/content/97 /3/985.full.pdf [retrieved on 2012-04-26]
- WANG ET AL: "Identification of some novel AHAS inhibitors via molecular docking and virtual screening approach", BIOORGANIC & MEDICINAL CHEMISTRY, PERGAMON, GB, vol. 15, no. 1, 15 November 2006 (2006-11-15), pages 374-380, XP005764619, ISSN: 0968-0896, DOI: 10.1016/J.BMC.2006.09.050
- HU D-J ET AL: "Synthesis and herbicidal activities of novel 4-(4-(5-methyl-3-arylisoxazol-4-yl)thiazol -2-yl)piperidyl carboxamides and thiocarboxamides", MOLECULES, MOLECULAR DIVERSITY PRESERVATION INTERNATIONAL, BASEL, CH, vol. 14, no. 3, 24 March 2009 (2009-03-24) , pages 1288-1303, XP002609686, ISSN: 1420-3049, DOI: 10.3390/MOLECULES14031288
- MIRIAM LOPEZ-RAMOS ET AL: "HPPD: Ligand- and Target-Based Virtual Screening on a Herbicide Target", JOURNAL OF CHEMICAL INFORMATION AND MODELING, vol. 50, no. 5, 24 May 2010 (2010-05-24), pages 801-814, XP055025817, ISSN: 1549-9596, DOI: 10.1021/ci900498n
- MANCERA ET AL: "The molecular binding interactions of inhibitors and activators of phosphoenolpyruvate carboxylase", JOURNAL OF MOLECULAR STRUCTURE (THEOCHEM), ELSEVIER SCIENCE PUBLISHERS B.V., AMSTERDAM, NL, vol. 755, no. 1-3, 30 November 2005 (2005-11-30), pages 151-159, XP005184748, ISSN: 0166-1280, DOI: 10.1016/J.THEOCHEM.2005.08.014
- Pierre Rustin ET AL: "Fluorescence Study of Chemical Modification of Phosphoenolpyruvate Carboxylase from Crassula argentea", Plant Physiol., 1 January 1991 (1991-01-01), pages 1011-1016, XP55056100, Retrieved from the Internet: URL:http://www.ncbi.nlm.nih.gov/pmc/articl es/PMC1081117/pdf/plntphys00698-0171.pdf [retrieved on 2013-03-12]
- CLAUDE CRÉTIN ET AL: "Differential inhibition of phosphoenol-pyruvate carboxylases by 2,4-dichlorophenoxyacetic acid and two newly synthesized herbicides", PHYTOCHEMISTRY, vol. 22, no. 12, 1 January 1983 (1983-01-01), pages 2661-2664, XP055025854, ISSN: 0031-9422, DOI: 10.1016/S0031-9422(00)97668-4 -& CRETIN C ET AL: "Study on plant phosphoenolpyruvate carboxylases: sensitivity to herbicides and immunochemical reactivity", PHYSIOLOGIE VEGETALE, UTHIER-VILLARS, PARIS, GA, vol. 21, 1 January 1983 (1983-01-01), pages 927-933, XP009158904, ISSN: 0031-9368
- RAO M ET AL: "HERBRICIDE-INHIBITED PHOSPHOENOLPYRUVATE CARBOXYLASE IN LEAVES OF SIX NONSUCCULENT SCRUB SPECIES", ZEITSCHRIFT FUER PFLANZENPHYSIOLOGIE, STUTTGART, DE, vol. 99, no. 1, 1 January 1980 (1980-01-01), pages 69-74, XP009013516, ISSN: 0044-328X
- SASCHA ENGELMANN ET AL: "Molecular evolution of C4 phosphoenolpyruvate carboxylase in the genus Flaveria?a gradual increase from C3 to C4 characteristics", PLANTA, vol. 217, no. 5, 1 September 2003 (2003-09-01), pages 717-725, XP55025746, ISSN: 0032-0935, DOI: 10.1007/s00425-003-1045-0
- PAULUS JUDITH KATHARINA ET AL: "Direct and selective small-molecule inhibition of photosynthetic PEP carboxylase: New approach to combat C4weeds in arable crops", FEBS LETTERS, vol. 588, no. 12, 8 May 2014 (2014-05-08), pages 2101-2106, XP029032401, ISSN: 0014-5793, DOI: 10.1016/J.FEBSLET.2014.04.043
- "Hydrilla: retrofitting a C3 leaf with a single cell C4 NADP-ME system - Phosphoenolpyruvate carboxylase" In: "Charting New Pathways to C4 Rice", 1 January 2007 (2007-01-01), International Rice Research Institute (IRRI), Philippines, XP55224542, ISBN: 978-971-220-216-2 pages 283-285,

## Description

In the past several different targets for selective and non-selective herbicides were identified. However, there still is a great need for new herbicides, especially selective herbicides. In particular specific herbicides against C4 plants would be of outmost importance for the agribusiness and a major turning point in the control of the worst weeds in the world. These weeds reduce the crop yield of the most important C3 cereals such as rice, wheat, barley and oat.

The C4 metabolism offers three potential targets for a selective herbicide. The first target is the enzyme phosphoenol pyruvate (PEP) carboxylase, which has a role in CO₂ fixation. The second target is the malic enzyme (ME) or the enzyme PEP carboxykinase (PEP CK) which catalyse the release of carbon dioxide in the bundle sheath cells. The third target is the enzyme pyruvate phosphate dikinase (PPDK), which regenerates PEP.

The substances unguinol and ilimaquinone were identified as selective inhibitors of PPDK from C4 plants maize, millet and proso millet. These substances were isolated from marine organisms (Motti *et al.,* 2007). The only universal target, that is common to all variants of C4 metabolism, is the enzyme PEP carboxylase. Due to the high sequence homology between the C4 specific PEP carboxylase and the C3 isoforms, which have further roles in the general C3 metabolism in plants and other organism, it is not obvious to use this enzyme as selective target.

The PEP carboxylase inhibitors described so far (Jenkins *et al*., 1987; Jenkins, 1989; McFadden *et al.,* 1989; Mancera *et al.,* 1995) are based on PEP analogues (e. g., 3,3-Dichloro-2-(dihydroxyphosphinoylmethyl) propenoate (DCDP)). However, these compounds also inhibit C3 type PEP carboxylases at rates between 12-46% (Jenkins, 1989) and do not inhibit growth of C4 plants (Doyle et al., 2005). Pairoba *et al.* (1996) showed that PEP carboxylase from C4 plant *Amaranthus viridis* is inhibited by several flavonoles and flavones. The inhibiting flavonoles are quercetin, quercitrin, rutin, kaempferol, fisetin, morin, and myricetin. The inhibiting flavone was baicalein/baicalin.

But in that work, neither the binding site of the inhibiting flavonoles (or flavones, resp.), nor the molecular mechanism of inhibition were identified. Thus, the mechanism of the inhibiting flavonoles and flavones is unknown. Furthermore, the substances were also described as inhibitors of the malic enzyme from *A. viridis,* a fact that implies a fairly unspecific inhibition. Furthermore, the authors failed to show that the tested flavonoles and flavones inhibit C4 type PEP carboxylases only, but not C3 type isoforms.

The crystal structures of PEP carboxylase from *Escherichia coli* (C3 type) and from the C4 plant maize are known (Matsumura *et al.,* 2002). In the publications of these structures, several amino acid side chains were identified as important for the binding of the feedback inhibitors malate and aspartate. However, the authors failed to note the structural difference of the malate binding site that we use in the development of C4 selective herbicides.

Brown discloses the use of 3,3-dichloro-2-(dihydroxyphosphinoylmethyl) propenoate (PEPC inhibitor) in the inhibition of apparent photosynthesis of C3 and C4 plants. Inhibition of the photosynthesis was 72% for the C4 plant Flaveria trinervia, and 14% for the C3 plant Flaveria pringlei (Brown RH, Byrd GT, Black CC. Assessing the Degree of C4 Photosynthesis in C3-C4 Species Using an Inhibitor of Phosphoenolpyruvate Carboxylase . Plant Physiology. 1991;97(3):985-989).

WO 99/54495 discloses a screening assay system to identify inhibitors of the C4 acid cycle in plants. The assay involves incubating compounds with PEPC and determining the inhibition of the enzyme in a malate dehydrogenase coupled system via the measurement of the oxidation of NADH.

Wang discloses a virtual screening method for herbicides. In this method, a three-dimensional structure of a compound to be tested and a three-dimensional structure of the binding site of the enzyme to be inhibited (acetohydroxyacid synthase) are provided. Binding modes and binding energy were calculated using computational docking algorithms (Wang, J.-G.; Xiao, Y.-J.; Li, Y.-H.; Ma, Y.; Li, Z.-M. Identification of some novel AHAS inhibitors via molecular docking and virtual screening approach. Bioorg. Med. Chem. 2007, 15, 374- 380).

Hu discloses the virtual design of herbicidal compounds based on a screening hit structure. The virtual library of three-dimensional structures obtained was the docked into the three-dimensional structure of an enzyme binding site (Hu, De-Jin & Liu, Su-Fang & Huang, Tong-Hui & tu, Hai-Yang & Zhang, Aidong. (2009). Synthesis and Herbicidal Activities of Novel 4-(4-(5-methyl-3-arylisoxazol-4-yl)thiazol-2-yl)piperidyl Carboxamides and Thiocarboxamides. Molecules (Basel, Switzerland). 14. 1288-303).

Lopez-Ramos discloses the use of various virtual screening methods to identify potential herbicides. In the latter method, a three-dimensional structure of the compound to be tested was provided and docked computationally into a three dimensional structure of the binding site of the target enzyme, a hydroxyphenylpyruvate dioxygenase (J. Chem. Inf. Model. 50, 5, 801-814).

Manchera discloses a computational study on the binding interactions of inhibitors and activators of PEPC. The three-dimensional structure of the active site was provided and various inhibitors were modelled into it (Mancera, R.L., and Carrington, B.J. (2005). The molecular binding interactions of inhibitors and activators of phosphoenolpyruvate carboxylase. J. Mol. Struct. Theochem. 755, 151-159).

Rustin discloses that phosphoenolpyruvate is in competition with malate for the active site of PEPC. An additional malate specific binding site exists where malate causes non-competitive inhibition (Pierre Rustin, Christopher R. Meyer, Randolph T. Wedding Plant Physiology Nov 1991, 97 (3) 1011-1016).

Cretin discloses the differential inhibition of PEPC from C4 plants as compared to the same enzyme from C3 plants by the herbicide 2,4-D and two related compounds (PHYTOCHEMISTRY, vol. 22, no. 12, 1 January 1983 (1983-01-01), pages 2661-2664).

Rao discloses the use of the herbicides paraquat for inhibiting PEPC from various plant species both in vitro and in vivo (Rao, Idupulapati & Swamy, P.M. & Das, V.S.R.. (1980). Herbicide-inhibited phosphoenolpyruvate carboxylase in leaves of six nonsucculent scrub species. Zeitschrift für Pflanzenphysiologie. 99. 69-74. 10.1016/S0044-328X(80)80114-0).

Engelmann discloses that the PEPC of C4 plants is more sensitive to malate than the corresponding enzyme of C3 plants when the enzymes are in their activated state, as caused by the presence of glucose-6-phosphate. The sensitivity is inversed in absence of glucose-6-phosphate (Engelmann et al. 2003. Molecular evolution of C4 phosphoenolpyruvate carboxylase in the genus Flaveria - a gradual increase from C3 to C4 characteristics. Planta 217: 717-725).

As set forth above, ilimaquinone and unguinol were identified as inhibitors of PPDK. These inhibitors, however, have several disadvantages, since they inhibit a subgroup of C4 plants. Tropical grasses, which use PEP carboxykinase for CO2 release and PEP regeneration, are not inhibited. Further, the inhibitors need complex biosynthesis pathways and are expensive to produce (100 µg ilimanquinone cost 150 Euro). Also, both compounds have a low solubility in water (high clog values), which hampers the resorption by the plants.

Moreover, the inhibitory concentration of the compounds is high. The half maximal inhibitory concentration (IC50) for unguinol is 40 µM for the isolated enzyme. Therefore, an inhibition of the plant would need much higher concentrations up to millimolar range. Due to the high production costs, the use of these compounds is not economical.

Finally, unguinol is used as growth hormone for animals, has anti-microbial and cytotoxic activity and inhibits the enzyme bile salt hydrolase. Therefore, it is unsuitable as herbicide.

For those flavonoles and flavones that were identified as inhibitors of PEP carboxylase from C4 plant *A. viridis,* the control experiments with C3 plants are missing to show the selective inhibition of the C4 isoform. The title of the publication suggests inhibitory effect of the complete group of flavonoids (Pairoba *et al.,* 1996), but the authors failed to show this claim. Flavonoids are with approx. 7000 different substances the largest class of plant secondary metabolites and have a high structural diversity. From this structurally heterogeneous class of substances, only a few flavonoles and flavones were tested. Flavanones, flavandioles, flavanes, flavanoles, isoflavones and anthocyanes, which also belong to the flavanoides, were not tested.

Furthermore, an inherent disadvantage of naturally occurring flavonoids is their low solubility in water, impeding the application as well the resorption of these substances.

The inherent disadvantage of all tested PEP analogues is the fact that they also inhibit C3 type PEP carboxylases to a certain degree. They do not show any effect on the growth of C4 plants.

Thus, there is a need for effective C4 selective inhibitors. Thus, the present invention relates to the use of a compound, a salt or solvate thereof as C4 plant selective herbicide wherein said compound comprises a cyclic alkyl, aryl, heterocycloalkyl or heteroaryl group, and wherein said compound further comprises
(a) at least a functional group Z being -C(=Y^{Z})R¹, wherein Y^{Z} is selected from the group consisting of O, NH and S, preferably wherein Y^{Z} is O, and wherein R¹ is selected from the group consisting of H, OH and N(R²R³), - O(R²) and -S(R²), wherein R² and R³, are independently of each other, selected from the group consisting of H, Alkyl, cycloalkyl, aryl and heteroaryl and a functional group Q which is -C(=Y^{Q})R⁴, wherein Y^{Q} is selected from the group consisting of O, NH and S, preferably wherein Y^{Q} is O, and wherein R⁴ is selected from the group consisting of H, OH and NR^{4#}R^{5#}, OR^{4#}, SR^{4#} wherein R^{4#} and R^{5#}, are independently of each other, selected from the group consisting of H, alkyl, cycloalkyl, aryl and heteroaryl, wherein the compound has a structure according to the formula (I) 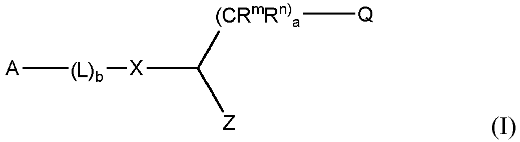
   wherein A is selected from the group consisting of 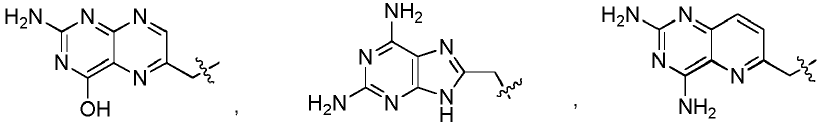 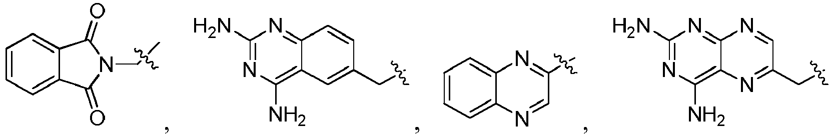 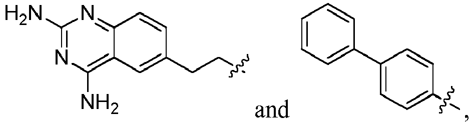
   X is -Y^{X}-, or -C(=Y^{XX})-, wherein Y^{X} is selected from the group consisting of -S-, -O-, -NH-, -N(CH₃)- and NH-NH-,
   and Y^{XX} is selected from the group consisting of S, O and NH,
   in particular -NH- or -C(=Y)- with Y being S or O,
   L is a linking moiety, preferably a linking moiety comprising an aryl group,
   integer b is 0 or 1,
   integer a is in the range of from 2 to 4
   and wherein R^{m} and Rⁿ are, independently of each other, H or alkyl, preferably H or methyl, in particular H,
   or
(b) at least one electron donating group selected from the group consisting of OH, -SH, -O-R^{p}, -S-R^{p}, and -NR^{p}R^{q}, wherein R^{p} and R^{q} are, independently of each other, selected from the group consisting of H, alkyl, cycloalkyl and heteroaryl, wherein the compound has a structure of the formula (IV') or (IV") 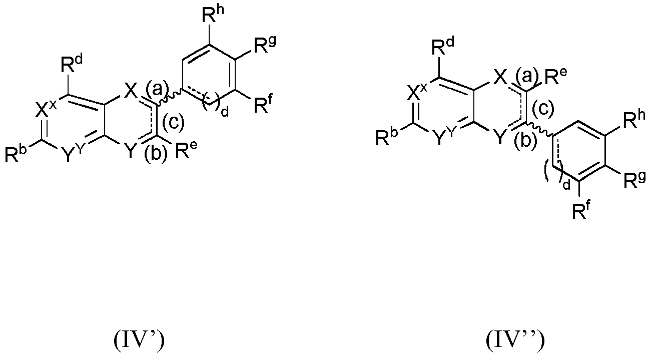 wherein
   X is selected from the group consisting of -C(=O)-, -O- and -N-,
   Y is selected from the group consisting of -C(H)ₚ-, -C(=O)-, -O- and -N-,
   X^{x} is selected from the group consisting of -C(R^{a})- or N, Y^{y} is selected from the group consisting of -C(R^{c})- or N,
   with p being 1 or 2,
   the bond (a) is a single or double bond,
   the bond (b) is a single or double bond,
   the bond (c) is a single or double bond,
   and wherein R^{a} and R^{b} are, independently of each other selected from the group consisting of H, Alkyl, halogene, -C(=Y^{p})R^{r}, and an electron donating group, wherein R^{r} is -OH, -NH₂, -NH-Alkyl, -O-Alkyl, or -S-Alkyl, and
   wherein Y^{p} is selected from the group consisting of S, O and NH, or wherein R^{a} and R^{b} form together a cycloalkyl, cycloheteroalkyl, aryl or heteroaryl, wherein the cycloalkyl, cycloheteroalkyl, aryl or heteroaryl is a single-ring group or a multicyclic group,
   and wherein R^{c}, R^{d}, R^{f}, R^{g} and R^{h} are, independently of each other, selected from the group consisting of H, Alkyl, halogene, -C(=Y^{p})R^{r}, and an electron donating group, wherein R^{r} is -OH, -NH₂, -NH-Alkyl, -O-Alkyl, or -S-Alkyl, and wherein Y^{p} is selected from the group consisting of -S-, -O-, - NH,
   and wherein R^{e} is selected from the group consisting of H, Alkyl, halogene, -C(=Y^{p})R^{r}, -O-C(=Y^{p})R^{r}, an electron donating group, aryl, heteroaryl group and -O-C(=O)-R*, wherein R* is an aryl or heteroaryl group, and wherein
   R^{r} is -OH, -NH₂, -NH-Alkyl, -O-Alkyl, or -S-Alkyl, and wherein Y^{p} is selected from the group consisting of -S-, -O-, -NH
   wherein at least one of R^{a}, R^{b}, R^{c}, R^{d}, R^{f}, R^{g}, R^{e} and R^{h} is the electron donating group selected from the group consisting of OH, -SH, -O-R^{p}, -S-R^{p}, and -NR^{p}R^{q},, with the proviso that in case the bond (c) is a double bond (a) and (b) are both single bonds, and in case X is -C(=O)-,the bond (a) is a single bond, and wherein in case Y is -C(=O)-, the bond (b) is a single bond, and wherein integer d is 1,
said compound being capable of binding to the malate binding site comprised by a phosphoenolpyruvate carboxylase from a C4 plant, thereby inhibiting said phosphoenolpyruvate carboxylase,
and wherein the cyclic alkyl, aryl, heterocycloalkyl or heteroaryl group inhibits binding to the malate binding site of a phosphoenolpyruvate carboxylase from a C3 plant.

In an embodiment of the use of the present invention, Q in (a) is COOH, and the compound has the structure (II):

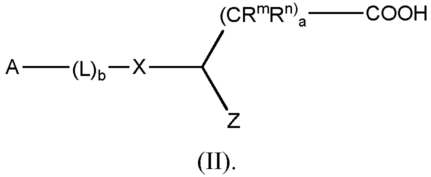

In an embodiment of the use of the present invention, wherein Z in (a) is COOH, the compound having the structure (III):

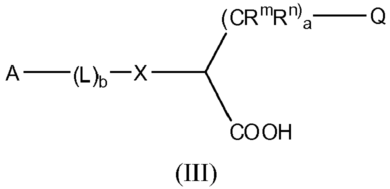

In an embodiment of the use of the present invention, wherein in (a) L has a structure according to the following formula

-[F²]_{c}-[L¹]_{d}-[F¹]ₑ-

wherein L¹ is a linking moiety, preferably selected from the group consisting of alkyl, alkenyl, alkylaryl, arylalkyl, aryl, heteroaryl, alkylheteroaryl and heteroarylalkyl,
and wherein integer d is 0 or 1,
and wherein
F¹ is a functional group consisting of -Y^{F1}-, -C(=Y^{F})-, -C(=Y^{F})-NR^{Y1}-,
wherein Y^{F1} is selected from the group consisting of -S-, -O-, -NH-, -N(CH₃)-and NH-NH-,
Y^{F} is selected from the group consisting of S, O and NH,
and R^{Y1} is H or alkyl,
and wherein in case X is -Y^{X}-, F¹ is -C(=Y^{F})- or -C(=Y^{F})-NR^{Y1} and wherein in case X is -C(=Y^{X})-, -C(=Y^{XX})-NR^{X}-, F¹ is -Y^{F1}-,
integer e is 0 or 1,
F² is a functional group selected from the group consisting of -Y^{F2}-, -C(=Y^{FF})-, -C(=Y^{FF})-NR^{Y2}- and -NR^{Y2}-C(=Y^{FF})-
wherein Y^{F2} is selected from the group consisting of -S-, -O-, -NH-, -N(CH₃)- ,-NH-NH-,
YF^{F} is selected from the group consisting of S, O and NH,
and R^{Y2} is H or alkyl,
and wherein integer c is 0 or 1.

In an embodiment of the use of the present invention, X in (a) is -NH-, wherein L is

-Y^{F2}-L¹-C(=Y^{F})-

wherein L¹ is alkylaryl, arylalkyl, heteroaryl or aryl group, preferably a group having one of the following structures: 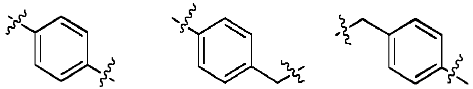 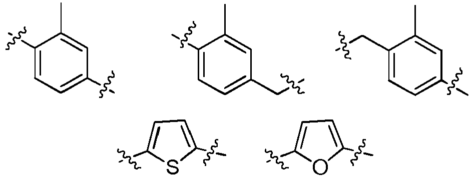 more preferably wherein L is selected from the following structures:

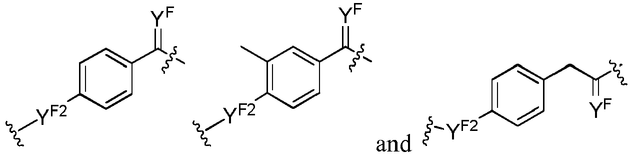

In an embodiment of the use of the present invention, wherein the compound in (b) has a structure of the formula (IVa) or (IVb)

Disclosed is the use of a compound, a salt or solvate thereof as C4 plant selective herbicide wherein said compound comprises a cyclic alkyl, aryl, heterocycloalkyl or heteroaryl group, and wherein said compound further comprises
(a) at least a functional group Z being -C(=Y^{Z})R¹, wherein Y^{Z} is selected from the group consisting of O, NH and S, preferably wherein Y^{Z} is O, and wherein R¹ is selected from the group consisting of H, OH and N(R²R³), -O(R²) and -S(R²), wherein R² and R³, are, independently of each other, selected from the group consisting of H, Alkyl, cycolalkyl, aryl and heteroaryl and a functional group Q which is -C(=Y^{Q})R⁴, wherein Y^{Q} is selected from the group consisting of O, NH and S, preferably wherein Y^{Q} is O, and wherein R⁴ is selected from the group consisting of H, OH and NR^{4#}R^{5#}, OR^{4#}, SR^{4#} wherein R^{4#} and R^{5#}, are independently of each other, selected from the group consisting of H, alkyl, cycloalkyl, aryl and heteroaryl, or
(b) at least one electron donating group selected from the group consisting of OH, -SH, -O-R^{p}, -S-R^{p}, -NR^{p}R^{q} and -O-C(=Y^{p})R^{r}, wherein R^{p} and R^{q} are, independently of each other, selected from the group consisting of H, alkyl, cycloalkyl and heteroaryl, and wherein R^{r} is -OH, -NH₂, -NH-Alkyl, -O-Alkyl or -NH-Alkyl, and wherein Y^{p} is selected from the group consisting of S, O and NH,
said compound being capable of binding to the malate binding site comprised by a phosphoenolpyruvate carboxylase from a C4 plant, thereby inhibiting said phosphoenolpyruvate carboxylase,
and wherein the cyclic alkyl, aryl, heterocycloalkyl or heteroaryl group inhibits binding to the malate binding site of a phosphoenolpyruvate carboxylase from a C3 plant.

The term "alkyl" relates to non-branched alkyl residues and branched alkyl residues, as well as residues comprising one or more heteroatoms or functional groups, such as, by way of example, -O-, -S-, -NH-, -NH-C(=O)-, -C(=O)-NH-. The term also encompasses alkyl groups which are further substituted by one or more suitable substituent.

The term "cyclic alkyl" or "cycloalkyl" relates to optionally suitably substituted 4 to 8-membered single-ring alkyl groups as well as optionally suitably substituted multicyclic alkyl residues.

The term "heterocycloalkyl" relates to optionally suitably substituted 4 to 8-membered single-ring alkyl groups as well as optionally suitably substituted multicyclic alkyl residues comprising one or more, preferably from 1 to 4 such as 1, 2, 3 or 4, heteroatoms, wherein in case the cycloalkyl residue comprises more than 1 heteroatom, the heteroatoms may be the same or different.

The term "alkyl", "cycloalkyl", "cyclic alkyl" and "heterocycloalkyl", also encompasses groups which are further substituted by one or more suitable substituent.

The term "substituted" preferably refers to alkyl, cycloalkyl, cyclic alkyl, aryl, heteroaryl and heterocylcoalkyl groups being substituted in any position by one or more substituents, preferably by 1, 2, 3, 4, 5 or 6 substituents, more preferably by 1, 2, or 3 substituents. If two or more substituents are present, each substituent may be the same or may be different from the at least one other substituent.

The substituents may be, for example, selected from the group consisting of aryl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxy, phosphate, phosphonato, phosphinato, amino, acylamino, including alkylcarbonylamino, arylcarbonylamino, carbamoyl and ureido, amidino, nitro, imino, sulfhydryl, alkylthio, arylthio, thiocarboxylate, sulfates, alkylsulfinyl, sulfonate, sulfamoyl, sulfonamido, trifluoromethyl, cyano, azido, cycloalkyl such as e.g. cyclopentyl or cyclohexyl, heterocycloalkyl such as e.g. morpholino, piperazinyl or piperidinyl, alkylaryl, arylalkyl and heteroaryl. Preferred substituents of such organic residues are, for example, halogens, such as fluorine, chlorine, bromine or iodine, amino groups, hydroxyl groups, carbonyl groups, thiol groups and carboxyl groups.

Preferred examples for cycloalkyl groups are, e.g., cyclopentyl or cyclohexyl or stereoids, e.g., steroids having a pregnane skeleton, such as 12, 20-dioxypregnane-3-yl.

Preferred examples for heterocycloalkyl groups are, e.g., morpholino, piperazinyl or piperidinyl.

The term "aryl" refers to optionally suitably substituted 5- and 6-membered single-ring aromatic groups as well as optionally suitably substituted multicyclic groups, for example bicyclic or tricyclic aryl groups. The term "aryl" thus includes, for example, optionally substituted phenyl groups or optionally suitably substituted naphthyl groups. Aryl groups can also be fused or bridged with alicyclic or heterocycloalkyl rings are not aromatic so as to form a polycycle, e.g., benzodioxolyl or tetraline. The term "aryl" further includes aromatic groups which linked via a single bond to further aromatic groups so as to form, e.g., biphenyl groups.

The term "heteroaryl" includes optionally suitably substituted 5- and 6-membered single-ring aromatic groups as well as substituted or unsubstituted multicyclic aryl groups, for example bicyclic or tricyclic aryl groups, comprising one or more, preferably from 1 to 4 such as 1, 2, 3 or 4, heteroatoms, wherein in case the aryl residue comprises more than 1 heteroatom, the heteroatoms may be the same or different. Such heteroaryl groups including from 1 to 4 heteroatoms are, for example, benzodioxolyl, pyrrolyl, furanyl, thiophenyl, thiazolyl, isothiazolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, napthyridinyl, quinolinyl, isoquinolinyl, indolyl, isoindolyl, benzofuranyl, purinyl, deazapurinyl, pteridinyl, or indolizinyl.

The term "substituted aryl" and the term "substituted heteroaryl" describes moieties having substituents replacing a hydrogen on one or more atoms, e.g. C or N, of an aryl or heteroaryl moiety. The substituents may be, for example, selected from the group consisting of alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxy, phosphate, phosphonato, phosphinato, amino, acylamino, including alkylcarbonylamino, arylcarbonylamino, carbamoyl and ureido, amidino, nitro, imino, sulfhydryl, alkylthio, arylthio, thiocarboxylate, sulfates, alkylsulfinyl, sulfonate, sulfamoyl, sulfonamido, trifluoromethyl, cyano, azido, cycloalkyl such as e.g. cyclopentyl or cyclohexyl, heterocycloalkyl e.g. morpholino, piperazinyl or piperidinyl, alkylaryl, arylalkyl and heteroaryl.

Preferred substituents of such organic residues are, for example, halogens, e.g. fluorine, chlorine, bromine or iodine, amino groups, hydroxyl groups, carbonyl groups, thiol groups, amino groups, and carboxyl groups.

Thus, the cyclic alkyl (cycloalkyl), heterocycloalkyl, aryl or heteroaryl group is, e.g., selected from the group consisting of, optionally suitably substituted, benzodioxolyl, pyrrolyl, furanyl, thiophenyl, thiazolyl, isothiazolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, napthyridinyl, quinolinyl, isoquinolinyl, indolyl, isoindolyl, benzofuranyl, purinyl, deazapurinyl, pteridinyl, indolizinyl, phenyl, biphenyl and stereoids.

According to a first preferred embodiment, the present invention relates to a use of a compound a salt or solvate thereof as C4 plant selective herbicide wherein said compound comprises a cyclic alkyl, aryl, heterocycloalkyl or heteroaryl group, wherein the compound has a structure according to the formula (I) 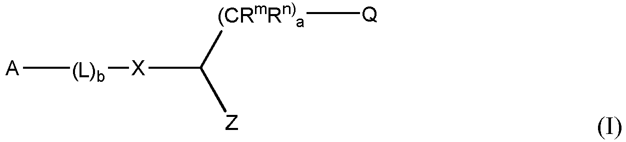 wherein A is the cyclic alkyl, heterocycloalkyl, aryl or heteroaryl group. Preferably, A is an at least bicyclic alkyl, heterocycloalkyl, aryl or heteroaryl group, X is a functional group, L is a linking moiety, integer b is 0 or 1, integer a is in the range of from 1 to 5, preferably 1 to 4, and wherein R^{m} and Rⁿ are, independently of each other, H or alkyl.

The term "at least bicyclic" refers to groups comprising at least two cycles, wherein these cycles may be fused or may be bridged with alicyclic groups as to form the at least bicyclic group. Each cylce may be selected from aromatic, heteroaromatic and non aromatic cyloalkyl or cycloheteroaryl groups. The term "at least bicyclic" thus also relates to groups such as biphenyl groups.

According to a preferred embodiment, A is a bicyclic aryl or bicyclic heteroralryl group. The term "bicyclic aryl group" refers to groups in which at least one of both cycles is an aryl group. The term "bicyclic heteroaryl group" refers to groups in which at least one of both cycles is a heteroaryl group.

Preferably, the group A comprises one of the following core structures: 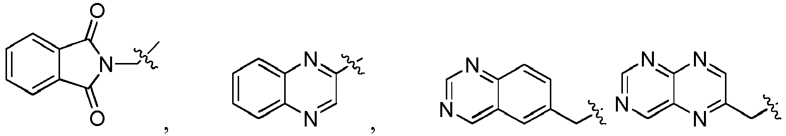 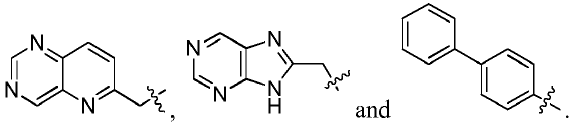 more preferably one of the following core structures

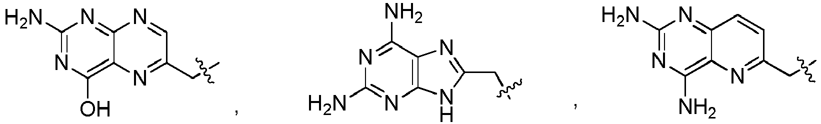

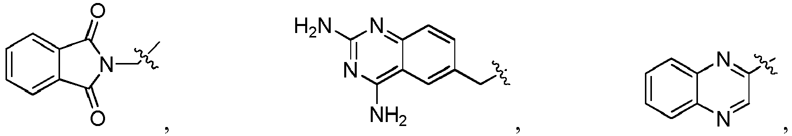

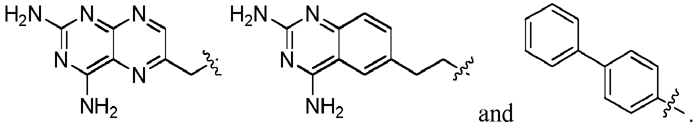

The term "comprising the core structure" is denoted to mean that the shown structure may be optionally suitably substituted.

Thus, the present invention also relates to the to a use of a compound a salt or solvate thereof as C4 plant selective herbicide according to the formula (I) 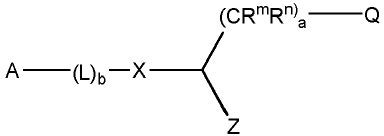 as described above, wherein A comprises one of the following core structures: 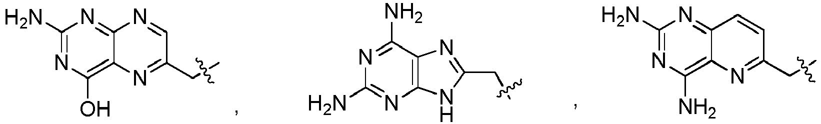 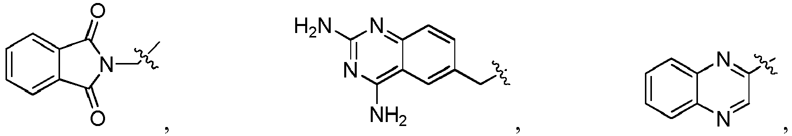 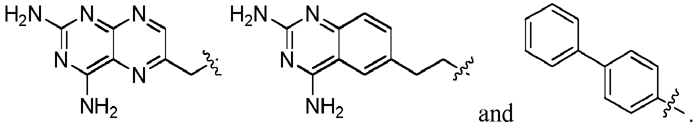 or the core structure:

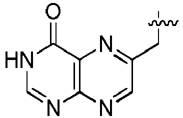

In particular, A has a structure selected from the group consisting of: 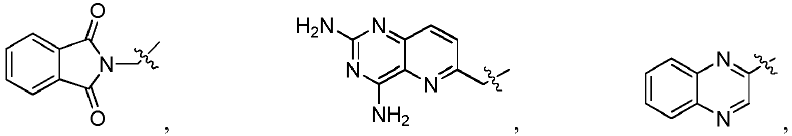 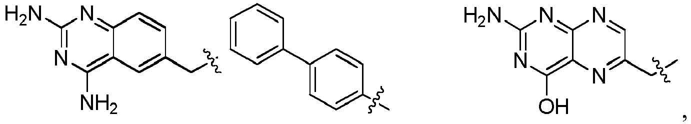 and 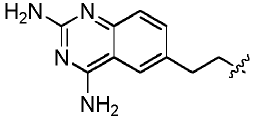 or the structure: 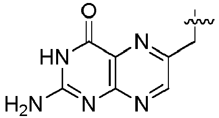 more preferably, of the group consisting of 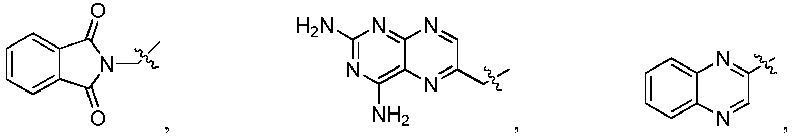 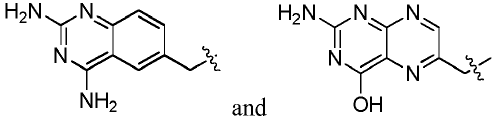 or A is

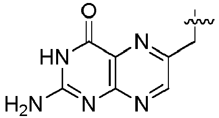

Thus, the present invention also relates to the use of a compound a salt or solvate thereof as C4 plant selective herbicide, the compound having a structure according to one of the following formulas: 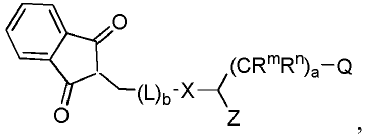 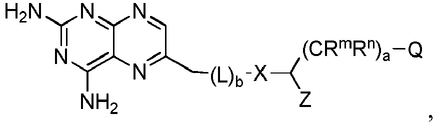 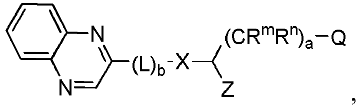 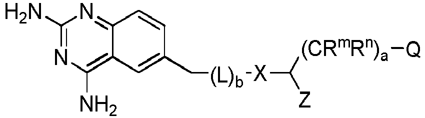 and 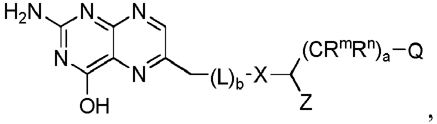 or according to the following formula:

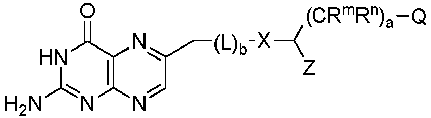

According to the disclosure, A is a stereoid comprising the core structure 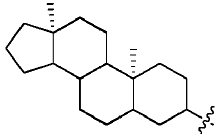 preferably comprising the core structure

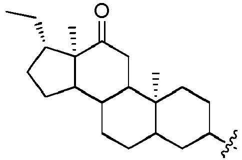

In case A is a steroid, A is preferably selected from one of the following structures: 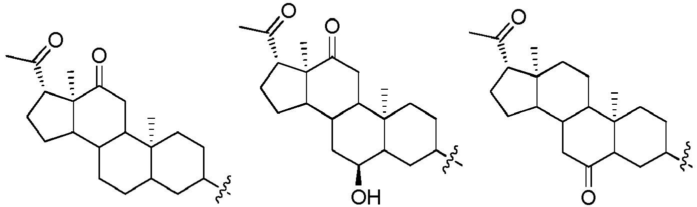 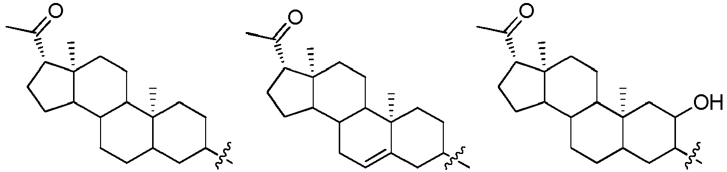 more preferably A has the structure

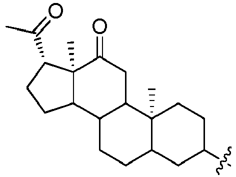

Thus, the present invention also relates to the use of a compound a salt or solvate thereof as C4 plant selective herbicide according wherein A has the structure: 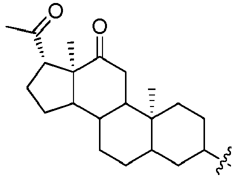 and the compound thus has the following structure:

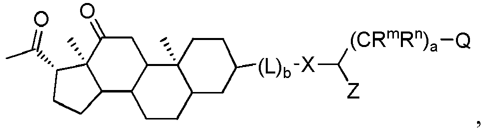

### The functional group Q

As described above, the functional group Q is -C(=Y^{Q})R⁴, wherein Y^{Q} is selected from the group consisting of O, NH and S, preferably wherein Y^{Q} is O or S, and wherein R⁴ is selected from the group consisting of H, OH, -O-R^{4#}, -S-R^{4#} and NR^{4#}R^{5#}, wherein R^{4#} and R^{5#}, are independently of each other, selected from the group consisting of H, alkyl, cycloalkyl, aryl, cycloheteroalkyl and heteroaryl.

Thus, the functional group Q is, for example, one of the following groups: - C(=O)H, -C(=O)OH, -C(=O)-O-Alkyl, -C(=O)-O-Aryl, -C(=O)-O-cycloalkyl, -C(=O)-O-heteroaryl, -C(=O)-O-cycloheteroalkyl -C(=O)NH₂, - C(=O)NHAlkyl, -C(=O)NHAryl, -C(=O)NHcycloalkyl, -C(=O)NHcycloheteroalkyl, - C(=O)N(Hheteroaryl) -C(=S)H, -C(=S)OH, -C(=S)-O-Alkyl, -C(=S)-O-Aryl, -C(=O)-O-cycloalkyl, -C(=S)-O-heteroaryl, -C(=S)-O-cycloheteroalkyl.

More preferably Y^{Q} is O. Thus, the functional group Q is, preferably selected from the group consisting of -C(=O)H, -C(=O)OH, -C(=O)-O-Alkyl, -C(=O)-O-Aryl, -C(=O)-O-cycloalkyl, -C(=O)-O-heteroaryl, -C(=O)-O-cycloheteroalkyl -C(=O)NH₂, - C(=O)NHAlkyl, -C(=O)NHAryl, -C(=O)NHcycloalkyl, -C(=O)NHcycloheteroalkyl, - C(=O)N(Hheteroaryl) , More preferably, the functional group Q is selected from the group consisting of -C(=O)H, -C(=O)OH, -C(=O)-O-Alkyl, -C(=O)-O-Aryl, -C(=O)-O-cycloalkyl, -C(=O)-O-heteroaryl, -C(=O)-O-cycloheteroalkyl.
As to the groups R⁴ and R⁵, these are, independently of each other, H , Alkyl, cycloalkyl, cycloheteroalkyl, aryl or heteroaryl, preferably H or alkyl or cycloalkyl, more preferably H or a linear or branched alkyl group or a cycloalkyl group having from 1 to 10 carbon atoms, preferably an alkyl group or cycloalkyl group selected from the group consisting of methyl, ethyl, propyl, pentyl, butyl, cyclopropyl, cyclobutyl, cyclopropenyl, cyclobutenyl, and hexyl, more preferably H or methyl or ethyl. More preferably one of R^{4#} and R^{5#} is H and one of R^{4#} and R^{5#} is an alkyl group, preferably H or a linear or branched alkyl group having from 1 to 10 carbon atoms, preferably an alkyl group selected from the group consisting of methyl, ethyl, propyl, pentyl, butyl and hexyl, more preferably methyl or ethyl.

In case, Y^{Q} is -C(=O)-O-Alkyl, the alkyl group is preferably a linear or branched alkyl group having from 1 to 10 carbon atoms, preferably an alkyl group selected from the group consisting of methyl, ethyl, propyl, pentyl, butyl and hexyl, more preferably methyl or ethyl.

In case, Y^{Q} is -C(=O)-O-aryl, the aryl group is preferably an optionally substituted phenyl group, preferably phenyl.

Thus, according to a preferred embodiment of the invention, the functional group Q is selected from the group consisting of -C(=O)H, -C(=O)OH, -C(=O)-O-methyl, -C(=O)-O-ethyl, -C(=O)-O-propyl, -C(=O)-O-pentyl, -C(=O)-O-hexyl, -C(=O)-O-phenyl.

In particular, the functional group Q is-C(=O)OH, i.e. -COOH, or -C(=O)-O-methyl, most preferably -COOH.

Thus, the present invention also relates to the use, as described above, the compound having the structure (II):

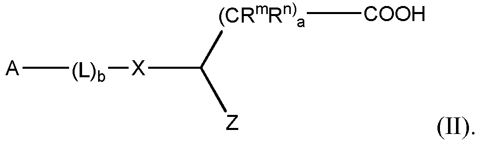

According to an even more preferred embodiment, the present invention also relates to the use of a compound, a salt or solvate thereof as C4 plant selective herbicide according to one of the following formulas: 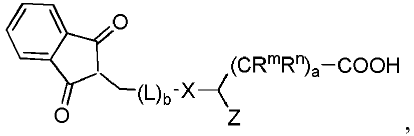 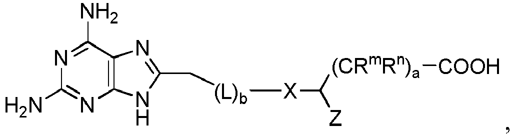 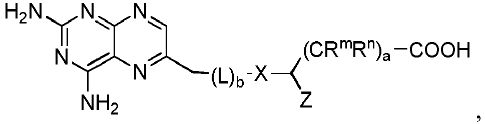 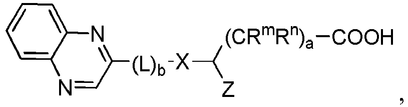 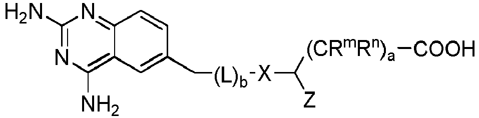 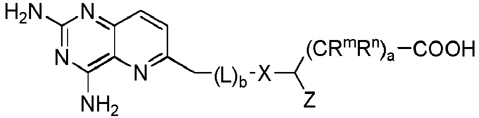 and

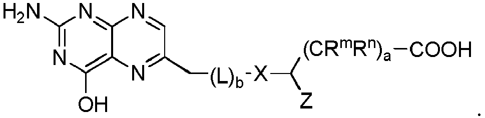

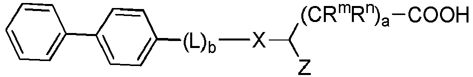

### The functional group Z

As described above, the functional group Z is -C(=Y^{Z})R¹, wherein Y^{Z} is selected from the group consisting of O, NH and S, preferably wherein Y^{Z} is O or S, wherein R¹ is selected from the group consisting of H, OH, -O-R², -S-R² and -NR²R³, wherein R² and R³, are independently of each other, selected from the group consisting of H, Aryl, Alkyl, cycloalkyl, heteroaryl and cyloheteroalkyl.

Thus, the functional group Z is, for example, one of the following groups: - -C(=O)H, -C(=O)OH, -C(=O)-O-Alkyl, -C(=O)-O-Aryl, -C(=O)-O-cycloalkyl, -C(=O)-O-heteroaryl, -C(=O)-O-cycloheteroalkyl -C(=O)NH₂, - C(=O)NHAlkyl, -C(=O)NHAryl, -C(=O)NHcycloalkyl, -C(=O)NHcycloheteroalkyl, - C(=O)N(Hheteroaryl) -C(=S)H, -C(=S)OH, -C(=S)-O-Alkyl, -C(=S)-O-Aryl, -C(=O)-O-cycloalkyl, -C(=S)-O-heteroaryl, -C(=S)-O-cycloheteroalkyl.

More preferably Y^{Z} is O. Thus, the functional group Z is, preferably selected from the group consisting of -C(=O)H, -C(=O)OH, -C(=O)-O-Alkyl, -C(=O)-O-Aryl, -C(=O)-O-cycloalkyl, -C(=O)-O-heteroaryl, -C(=O)-O-cycloheteroalkyl -C(=O)NH₂, - C(=O)NHAlkyl, -C(=O)NHAryl, -C(=O)NHcycloalkyl, -C(=O)NHcycloheteroalkyl and -C(=O)NHheteroaryl.

As to the groups R² and R³, these are, independently of each other, selected from the group consisting of H, Aryl, Alkyl, cycloalkyl, heteroaryl and cyloheteroalkyl, peferably H or alkyl or cycloalkyl, preferably H or a linear or branched, alkyl group or cycloalkyl group, having from 1 to 10 carbon atoms, preferably an alkyl group or cycloalkyl group selected from the group consisting of methyl, ethyl, propyl, pentyl, butyl, cyclopropyl, cyclobutyl, cyclopropenyl, cyclobutenyl and hexyl. More preferably the groups R² and R³ are, independently of each other, H or methyl or ethyl. More preferably one of R² and R³ is H and one of R² and R³ is an alkyl group or cycloalkyl group, preferably H or a linear or branched alkyl group or cycloalkyl group, having from 1 to 10 carbon atoms, preferably H or an alkyl group selected from the group consisting of methyl, ethyl, propyl, pentyl, butyl and hexyl, more preferably H or methyl or ethyl.

In case, Y^{Z} is -C(=O)-O-Alkyl, the alkyl group is preferably a linear or branched alkyl group having from 1 to 10 carbon atoms, preferably an alkyl group selected from the group consisting of methyl, ethyl, propyl, pentyl, butyl and hexyl, more preferably methyl or ethyl.

In case, Y^{Z} is -C(=O)-O-Aryl, the aryl group is preferably an optionally substituted phenyl group, preferably phenyl.

Thus, according to a preferred embodiment of the invention, the functional group Z is selected from the group consisting of -C(=O)H, -C(=O)OH, -C(=O)-O-methyl, -C(=O)-O-ethyl , -C(=O)-O-propyl, -C(=O)-O-pentyl, -C(=O)-O-hexyl, and -C(=O)-O-phenyl.

In particular, the functional group Z is-C(=O)OH, i.e. -COOH, or -C(=O)-O-methyl, most preferably -COOH.

Thus, the present invention also relates to the use, as described above, the compound having the structure (III):

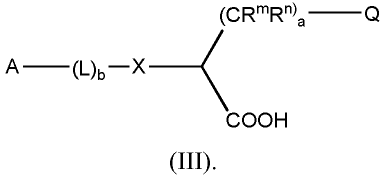

According to an even more preferred embodiment, the present invention also relates to the use of a compound, a salt or solvate thereof as C4 plant selective herbicide according to one of the following formulas: 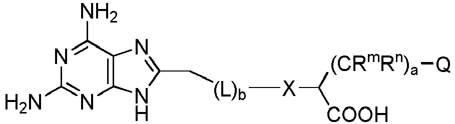 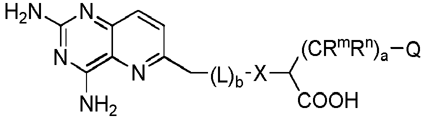 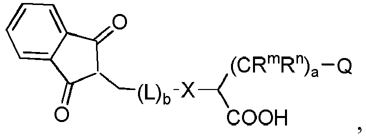 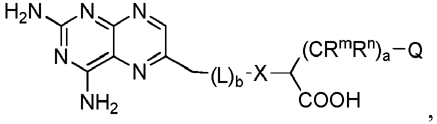 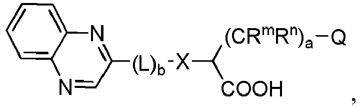 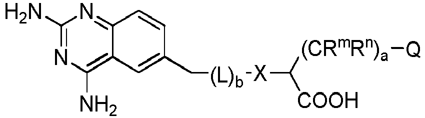 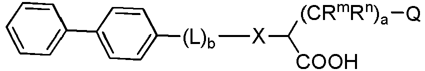 and

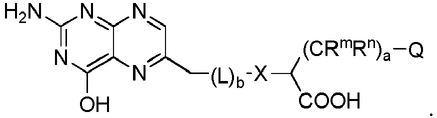

In particular, Z and Q are both -COOH. Thus, the present invention also relates to the use, as described above, the compound having the structure (IV):

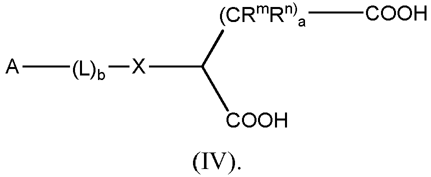

In particular, the present invention also relates to the use of a compound a salt or solvate thereof as C4 plant selective herbicide, the compound having a structure according to one of the following formulas: 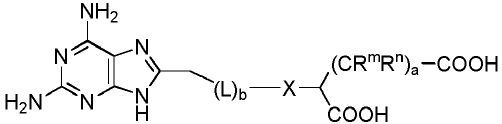 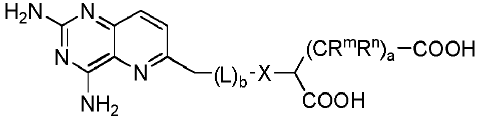 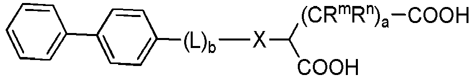 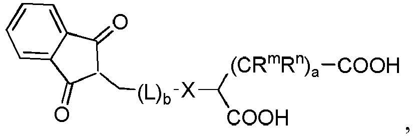 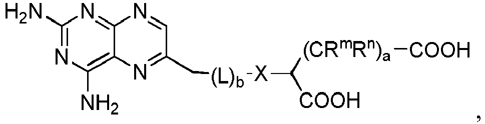 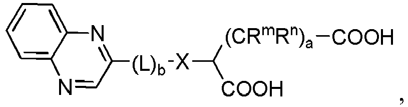 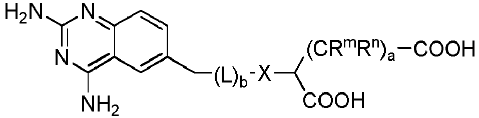 and 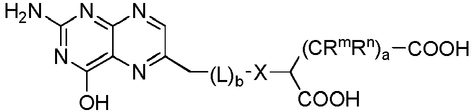 or according to the following formula:

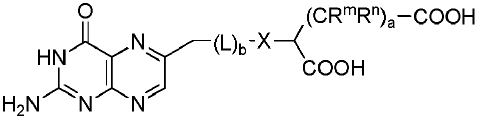

### The functional group X

As described above, X is a functional group, linking the moiety A-(L)_{b} and the carbon atom linked to Z and the structural unit (CR^{m}Rⁿ)ₐ-Q.

Preferably X is -Y^{X}- or -C(=Y^{XX})-, wherein Y^{X} is selected from the group consisting of -S-, -O-, -NH-, -N(CH₃)- and NH-NH-, and Y^{XX} is selected from the group consisting of - S-, -O-, -NH. Thus, X is preferably selected from the group consisting of -S-, -NH-, -O-, - N(CH₃)- and NH-NH-, -C(=O)-, -C(=S)- and -C(=NH)-,

In particular X is -NH- or -C(=Y)- with Y being S or O,

Thus, the present invention also relates to the use, as described above, wherein X is -NH- or -C(=Y)- with Y being S or O, the compound thus having a structure according to one of the following formulas: 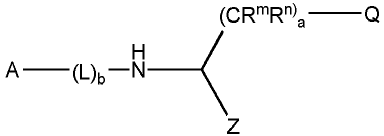 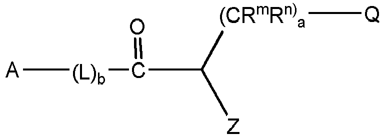 and 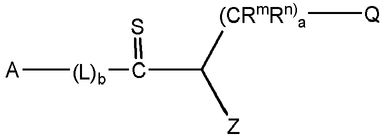 wherein X is most -NH-.

### Integer a

As mentioned above, integer a is in the range of from 1 to 5, such as 1, 2, 3, 4 or 5, preferably in the range of from 1 to 4 In case integer a is greater than 1, each repeating unit [CR^{m}Rⁿ] may be the same or may be different from each other.

Preferably a is in the range of from 1 to 3.

According to a preferred embodiment of the present invention, R^{m} and Rⁿ are, independently of each other, selected from H or branched or linear alkyl groups, comprising 1 to 10, preferably 1 to 8, more preferably 1 to 5, most preferably 1 to 3 carbon atoms. More preferably R^{m} and Rⁿ are, independently of each other, selected from the group consisting of H, methyl, ethyl, propyl, butyl, sec-butyl and tert-butyl, more preferably R^{m} and Rⁿ are, independently of each other, H or methyl.

By way of example, the following preferred structures for the structural unit [CR^{m}Rⁿ]ₐ are mentioned: -CH₂-CH₂-CH₂-, -CH₂-CH₂-, -CH₂-, -CH(CH₃)-, -C(CH₃)₂-, -CH(CH₂CH₃)-, - CH(CH(CH₃)₂)-, -CH(CH₃)-CH₂-, -CH₂-CH(CH₃)-, -CH(CH₃)-CH₂-CH₂-, -CH₂-CH(CH₃)-CH₂-, -CH₂-CH₂-CH(CH₃)-, -CH(CH₃)-CH(CH₃)-, CH(CH₃)-CH(CH₃)-CH₂-, -CH₂-CH(CH₃)-CH(CH₃)-, -CH(CH₃)-CH₂-CH(CH₃)-.

More preferably integer a is 1 or 2, most preferably 2.

According to a particularly preferred embodiment of the present invention, R^{m} and Rⁿ are both H. The structural unit [CR^{m}Rⁿ]ₐ is thus preferably -CH₂-CH₂-CH₂-, -CH₂-CH₂- or -CH₂-, more preferably a is 1 or 2, the structural unit [CR^{m}Rⁿ]ₐ thus preferably having the structure -CH₂-CH₂- or -CH₂-, most preferably a is 2 and the structural unit [CR^{m}Rⁿ]ₐ is -CH₂-CH₂-.

Thus, the present invention also relates to the to a use of a compound a salt or solvate thereof as C4 plant selective herbicide according to the following formula: 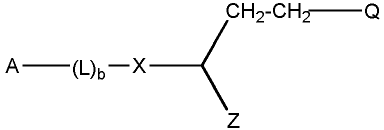 more preferably according to one of the following formulas, 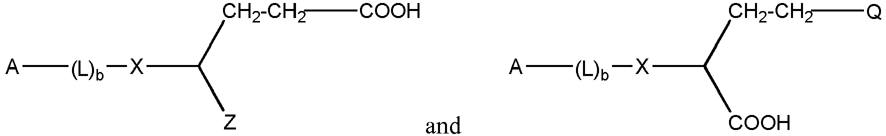 more preferably according to the following formula

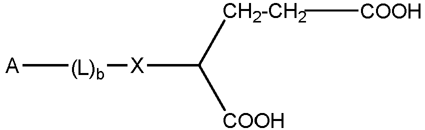

### The linking moiety L

As described above, L is a linking moiety. This linking moiety, if present, i.e. if integer b is 1, links A with the functional group X.

Preferably, the linking moiety L has a structure according to the following formula

-[F²]_{c}-[L¹]_{d}-[F¹]ₑ-

wherein L¹ is a linking moiety linking the structural units -[F²]_{c} and -[F¹]ₑ, wherein L¹ is preferably selected from the group consisting of alkyl, alkenyl, alkylaryl, arylalkyl, aryl, heteroaryl, alkylheteroaryl and heteroarylalkyl, wherein integer d is 0 or 1, and wherein F1 and F2 are functional groups which may be the same or may differ from each other, integer e is 0 or 1 and integer c is 0 or 1.

The term "alkenyl" as used in the context of the present invention refers to unsaturated alkyl groups having at least one double bond. The term also encompasses alkenyl groups which are substituted by one or more suitable substituents.

The term "alkynyl" refers to unsaturated alkyl groups having at least one triple bond. The term also encompasses alkynyl groups which are substituted by one or more suitable substituents.

The term "alkylaryl" as used in the context of the linking moiety described in the present invention is denoted to mean a linking moiety having the structure -alkyl-aryl-, thus being linked on one side via the alkyl group and on the other side via the aryl group, wherein this term is meant to also encompass linking moieties such as -alkyl-aryl-alkyl- linking moieties.

The term "alkylaryl group", when used in the context of any substituent described hereinunder and above, is denoted to mean a residue being linked via the alkyl portion, said alkyl portion being further substituted with an aryl moiety.

The term "arylalkyl" as used in the context of any linking moiety described in the present invention is denoted to mean a linking moiety having the structure -aryl-alkyl-, thus being linked on one side via the aryl group and on the other side via the alkyl group, wherein this term is meant to also encompass linking moieties such as -aryl-alkyl-aryl- linking moieties.

The term "arylalkyl group", when used in the context of any substituent described hereinunder and above, is denoted to mean a residue being linked via the aryl portion, said aryl portion being further substituted with an alkyl moiety.

The term "alkylheteroaryl" as used in the context of any linking moiety described in the present invention is denoted to mean a linking moiety having the structure -alkyl-heteroaryl-, thus being linked on one side via the alkyl group and on the other side via the heteroaryl group, wherein this term is meant to also encompass linking moieties such as -alkyl-heteroaryl-alkyl- linking moieties.

The term "alkylheteroaryl group", when used in the context of any substituent described hereinunder and above, is denoted to mean a residue being linked via the alkyl portion, said alkyl portion being further substituted with a heteroaryl moiety.

The term "heteroarylalkyl" as used in the context of any linking moiety described in the present invention is denoted to mean a linking moiety having the structure -heteroaryl-alkyl-, thus being linked on one side via the heteroaryl group and on the other side via the alkyl group, wherein this term is meant to also encompass linking moieties such as -heteroaryl-alkyl-heteroaryl- linking moieties.

The term "heteroarylalkyl group", when used in the context of any substituent described hereinunder and above, is denoted to mean a residue being linked via the heteroaryl portion, said heteroaryl portion being further substituted with an alkyl moiety.

Preferably L¹ is an alkylaryl, arylalkyl, heteroaryl or aryl group, preferably a group having one of the following structures: 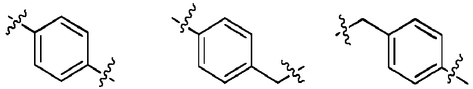 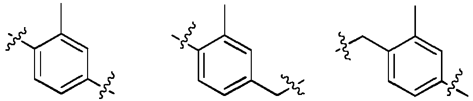 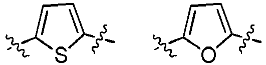 more preferably a group having one of the following structures: 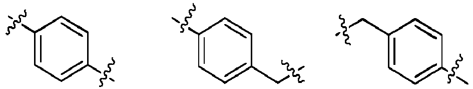 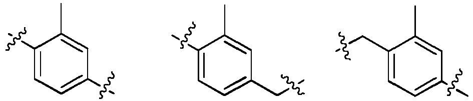 and even more preferably a group having one of the following structures:

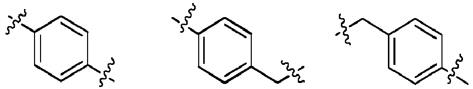

### The functional group F¹:

F¹ is a functional group linking X and the structural unit A-[F²]_{c}-[L¹]_{d}. There are, in general, no particular restrictions as regards the chemical nature of the functional group F¹ provided that a stable bond is formed linking X and the structural unit A-[F²]_{c}-[L¹]_{d}. Preferably F¹ is a functional group is a functional group selected from the group consisting of -Y^{F1}-, -C(=Y^{F})-, and -C(=Y^{F})-NR^{Y1}-, wherein Y^{F1} is selected from the group consisting of -S-, -O-, -NH-, -N(CH₃)- and NH-NH-, and wherein Y^{F} is selected from the group consisting of S, O, NH and R^{Y1} is H or alkyl.

Thus, F¹ is preferably selected from the group consisting of -S-, -O-, -NH-, -N(CH₃)- and NH-NH-, -C(=S)-, -C(=O)-, -C(=NH)-, -C(=O)-NH-, -C(=O)-N(alkyl)-, -C(=S)-NH-, -C(=S)-N(alkyl)-, -C(=NH)-NH-, -C(=NH)-N(alkyl)-.

In case X is -Y^{X}-, F¹ is -C(=Y^{F})- or -C(=Y^{F})-NR^{Y1}.

### The functional group F²:

F² is a functional group linking A and the structural unit -[L¹]_{d}-[F¹]ₑ-X. There are, in general, no particular restrictions as regards the chemical nature of the functional group F¹ provided that a stable bond is formed linking A and the structural unit -[L¹]_{d}-[F¹]ₑ-X. Preferably F² is selected from the group consisting of -Y^{F2}-, -C(=Y^{FF})-, -C(=Y^{FF})-NR^{Y2}- and -NR^{Y2}-C(=Y^{FF})-, wherein Y^{F2} is selected from the group consisting of -S-, -O-, -NH-, -N(CH₃)- , -NH-NH-, Y^{FF} is selected from the group consisting of S, O and NH and R^{Y2} is H or alkyl.

Thus, F² is preferably selected from the group consisting of -S-, -O-, -NH-, -N(CH₃)-, NH-NH-, -C(=S)-, -C(=O)-, -C(=NH)-, -C(=O)-NH-, -C(=O)-N(alkyl)-, -C(=S)-NH-, - C(=S)-N(alkyl)-, -C(=NH)-NH-, -C(=NH)-N(alkyl)-. -NH-C(=O)-, -N(alkyl)-C(=O)-, - NH-C(=S)-, -N(alkyl)-C(=S)-, -NH-C(=NH)- and -N(alkyl)-C(=NH)-.

### Preferred linking moieties according to the invention

According to a preferred embodiment of the invention, L has the structure

-Y^{F2}-L¹-C(=Y^{F})-

with Y^{F2}, L¹ and Y^{F2} being as described above. Preferably, in this preferred case, L¹ is aryl or alkylaryl.

In particular L is selected from the following structures:

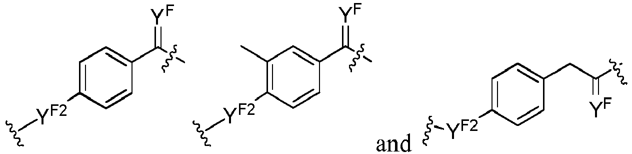

By way of example, the following preferred embodiments for L are described:

**Table 1: Preferred linking moieties L**

| -[F²]_{c}- | -[F¹]ₑ- | -[L¹]_{d}- |
|---|---|---|
| -NH- | -C(=O)- | selected from the group consisting of |
| -N(Me)- | -C(=O)- | |
| -O- | -C(=O)- | 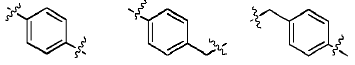 |
| -S- | -C(=O)- | |
| -C(=O)-NH- | -C(=O)- | |
| -NH-C(=O)- | -C(=O)- | 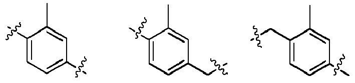 |
| -NH- | -C(=S)- | |
| -N(Me)- | -C(=S)- | |
| -O- | -C(=S)- | 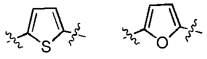 |
| -S- | -C(=S)- | |
| -C(=O)-NH- | -C(=SO)- | |
| -NH-C(=O)- | -C(=S)- | |

**Table 2: Preferred compounds of the invention**

| | | | | | |
|---|---|---|---|---|---|
| 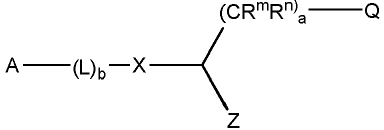 | | | | | |

| A | **(L)b** | **X** | **Z** | **(CR^{m}Rⁿ)ₐ** | Q |
|---|---|---|---|---|---|
| 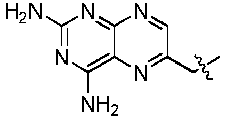 | Selected from the group consisting of | -NH- | -COOH | -CH₂-CH₂- | -COOH |
| | 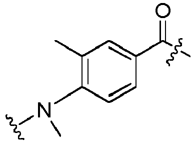 | | | | |
| | 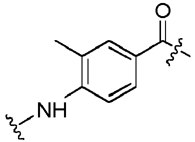 | | | | |
| | 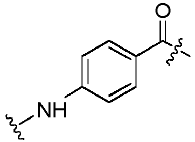 | | | | |
| | 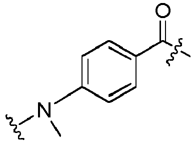 | | | | |
| | 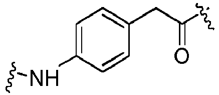 | | | | |
| | 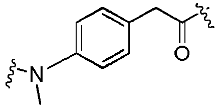 | | | | |
| 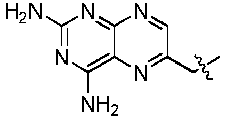 | Selected from the group consisting of | -NH- | -COOH | -CH₂-- | -COOH |
| | 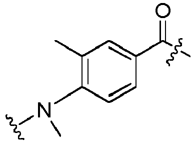 | | | | |
| | 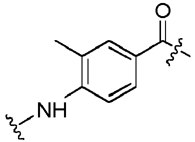 | | | | |
| | 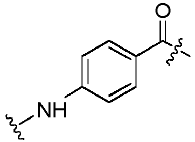 | | | | |
| | 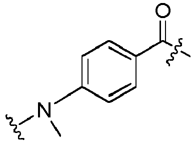 | | | | |
| | 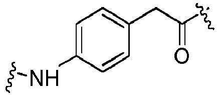 | | | | |
| | 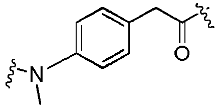 | | | | |
| 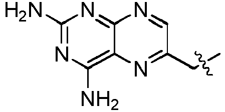 | 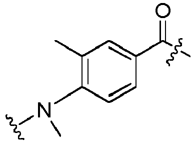 Selected from the group consisting of | -N(Me)- | -COOH | -CH₂-CH₂- | -COOH |
| | 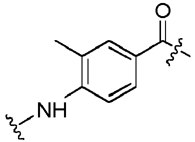 | | | | |
| | 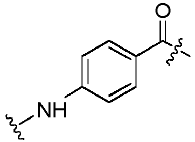 | | | | |
| | 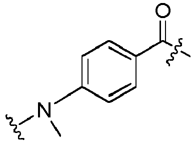 | | | | |
| | 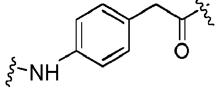 | | | | |
| | 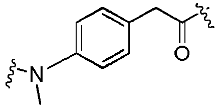 | | | | |
| 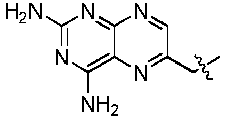 | Selected from the group consisting of | -N(Me)- | -COOH | -CH₂-- | -COOH |
| | 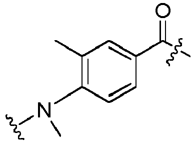 | | | | |
| | 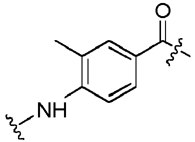 | | | | |
| | 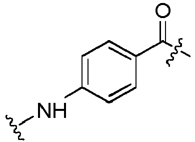 | | | | |
| | 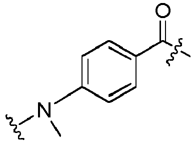 | | | | |
| | 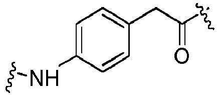 | | | | |
| | 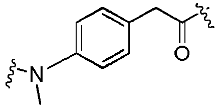 | | | | |
| 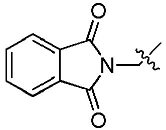 | | -C(=O)-NH | -COOH | -CH₂-CH₂- or | -COOH |
| | | | | -CH₂-- | |
| 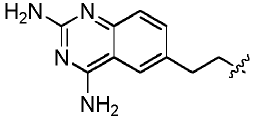 | Selected from the group consisting of | -NH- | -COOH | -CH₂-CH₂- | -COOH |
| | 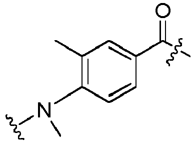 | | | | |
| | 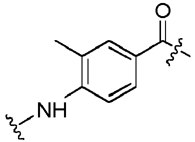 | | | | |
| | 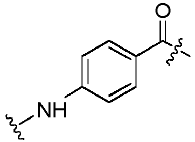 | | | | |
| | 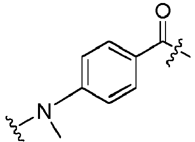 | | | | |
| | 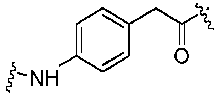 | | | | |
| | 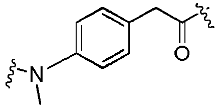 | | | | |
| 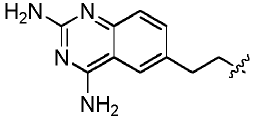 | Selected from the group consisting of | -NH- | -COOH | -CH₂- | -COOH |
| | 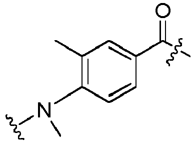 | | | | |
| | 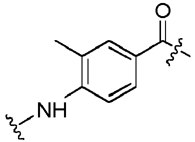 | | | | |
| | 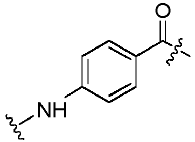 | | | | |
| | 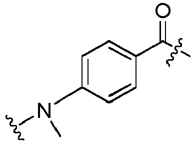 | | | | |
| | 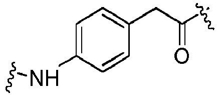 | | | | |
| | 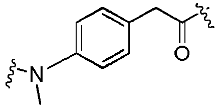 | | | | |
| 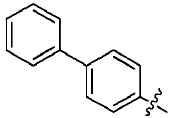 | b = 0 | -C(=O)-NH | -COOH | -CH₂- Or -CH₂-CH₂- | -COOH |
| 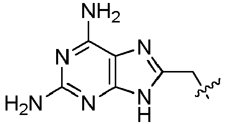 | Selected from the group consisting of | -C(=O)-NH | -COOH | -CH₂- | -COOH |
| | 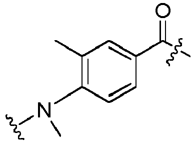 | | | or | |
| | | | | -CH₂-CH₂- | |
| | 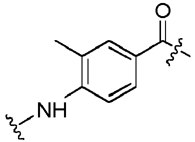 | | | | |
| | 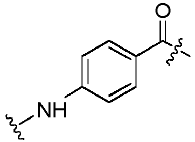 | | | | |
| | 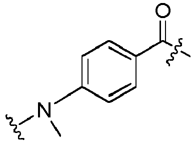 | | | | |
| | 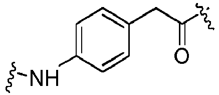 | | | | |
| | 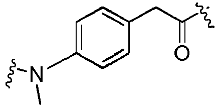 | | | | |
| 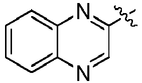 | Selected from the group consisting of | -C(=O)-NH | -COOH | -CH₂- | -COOH |
| | b = 0 | | | or | |
| | | | | -CH₂-CH₂ | |
| | 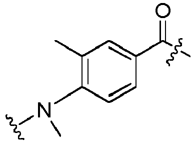 | | | | |
| | 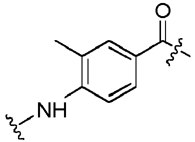 | | | | |
| | 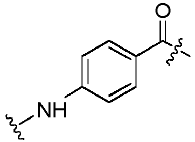 | | | | |
| | 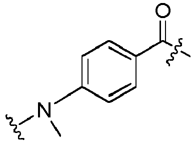 | | | | |
| | 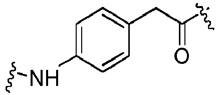 | | | | |
| | 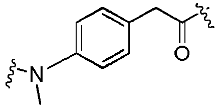 | | | | |
| 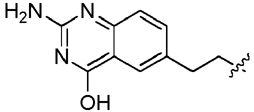 | Selected from the group consisting of | C(=O)-NH | -COOH | -CH₂-CH₂- or | -COOH |
| | 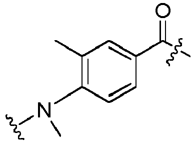 | | | -CH₂-- | |
| | 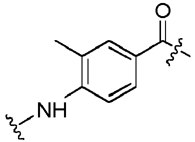 | | | | |
| | 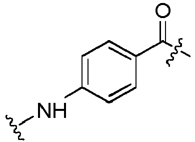 | | | | |
| | 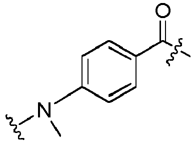 | | | | |
| | 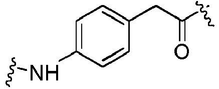 | | | | |
| | 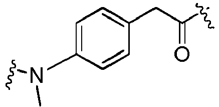 | | | | |
| 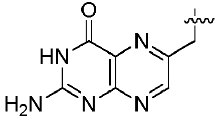 | Selected from the group consisting of | -NH- | -COOH | -CH₂-CH₂- | -COOH |
| | 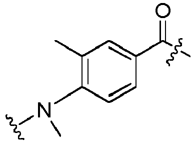 | | | | |
| | 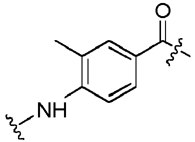 | | | | |
| | 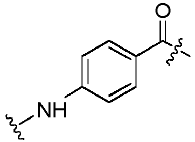 | | | | |
| | 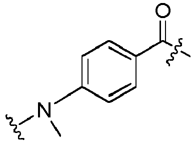 | | | | |
| | 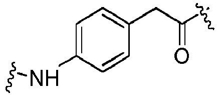 | | | | |
| | 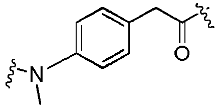 | | | | |

According to an alternative embodiment of the present invention, the compound has a structure of the formula (IVa) or (IVb) 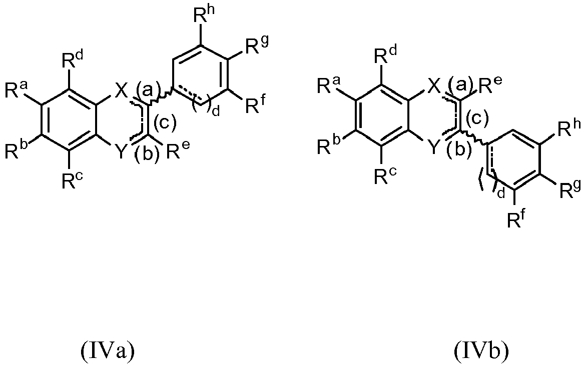 wherein
X is selected from the group consisting of -C(=O)-, -O- and -N-,
Y is selected from the group consisting of -C(H)ₚ-, -C(=O)-, -O- and -N-,
with p being 1 or 2,
the bond (a) is a single or double bond,
the bond (b) is a single or double bond,
the bond (c) is a single or double bond,
and wherein R^{a}, R^{b}, R^{c}, R^{d} , R^{e}, R^{f}, R^{g} and R^{h} are, independently of each other, selected from the group consisting of H, Alkyl and an electron donating group, or R^{a}, R^{b}, R^{c}, R^{d} , R^{e}, R^{f}, R^{g} and R^{h} independently of each other, selected from the group consisting of H, Alkyl, -C(=Y^{p})R^{r} and an electron donating group, wherein R^{r} is -OH, -NH₂, -NH-Alkyl, - O-Alkyl, or -S-Alkyl, and wherein Y^{p} is selected from the group consisting of S, O and NH, preferably R^{a}, R^{b}, R^{c}, R^{d} , R^{e}, R^{f}, R^{g} and R^{h} independently of each other, selected from the group consisting of H, Alkyl, -C(=Y^{p})R^{r} and an electron donating group, in particular R^{a}, R^{b}, R^{c}, R^{d} , R^{e}, R^{f}, R^{g} and R^{h} are, independently of each other, selected from the group consisting of H, Alkyl and an electron donating group,
wherein at least one of R^{a}, R^{b}, R^{c}, R^{d} , R^{e} and R^{f} is an electron donating group, with the proviso that in case the bond (c) is a double bond (a) and (b) are both single bonds, and in case X is -C(=O)-,the bond (a) is a single bond, and wherein in case Y is -C(=O)-, the bond (b) is a single bond.

The term "electron donating group" is recognized in the art, and denotes the tendency of a functional group to donate valence electrons to neighboring atoms by means of a difference in electronegativity with respect to the neighbouring atom (inductive effect) and/or by donating of pi-electrons via conjugation (mesomeric effect). According to the invention said electron donating group is preferably selected from the group consisting of - OH, -SH, -O-R^{p}, -S-R^{p}, -NR^{p}R^{q} and -OC(=Y^{p})R^{r}, wherein R^{p} and R^{q} are, independently of each other, selected from the group consisting of H, alkyl, cycloalkyl, heterocycloalkyl, aryl and heteroaryl, R^{r} is is preferably selected from -OH, -NH₂, -NH-Alkyl, -O-Alkyl, or - S-Alkyl, and wherein Y^{p} is selected from the group consisting of S, O and NH, more preferably the electron donating group is selected from the group consisting of - OH, -SH, -O-R^{p}, -S-R^{p} and -NR^{p}R^{q}, wherein R^{p} and R^{q} are, independently of each other, selected from the group consisting of H, alkyl, cycloalkyl, heterocycloalkyl, aryl and heteroaryl,
R^{p} and R^{q} are, independently of each other, preferably selected from the group consisting of H or alkyl or cycloalkyl, more preferably H or a linear or branched alkyl group or a cycloalkyl group having from 1 to 10 carbon atoms, preferably an alkyl group or cycloalkyl group selected from the group consisting of methyl, ethyl, propyl, pentyl, butyl, cyclopropyl, cyclobutyl, cyclopropenyl, cyclobutenyl, and hexyl, more preferably H or methyl or ethyl. More preferably at least one of R^{p} and R^{q} is H and one of R^{p} and R^{q} is H or an alkyl group, preferably H or a linear or branched alkyl group having from 1 to 10 carbon atoms, preferably H or an alkyl group selected from the group consisting of methyl, ethyl, propyl, pentyl, butyl and hexyl, more preferably methyl or ethyl.

R^{r} is preferably selected from the group consisting of -OH, -NH₂, -NH-Alkyl, -O-Alkyl and -SH-Alkyl. Y^{p} is preferably selected from the group consisting of S, O and NH.

Preferably the at least one electron donation group is -OH, i.e. at least one of R^{a}, R^{b}, R^{c}, R^{d} , R^{e} and R^{f} is -OH. Thus, the present invention to the use of a compound, as described above, wherein the compound has the structure (IVa) or (IVb), wherein at least one of R^{a}, R^{b}, R^{c}, R^{d} , R^{e} and R^{f} is -OH.

In the above-mentioned formulas (IVa) and (IVb), the bond " 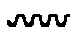 " represents a bond with non-defined stereochemistry, i.e. this term represents a bond encompassing both possible stereochemistries, i.e. atropisomers. Atropisomers are stereoisomers resulting from hindered rotation around this single bond.

The compounds may thus be a mixture of atropisomers or the isolated form of one specific stereoisomer. Preferably, the compound of the invention is used as isolated stereoisomer.

### The groups X and Y

As described above, the group X in the above-mentioned formulas (IVa) and (IVb) is selected from the group consisting of -C(=O)-, -O- and -N-, preferably X is O or N.

Thus, the compound has preferably one of the following structures:

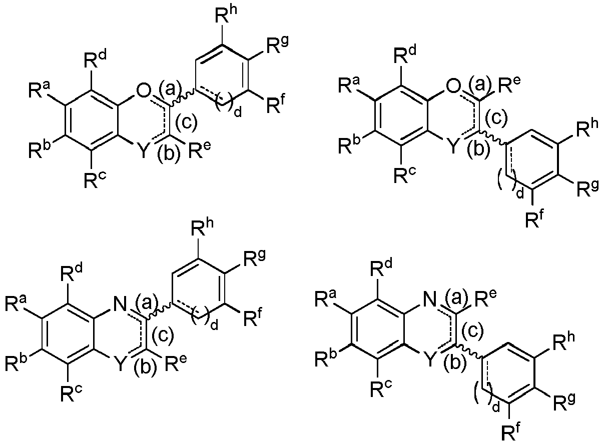

In case X is O, (a) and (b) are preferably single bonds, whereas (c) is a single or a double bond.

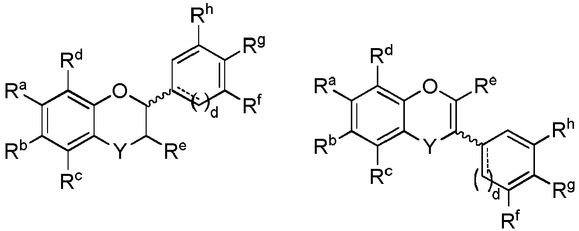

In case X is O, Y is preferably -C(H)ₚ- or -C(=O)-, the compound thus preferably having a structure according to one of the following formulas:

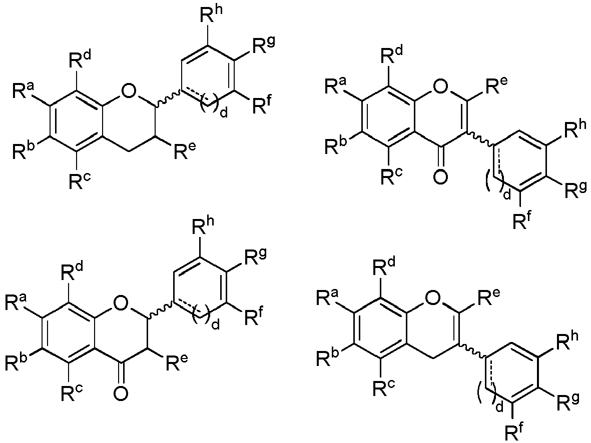

According to an alternative preferred embodiment, X is O, (a) and (b) are double bonds:

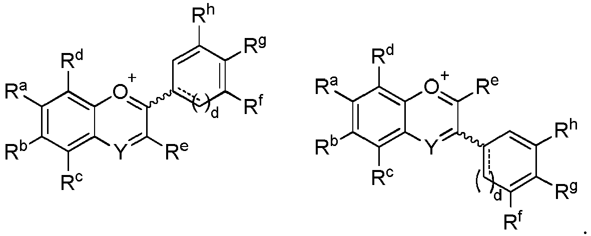

In case X is N, (a) and (b) are preferably double bonds, the compound thus having one of the following structures:

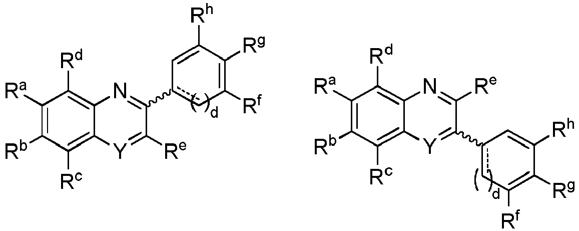

According to this embodiment, Y is most preferably -N-, the compound thus most preferably having a structure according to the following formulas:

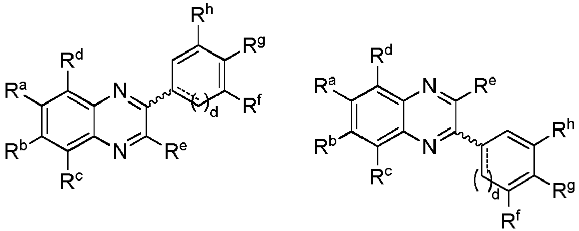

In the structural units 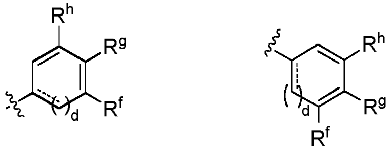 integer d is 0 or 1. In case d is 1, the core structure of the structure shown above is a 6-membered aromatic ring, being substituted with groups R^{h}, R^{g} and R^{f}.

In case (d) is 0, the core structure of the structure shown above is a 5-membered aromatic ring, being substituted with groups R^{h}, R^{g} and R^{f}.

According to a particularly preferred embodiment of the invention d is 1. More preferably, the compound has a structure selected from the group consisting of 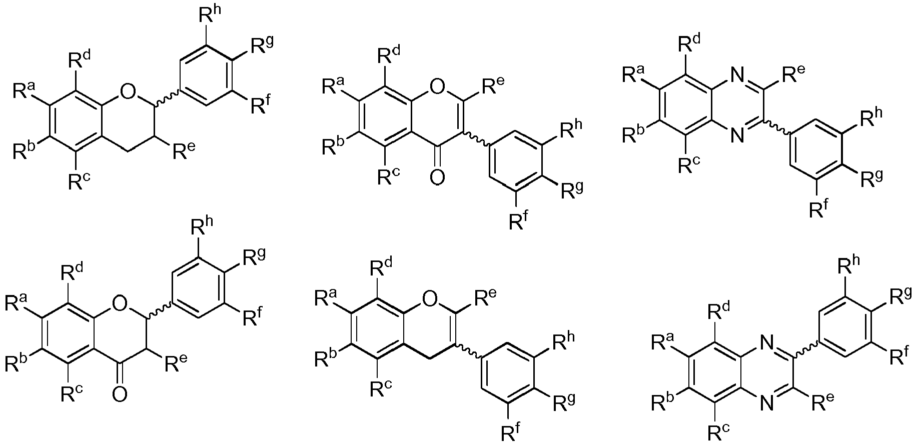 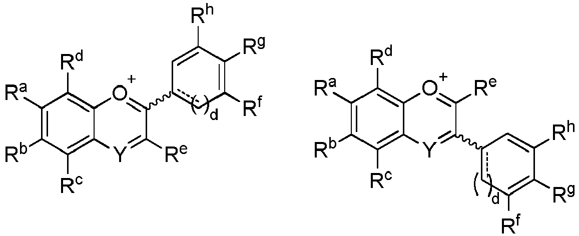 more preferably a structure selected from the group consisting of

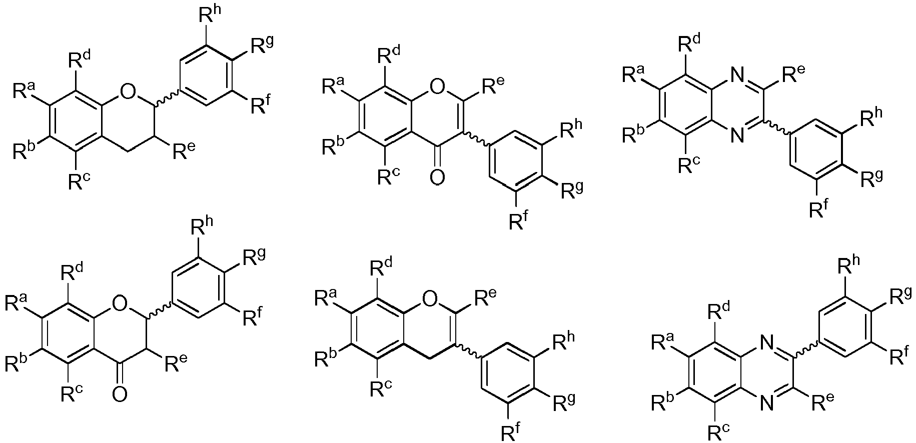

Most preferably, R^{a}, R^{b}, R^{c}, R^{d} , R^{e}, R^{f}, R^{g} and R^{h} are, independently of each other, selected from the group consisting of H, -O-Methyl, -OH and Methyl.

Thus, the present invention also relates to compound, as described above wherein the compound has a structure selected from the group consisting of 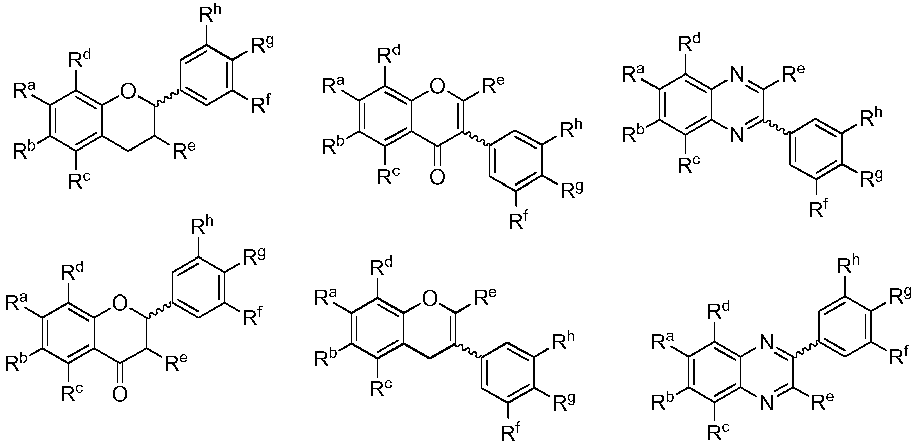 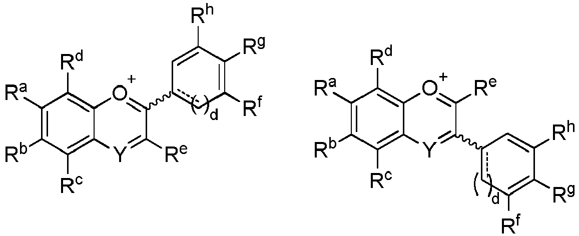 and wherein R^{a}, R^{b}, R^{c}, R^{d}, R^{e}, R^{f}, R^{g} and R^{h} are, independently of each other, selected from the group consisting of H, -O-Methyl, -OH and Methyl, or are, independently of each other, selected from the group consisting of H, -O-Methyl, -OH, -NH₂, -COOH and Methyl.

According to a further embodiment of the present invention, the compound has a structure of the formula (IVa) or (IVb) 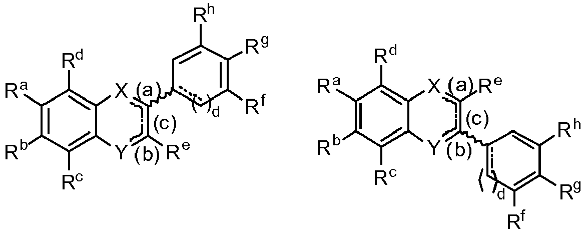 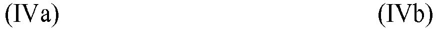 wherein
X is selected from the group consisting of -C(=O)-, -O- and -N-,
Y is selected from the group consisting of -C(H)ₚ-, -C(=O)-, -O- and -N-,
with p being 1 or 2,
the bond (a) is a single or double bond,
the bond (b) is a single or double bond,
the bond (c) is a single or double bond,
and wherein R^{a} and R^{b} are, independently of each other selected from the group consisting of H, Alkyl, halogen and an electron donating group or R^{a} and R^{b} are, independently of each other selected from the group consisting of H, Alkyl, halogene, -C(=Y^{p})R^{r}, and an electron donating group, wherein R^{r} is -OH, -NH₂, -NH-Alkyl, -O-Alkyl, or -S-Alkyl, and wherein Y^{p} is selected from the group consisting of S, O and NH, or wherein R^{a} and R form together a cycloalkyl, cycloheteroalkyl, aryl or heteroaryl, wherein the cycloalkyl, cycloheteroalkyl, aryl or heteroaryl is a single-ring group or a multicyclic group,
and wherein R^{c}, R^{d}, R^{f}, R^{g} and R^{h} are, independently of each other, selected from the group consisting of H, halogene, alkyl and an electron donating group, or wherein R^{c}, R^{d}, R^{f}, R^{g} and R^{h} are, independently of each other, selected from the group consisting of H, Alkyl, halogene, -C(=Y^{p})R^{r}, and an electron donating group, wherein R^{r} is -OH, -NH₂, - NH-Alkyl, -O-Alkyl, or -S-Alkyl, and wherein Y^{p} is selected from the group consisting of S, O and NH
and wherein R^{e} is selected from the group consisting of H, Alkyl, halogen, electron donating group, aryl, heteroaryl group, -C(=Y^{p})R^{r} and -O-C(=O)-R*, wherein R* is an aryl or heteroaryl group,
wherein at least one of R^{a}, R^{b}, R^{c}, R^{d}, R^{f}, R^{g}, R^{e},and R^{h} is an electron donating group, with the proviso that in case the bond (c) is a double bond (a) and (b) are both single bonds, and in case X is -C(=O)-,the bond (a) is a single bond, and wherein in case Y is -C(=O)-, the bond (b) is a single bond. Preferably, the electron donating group is selected from the group consisting of -OH, -SH, -O-R^{p}, -S-R^{p} and -NR^{p}R^{q}, wherein R^{p} and R^{q} are, independently of each other, selected from the group consisting of H, alkyl, cycloalkyl, heterocycloalkyl, aryl and heteroaryl, preferably H or alkyl or cycloalkyl, more preferably H or a linear or branched alkyl group or a cycloalkyl group having from 1 to 10 carbon atoms, preferably an alkyl group or cycloalkyl group selected from the group consisting of methyl, ethyl, propyl, pentyl, butyl, cyclopropyl, cyclobutyl, cyclopropenyl, cyclobutenyl, and hexyl, more preferably H or methyl or ethyl. More preferably at least one of R^{p} and R^{q} is H and one of R^{p} and R^{q} is H or an alkyl group, preferably H or a linear or branched alkyl group having from 1 to 10 carbon atoms, preferably H or an alkyl group selected from the group consisting of methyl, ethyl, propyl, pentyl, butyl and hexyl, more preferably methyl or ethyl. R^{r} is preferably selected from the group consisting of -OH, -NH₂, -NH-Alkyl, -O-Alkyl or -NH-Alkyl. Y^{p} is preferably selected from the group consisting of S, O and NH.
Preferably the at least one electron donation group is -OH, i.e. at least one of R^{a}, R^{b}, R^{c}, R^{d}, R^{f}, R^{g}, R^{e},and R^{h} is -OH. Thus, the present invention also relates to the use of a compound, as described above, wherein the compound has the structure (IVa) or (IVb), wherein at least one of R^{a}, R^{b}, R^{c}, R^{d}, R^{f}, R^{g}, R^{e},and R^{h} is -OH. As described above, the group X in the above-mentioned formulas (IVa) and (IVb) is selected from the group consisting of -C(=O)-, -O- and -N-, preferably X is O or N.

According to a preferred embodiment, R^{a} and R^{b} form a cycloalkyl, cycloheteroalkyl, aryl or heteroaryl, wherein the cycloalkyl, cycloheteroalkyl, aryl or heteroaryl is a single-ring group or a multicyclic group. It is to be understood that the cycloalkyl, cycloheteroalkyl, aryl or heteroaryl may be substituted or unsubstituted. Preferably, the cycloalkyl, cycloheteroalkyl, aryl or heteroaryl is unsubstituted.

Thus, the present invention also relates to a compound having a structure of the formula (IVa) or (IVb), as described above, 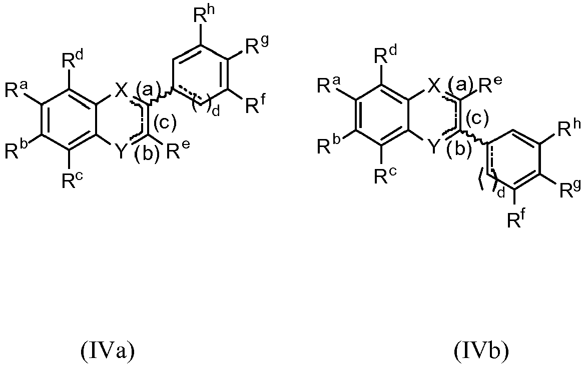 wherein
X is selected from the group consisting of -C(=O)-, -O- and -N-,
Y is selected from the group consisting of -C(H)ₚ-, -C(=O)-, -O- and -N-,
with p being 1 or 2,
the bond (a) is a single or double bond,
the bond (b) is a single or double bond,
the bond (c) is a single or double bond,
and wherein R^{a} and R^{b} form together a cycloalkyl ring, cycloheteroalkyl ring, an aryl ring or a heteroarylring, wherein the cycloalkyl, cycloheteroalkyl, aryl or heteroaryl is a single-ring group or a multicyclic group,
and wherein R^{c}, R^{d}, R^{f}, R^{g} and R^{h} are, independently of each other, selected from the group consisting of H, halogene, alkyl and an electron donating group, or wherein R^{c}, R^{d}, R^{f}, R^{g} and R^{h} are, independently of each other, selected from the group consisting of H, Alkyl, halogene, -C(=Y^{p})R^{r}, and an electron donating group, wherein R^{r} is -OH, -NH₂, - NH-Alkyl, -O-Alkyl, or -S-Alkyl, and wherein Y^{p} is selected from the group consisting of S, O and NH
and wherein R^{e} is selected from the group consisting of H, Alkyl, halogen, electron donating group, aryl, heteroaryl group, -C(=Y^{p})R^{r} and -O-C(=O)-R*, wherein R* is an aryl or heteroaryl group,
wherein at least one of R^{a}, R^{b}, R^{c}, R^{d}, R^{f}, R^{g}, R^{e},and R^{h} is an electron donating group, with the proviso that in case the bond (c) is a double bond (a) and (b) are both single bonds, and in case X is -C(=O)-,the bond (a) is a single bond, and wherein in case Y is -C(=O)-, the bond (b) is a single bond. In particular, the present invention also relates to a compound and the use of said compound, as described above, said compound having a structure selected from the group consisting of: 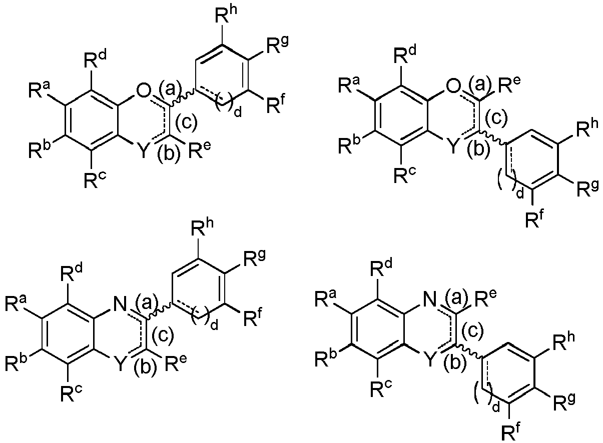 in particular from the group consisting of 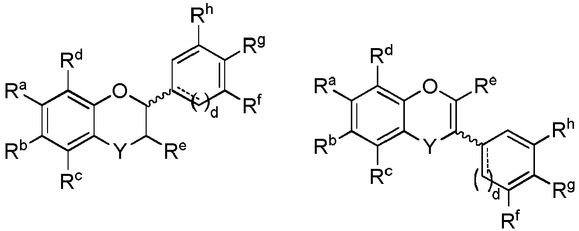 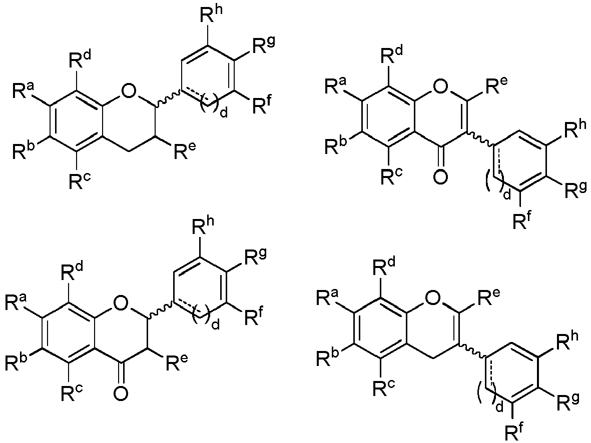 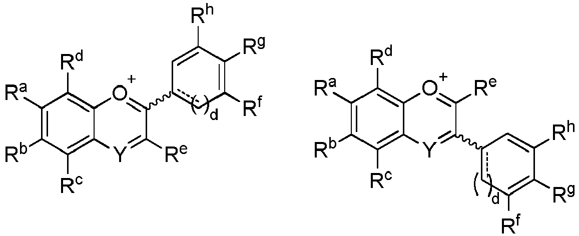 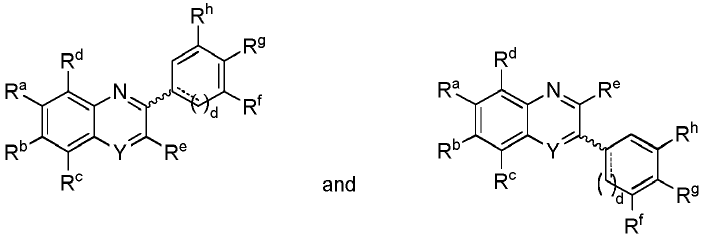 wherein
   X is selected from the group consisting of -C(=O)-, -O- and -N-,
   Y is selected from the group consisting of -C(H)ₚ-, -C(=O)-, -O- and -N-,
   with p being 1 or 2,
   and wherein R^{a} and R^{b} form together a cycloalkyl ring, cycloheteroalkyl ring, an aryl ring or a heteroarylring, wherein the cycloalkyl, cycloheteroalkyl, aryl or heteroaryl is a single-ring group or a multicyclic group,
   and wherein R^{c}, R^{d}, R^{f}, R^{g} and R^{h} are, independently of each other, selected from the group consisting of H, halogene, alkyl and an electron donating group, or wherein R^{c}, R^{d}, R^{f}, R^{g} and R^{h} are, independently of each other, selected from the group consisting of H, Alkyl, halogene, -C(=Y^{p})R^{r}, and an electron donating group, wherein R^{r} is -OH, -NH₂, - NH-Alkyl, -O-Alkyl, or -S-Alkyl, and wherein Y^{p} is selected from the group consisting of S, O and NH
   and wherein R^{e} is selected from the group consisting of H, Alkyl, halogen, electron donating group, aryl, heteroaryl group, -C(=Y^{p})R^{r} and -O-C(=O)-R*, wherein R* is an aryl or heteroaryl group,
   wherein at least one of R^{a}, R^{b}, R^{c}, R^{d}, R^{f}, R^{g}, R^{e},and R^{h} is an electron donating group.

Preferably R^{a} and R^{b} are together -O-CH₂-O- and thus form a 5 membered ring.

By way of example, the following preferred compound is mentioned:

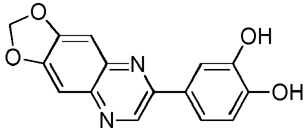

According to a preferred embodiment, R^{e} is selected from the group consisting of aryl, heteroaryl group and -O-C(=O)-R*, wherein R* is an aryl or heteroaryl group. In particular, R^{e} is a substituted or unsubstituted phenyl group or -O-C(=O)-R* with R* being a substituted or unsubstituted phenyl, preferably wherein R^{e} is a group having the structure 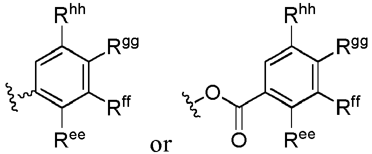 wherein R^{ee}, R^{ff}, R^{gg} and R^{hh} are, independently of each other, selected from the group consisting of H, Alkyl, -C(=Y^{pp})R^{rr}, -O-C(=Y^{pp})R^{rr} and an electron donating group, wherein R^{rr} is -OH, -NH₂, -NH-Alkyl, -O-Alkyl, or -S-Alkyl, and wherein Y^{pp} is selected from the group consisting of S, O and NH. More preferably, the compound has a structure selected from the group consisting of 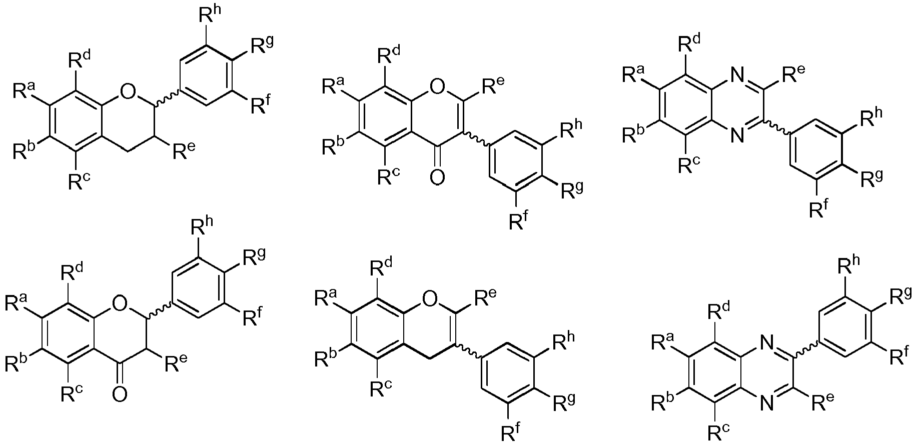 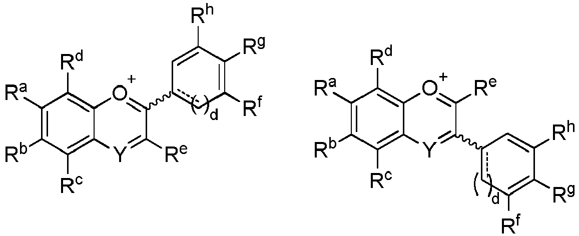 more preferably a structure selected from the group consisting of 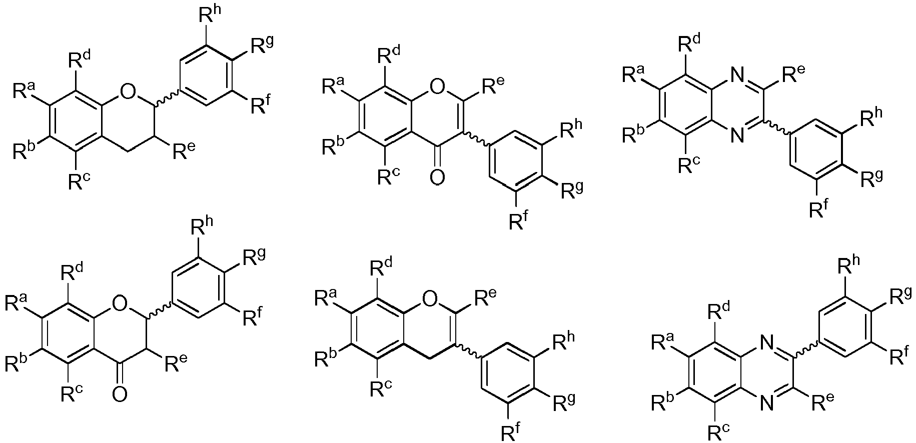 wherein R^{e} is a group having the structure 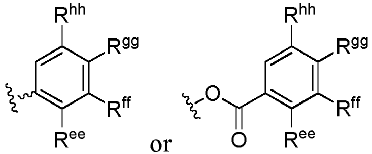 wherein R^{ee}, R^{ff}, R^{gg} and R^{hh} are, independently of each other, selected from the group consisting of H, Alkyl, -C(=Y^{pp})R^{rr}, -O-C(=Y^{pp})R^{rr} and an electron donating group, wherein R^{rr} is -OH, -NH₂, -NH-Alkyl, -O-Alkyl, or -S-Alkyl, and wherein Y^{pp} is selected from the group consisting of S, O and NH. More preferably, R^{a}, R^{b}, R^{c}, R^{d} , R^{f}, R^{g} and R^{h} are, independently of each other, selected from the group consisting of H, - O-Methyl, -OH and Methyl, and R^{ee}, R^{ff}, R^{gg} and R^{hh} are, independently of each other, selected from the group consisting of H, -O-Methyl, -OH and Methyl. By way of example, the following preferred compounds are mentioned:

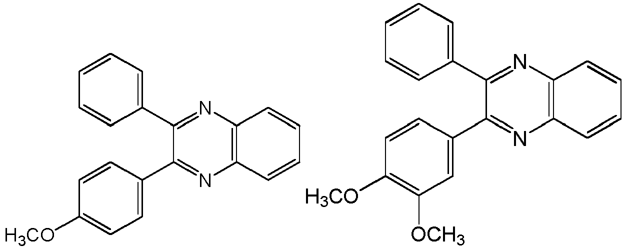

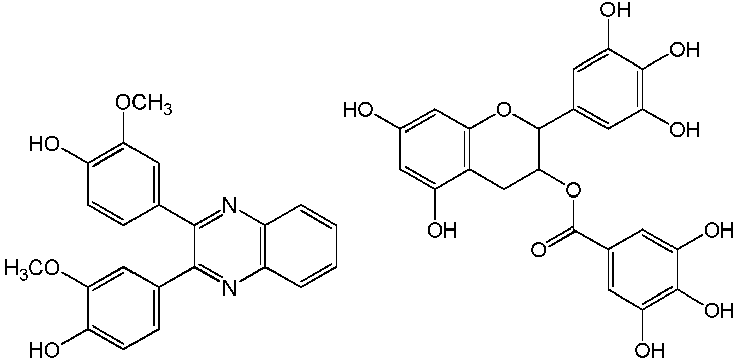

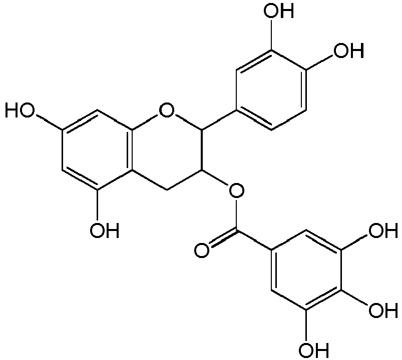

According to a further embodiment of the invention, the compound has a structure of the formula (IV') or (IV") 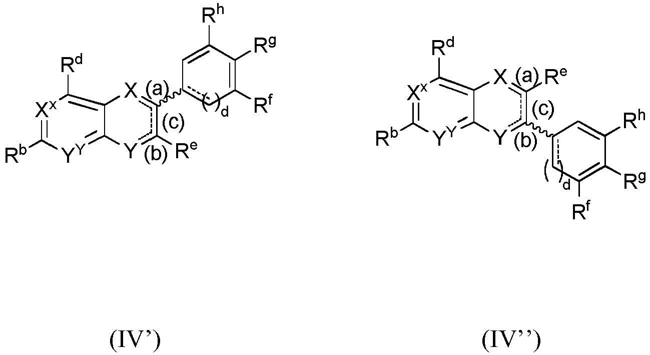 wherein
X is selected from the group consisting of -C(=O)-, -O- and -N-, Y is selected from the group consisting of -C(H)ₚ-, -C(=O)-, -O- and -N-, X^{x} is selected from the group consisting of -C(R^{a})- or N, Y^{y} is selected from the group consisting of -C(R^{c})- or N, wherein
with p being 1 or 2,
the bond (a) is a single or double bond,
the bond (b) is a single or double bond,
the bond (c) is a single or double bond,
and wherein R^{a} and R^{b} are, independently of each other selected from the group consisting of H, Alkyl, halogene, -C(=Y^{p})R^{r}, and an electron donating group, wherein R^{r} is -OH, - NH₂, -NH-Alkyl, -O-Alkyl, or -S-Alkyl, and wherein Y^{p} is selected from the group consisting of S, O, and NH,
or wherein R^{a} and R form together a cycloalkyl, cycloheteroalkyl, aryl or heteroaryl, wherein the cycloalkyl, cycloheteroalkyl, aryl or heteroaryl is a single-ring group or a multicyclic group,
and wherein R^{c}, R^{d}, R^{f}, R^{g} and R^{h} are, independently of each other, selected from the group consisting of H, Alkyl, halogene, -C(=Y^{p})R^{r}, and an electron donating group, wherein R^{r} is -OH, -NH₂, -NH-Alkyl, -O-Alkyl, or -S-Alkyl, and wherein Y^{p} is selected from the group consisting of S, O and NH,
and wherein R^{e} is selected from the group consisting of H, Alkyl, halogene, -O-C(=Y^{p})R^{r}, electron donating group, , aryl, heteroaryl group and -O-C(=O)-R*, wherein R* is an aryl or heteroaryl group, wherein R^{r} is -OH, -NH₂, -NH-Alkyl, -O-Alkyl, or -S-Alkyl, and wherein Y^{p} is selected from the group consisting of S, O and NH and wherein at least one of R^{a}, R^{b}, R^{c}, R^{d}, R^{f}, R^{g}, R^{e}, and R^{h} is an electron donating group, with the proviso that in case the bond (c) is a double bond (a) and (b) are both single bonds, and in case X is -C(=O)-, the bond (a) is a single bond, and wherein in case Y is -C(=O)-, the bond (b) is a single bond. More preferably X^{x} is N and Y^{y} is N, the compound thus having one of the following structures: 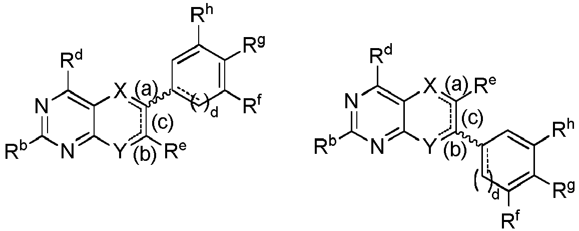 more preferably a structure selected from the group consisting of 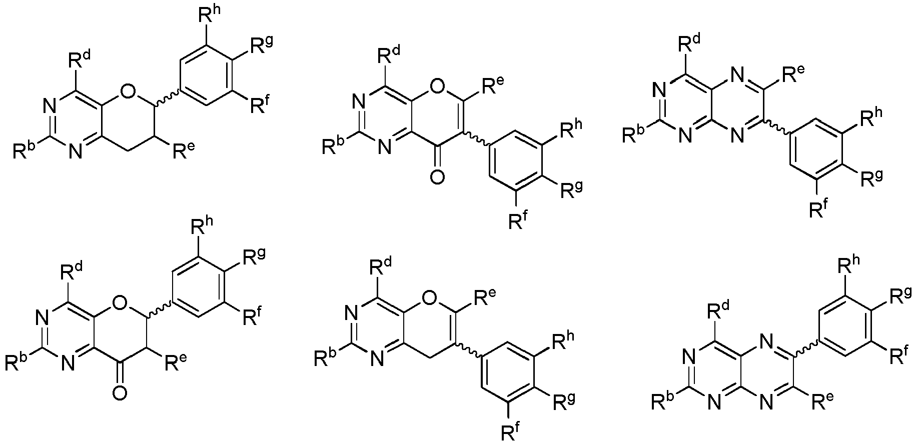 more preferably the structure: 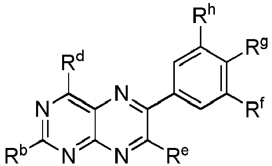 in particular

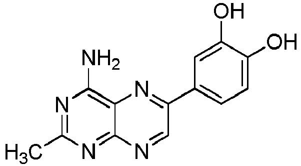

**Table 3: Preferred compounds of the invention, by way of example**

| **Structure** | **R^{a}** | **R^{b}** | **R^{c}** | **R^{d}** | **R^{e}** | **R^{f}** | **R^{g}** | **R^{h}** |
|---|---|---|---|---|---|---|---|---|
| | Selected from the group consisting of | Selected from the group consisting of | Selected from the group consisting of | Selected from the group consisting of | Selected from the group consisting of | Selected from the group consisting of | Selected from the group consisting of | Selected from the group consisting of |
| 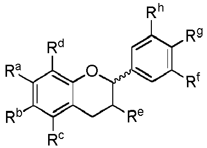 | H, OH | H | H, OH | H | -OH | H, OH | H, OH | H, OH |
| 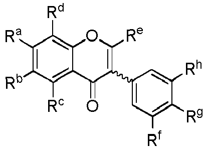 | OH | H, -O-CH₃ | H, OH | H | H | H | OH, -O-CH₃ | H |
| 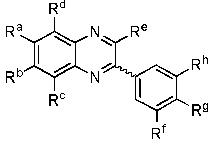 | H, CH_{3:} preferably CH₃ | H, CH₃, preferably CH₃ | H | H | H | OH | OH | H |
| 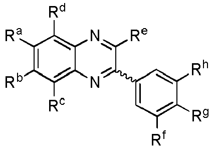 | F | F | H | H | H | OH | OH | OH |
| 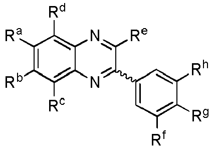 | H | H | H | H | H | H | -O-C(=O)-CH₃ | H |
| 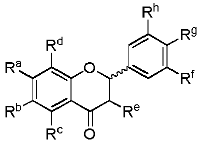 | OH | H | OH | H | H | H | H, OH, -O- CH₃ | H |
| 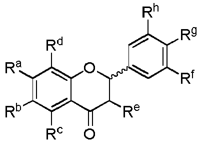 | OH | H | H, OH | H | OH | OH | OH | OH |
| 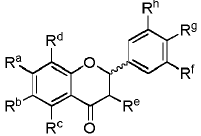 | OH | H | H | H | OH | H, OH | OH | OH |
| 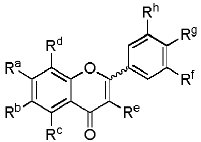 | OH | H | OH | H | H | H | H, OH, -O-CH₃ | H |
| 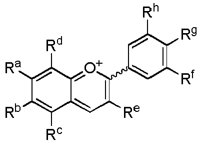 | OH | H | OH | H | OH | -O-CH₃ | OH | -O-CH₃ |
| 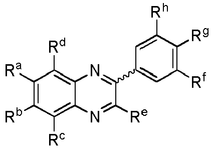 | H, CH₃ | H. CH₃ | H | H | H | H | OH | OH |
| 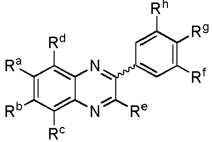 | H, CH₃ | H. CH₃ | H | H | H | OH | OH | H |
| 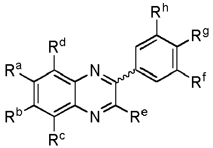 | H | H | H | H | H | H | O-C(=O)-CH₃ | H |

Further, the following compounds are preferred: 2-Hydroxy-5-(6,7,8-trimethyl-quinoxalin-2-yl)-benzoic acid, 3-(6,7-Dimethyl-quinoxalin-2-yl)-6-hydroxy-2-methyl-benzoic acid, 6-Hydroxy-2-methyl-3-(6,7,8-trimethyl-quinoxalin-2-yl)-benzoic acid, 5-(6,7-Dimethyl-4-trifluoromethyl-quinolin-3-yl)-2-hydroxy-benzoic acid, 4-(4-Amino-2-methyl-pteridin-6-yl)-benzene-1,2-diol, 4-(1,3-Dioxa-5,8-diaza-cyclopenta[b]naphthalen-6-yl)-benzene-1,2-diol, 2-Amino-5-(6,7-dimethyl-quinoxalin-2-yl)-3-hydroxy-benzoic acid, 3-(6,7-Dimethylquinoxalin-2-yl)-5-hydroxy-benzoic acid, 2-(3,4-Dihydroxy-phenyl)-quinoxaline-6,7-diol, 2-(3,4-Dihydroxyphenyl)-6,7-dimethylquinoxaline, 5-(6,7-Dimethyl-quinoxalin-2-yl)-benzene-1,3-diol, 2-(6,7-Dimethyl-quinoxalin-2-yl)-benzene-1,4-diol, 5-(6,7-Dimethylquinoxalin-2-yl)-2-hydroxy-benzoic acid, 4-(6,7-dimethylquinoxalin-2-yl)benzene-1,2-diol, 4-(7-Hydroxy-6-methyl-quinoxalin-2-yl)-benzene-1,2-diol, 2-(3,4-Dihydroxyphenyl)-quinoxaline-5,7-diol, -(3,4-dihydroxyphenyl)-3,7-dihydroxy-2,3-dihydrochromen-4-one (Fustin), 3,7-Dihydroxy-2-(3,4,5-trihydroxy-phenyl)-chroman-4-one, 3,5,7-Trihydroxy-2-(3,4,5-trihydroxy-phenyl)-chroman-4-one, and 4-(quinoxalin-2-yl)phenyl acetate

According to an alternative embodiment, the compound is selected from the group consisting of: 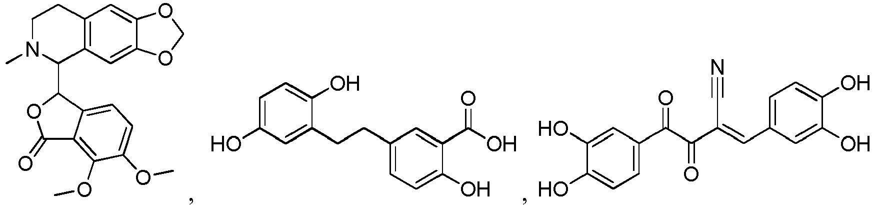 and

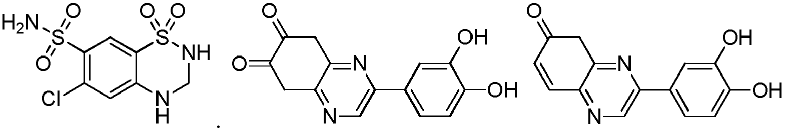

### Pharmaceutically acceptable salt

As described above, the compounds of the present invention can be formulated as pharmaceutically acceptable salt or solvate.

Typical pharmaceutically acceptable salts include those salts prepared by reaction of the compounds of the present invention with a pharmaceutically acceptable mineral or organic acid or an organic or inorganic base. Such salts are known as acid addition and base addition salts. Acids commonly employed to form acid addition salts are inorganic acids such as hydrochloric acid, hydrobromic acid, hydroiodic acid, sulfuric acid, phosphoric acid, and organic acids such as p-toluenesulfonic acid, methanesulfonic acid, oxalic acid, p-bromophenylsulfonic acid, carbonic acid, succinic acid, citric acid, benzoic acid, acetic acid. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprylate, acrylate, formate, hydrochloride, dihydrochloride, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, xylenesulfonate, phenylacetate, phenylpropionate, phenylbutyrate, citrate, lactate, gammahydroxybutyrate, glycolate, tartrate, methanesulfonate, propanesulfonate, naphthalene-1-sulfonate, napththalene-2-sulfonate, mandelate. Preferred pharmaceutically acceptable acid addition salts are those formed with mineral acids such as hydrochloric acid and hydrobromic acid, and those formed with organic acids such as maleic acid and methanesulfonic acid. Salts of amine groups may also comprise quaternary ammonium salts in which the amino nitrogen carries a suitable organic group such as an alkyl, alkenyl, alkynyl, or aralkyl moiety. Base addition salts include those derived from inorganic bases, such as ammonium or alkali or alkaline earth metal hydroxides, carbonates, bicarbonates. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate. The potassium and sodium salt forms are particularly preferred. It should be recognized that the particular counter ion forming a part of any salt of this invention is usually not of a critical nature, so long as the salt as a whole is pharmacologically acceptable and as long as the counter ion does not contribute undesired qualities to the salt as a whole.

The term acceptable solvate encompasses also pharmaceutically acceptable solvates of the compounds of the invention, wherein the compound combines with a solvent such as water, methanol, ethanol or acetonitrile to form a pharmaceutically acceptable solvate such as the corresponding hydrate, methanolate, ethanolate or acetonitrilate.

Moreover, the disclosure relates to a method for identifying a C4 plant selective herbicide, comprising screening a compound library for a compound, which is
i) capable of binding to the malate binding site comprised by a phosphoenolpyruvate carboxylase from a C4 plant, thereby inhibiting the activity of said phosphoenolpyruvate carboxylase, and
ii) not capable of binding to the malate binding site comprised by a phosphoenolpyruvate carboxylase from a C3 plant.

A compound which is capable of binding to the malate binding site comprised by the phosphoenolpyruvate carboxylase from a C4 plant, but which is not capable of binding to the malate binding site comprised by the phosphoenolpyruvate carboxylase from a C3 plant, preferably, is a candidate compound that can be used as a C4 selective herbicide.

The term "herbicide" is well understood in the art. As used herein, the term refers to a compound that inhibits the growth of plants. The inhibition of the growth of plants shall include all deviations from natural development. In particular, the term refers to reducing the growth of the plant or to kill a plant. Moreover, the term may include inhibition of the development. Preferably, the term further includes dwarfing.

The herbicide to be identified by the method of the present invention shall be a C4 plant selective herbicide. A C4 plant selective herbicide, preferably, shall inhibit the growth of a C4 plant, but shall not inhibit the growth of a C3 plant. Also preferably, a C4 plant selective herbicide shall significantly inhibit the growth of a C4 plant, whereas growth of C3 plant shall remain unchanged, in particular essentially unchanged, in the presence of said herbicide (as compared to the absence of the herbicide). Whether growth of a plant is essentially unchanged or significantly inhibited can be determined by the skilled person without further ado.

As set forth above, the term "inhibiting the growth" also includes a "reducing the growth". Accordingly, it is envisaged that the growth of a C4 shall be reduced in the presence of said herbicide (in particular in the presence of an effective amount) as compared to the growth in the in the absence of said herbicide. In contrast, the growth of a C3 plant shall not be affected by the presence of the herbicide, in particular, shall not be significantly affected in the presence of said herbicide. Accordingly, a C3 plant, preferably, shows the same growth (in particular, essentially the same growth), regardless whether the herbicide is present or not. Thus, it is envisaged, that a C4 plant selective herbicide shall reduce the growth of a C4 plant, but shall not reduce the growth of a C3 plants.

It is to be understood that the inhibition of the growth of the C4 plant may dependent on the concentration. Preferably, the growth of the C4 plant is inhibited if the plant is contacted with an effective amount of the herbicide, i.e.. with a herbicidal effective amount. Whether an amount of the herbicide is effective, or not, can be determined by methods known in the art.

The terms "C3 plant" and "C4 plant" are well understood by the skilled person.

A C3 plant, in particular, shall be a plant in which the CO₂ is first fixed into a compound containing three carbon atoms (phosphoglyceric acid) before entering the Calvin cycle of photosynthesis. Preferably, it uses the C3 carbon fixation pathway as the sole mechanis to covert CO₂ into 3-phosphoglycerate. C3 plants are well known in the art and include tobacco, tomato, soybeans, potato, cucumber, cotton, wheat, rice and barley.

Particularly preferred C3 plants are Oryza sativa, Hordeum vulgare, Brassica napus, Triticum aestivum, and, in particular, Flaveria pringlei. Further preferred are Glycine max (soybean), cucumber, grapes, potato, tomato, cassava, sugar beet und watermelon. Moreover, it is also preferred that the C3 plant is selected from the group of C3 plants consisting of asparagus, green beans, cabbages and other brassicas, carrots and turnips, cassava, cauliflowers and broccoli, green chilies and peppers, cucumbers and gherkins, eggplants, grapes, lettuce and chicory, pumpkins, squash and gourds, spinach, sugar beet, sunflower seed, watermelons, and yams.

A C4 is plant in which the CO₂ is first fixed into a compound containing four carbon atoms before entering the Calvin cycle of photosynthesis. In particular, a C4 plant shall utilize the C4 carbon fixation pathway in which the CO₂ is first bound to a phosphoenolpyruvate resulting in the formation of four-carbon compound (oxaloacetate). C4 plants are well known in the art and include monocotyledonous plants such as maize, sugarcane, and sorghum, as well as dicotyledonous plants such as Amaranthus.

Preferably, the C4 plants belong to the C4 plants of the family selected from Aizoaceae (Genus, in particular, Cypselea, Gisekia, Trianthema, Zalaeya), Amaranthaceae (Acanthochiton, Aerva, Alteranthera, Amaranthus, Brayulinea), Caryophyllaceae (Polycarpaea), Chenopodiacea (Anabis, Aellenia, Arthrophytum, Atriplex, Bassia, Bienerta, Camphorosma, Chenolea, Climacoptera, Comulaca, Cytobasis, Echinopsilon, Gamanthus, Girgensohnia, Halanthium, Halimocnemis, Halocharis, Halogeton, Halostigmaria, Haloxylon, Hammada, Horaninovia, Hypocyclix, Kochia, Londesia, Noaea, Panderia, Petrosimonia, Salsola, Seidlitzia, Suaeda, Theleophyton, Traganum) Molluginaceae (Glinis, Mollugo) Nyctaginaceae (Ilionia, Boerhaavia, Okenia), Portulaceae (Portulaca), Polygonaceae (Calligonum), Euphorbiaceae (Chamaesyce, Euphorbia) Capparaceae (Gynandropsis), Zygophyllaceae (Kallstroemia, Tribulus, Zygophyllum) Asteraceae (Glossocordia, Glossogyne, Isostigma, Pectis), Boraginaceae, Convolvulaceae, Acanthaceae, Scrophulariaceae, Poaceae (Alloteropsis, Andropogon, Arundinella, Bouteloua, Cynodon, Echinochloa, Leptochloa, Microstegium, Panicum, Paspalum, Setaria, Sorghum, Spartina, Sporobolus, Zea). Preferred genera are indicated in brackets.

Particularly preferred C4 plants are selected from the group consisting of Bothriochloa saccharoides, Bothriochloa ischaemum, Imperata cylindrica (Cogon grass), Panicum capillare (Witchgrass), Panicum coloratum, Panicum fluviicola, Panicum miliaceum, Panicum phragmitoides, Panicum turgidum, Saccharum officinarum (Wild sugarcane), Sorghum bicolor, Zea mays (Maize), in particular Flaveria trinervia, Setaria italica (Foxtail millet), Setaria palmifolia, Setaria plicata, Paspalum conjugatum (Buffalo Gras), Paspalum quadrifarium, Paspalum dilatatum, Amaranthus hypochondriacus, Amaranthus spinosus (spiny amaranth), Sorghum halepense (Johnsongrass), Rottboellia cochinchinensis (itchgrass), Commelina benghalensis (tropical spiderwort), Trianthema portulacastrum (desert horse purslane), Ageratum conyzoides (Chick weed, Goatweed, Whiteweed), Bidens pilosa (Spanish Needle), Euphorbia hirta, Portulaca oleracea (Pigweed). Further preferred C4 plants are Alopecurues myosuroides and Gallium aparine. Particularly preferred C4 plants are Flaveria trinervia, Flaveria australasica, Zea mays, and Sorghum bicolor.

The phosphoenolpyruvate carboxylases to be used in the context of the method of the present invention shall be derived from a C3 and from a C4 plant, respectively. Thus, it is particular envisaged that the phosphoenolpyruvate carboxylase to be used is naturally present in a C3 and a C4 plant, respectively. Preferred C3 and C4 plants are disclosed herein above.

The term "phosphoenolpyruvate carboxylase" is well understood by the skilled person. Frequently, phosphoenolpyruvate carboxylases are also referred to as PEP carboxylase, PEPCase, or PEPC. A phosphoenolpyruvate carboxylase as referred to herein shall be capable of catalyzing the formation of oxaloacetate from phosphoenolpyruvate (PEP) and bicarbonate (EC 4.1.1.31). In particular, a phosphoenolpyruvate carboxylase shall be capable of catalyzing the irreversible beta-carboxylation of phosphoenolpyruvate by bicarbonate to yield oxalacetate and phosphate. The aforementioned enzymatic activity can be determined by assays well known in the art.

It is well known in the art, that the regulation of the PEP carboxylase is, inter alia, effected by allosteric effectors, L-aspartate and L-malate. L-aspartate or L-malate bind at the enzyme to an allosteric binding domain, the malate binding site (also called "aspartate binding site" or "aspartate/malate binding site"), thereby inhibiting the activity of the PEP carboxylase. The malate binding site of a PEP carboxylase comprises four conserved residues which participate directly in the binding of malate and aspartate. The conserved residues are well known in the art, and e.g. described by Jacobs et al. (Plant Cell and Enviroment (2008), 31, 793-803). In Flaveria, the four conserved residue which participate in the binding of aspartate/malate are R641, K829, R888 and N964 (see the sequences disclosed herein for the PEPC from Flaveria pringlei und trinervia.

In a preferred embodiment of the present invention the PEP carboxylase from a C4 plant is derived from Flaveria trinervia (GenBank Accession Number CAA43601.1 GI:498699), and the PEP carboxylase from a C3 plant is derived from Flaveria pringlei (GenBank Accession number CAA45505.1 GI:18458), i.e. two highly homologous PEP carboxylases.

The amino acid sequence of the PEP carboxylase from Flaveria trinervia is shown in SEQ ID NO: 1, the amino acid sequence of the PEP carboxylase from Flaveria pringlei is shown in SEQ ID NO: 2.

Thus, in a preferred embodiment, the phosphoenolpyruvate carboxylase derived from a C4 plant is, in particular, encoded by a polynucleotide comprising a nucleic acid selected from the group consisting of:
a. a nucleic acid encoding a polypeptide having an amino acid sequence as shown in SEQ ID No: 1;
b. a nucleic acid encoding a polypeptide having an amino acid sequence being at least 90%, 95%, 97% or in particular 99% identical to the amino acid sequence shown in SEQ ID No: 1, wherein said polypeptide has phosphoenolpyruvate carboxylase activity.

The enzymatic activity of a PEP carboxylase is disclosed elsewhere herein. Thus, the phosphoenolpyruvate carboxylase shall be capable of catalyzing the formation of oxaloacetate from phosphoenolpyruvate (PEP) and bicarbonate (EC 4.1.1.31, see above). Preferably, the phosphoenolpyruvate carboxylase derived from a C4, comprises, within its malate binding site, a glycine residue which corresponds to the conserved glycine residue found at position 884 of the PEP carboxylase from Flaveria pringlei. Moreover, the inventors have found that some of the PEP carboxylases from C4 plants comprise at a position which corresponds to the glycine residue, a serine, glutamine or isoleucine residue, i.e. amino acid residues which will also not inhibit binding of the identified herbicides by their side-chain. Alternatively, the said phosphoenolpyruvate carboxylase derived from a C4, comprises, within its malate binding site, a serine, glutamine or isoleucine residue which corresponds to said glycine residue. The malate binding site of the PEP Carboxylase from Flaveria trinervia is well known in the art, and, e.g. described by Jacobs et al. (Plant Cell and Enviroment (2008), 31, 793-803). Preferably, the variants of the phosphoenolpyruvate carboxylase set forth in c. and d. above are capable of binding malate.

Thus, in a preferred embodiment, the phosphoenolpyruvate carboxylase derived from a C3 plant is, in particular, encoded by a polynucleotide comprising a nucleic acid selected from the group consisting of:
a. a nucleic acid encoding a polypeptide having an amino acid sequence as shown in SEQ ID No: 2;
b. a nucleic acid encoding a polypeptide having an amino acid sequence being at least 90%, 95%, 97% or in particular 99% identical to the amino acid sequence shown in SEQ ID No: 2, wherein said polypeptide has phosphoenolpyruvate carboxylase activity.

The activity of a PEP carboxylase is disclosed elsewhere herein. Preferably, the phosphoenolpyruvate carboxylase derived from a C3, comprises, within its malate binding site, a conserved arginine residue which corresponds to the conserved arginine residue found at position 884 of the PEP carboxylase from Flaveria pringlei. Preferably, the conserved arginine residue is within the region of amino acid 875 to 895. The malate binding site of the PEP Carboxylase from Flaveria pringlei is well known in the art, and, e.g. described by Jacobs et al. (Plant Cell and Enviroment (2008), 31, 793-803), which is herewith incorporated by reference with respect to its entire disclosure content. Preferably, the variants of the phosphoenolpyruvate carboxylase set forth in b. above are capable of binding malate.

The term "polynucleotide" as used herein refers to a linear or circular nucleic acid molecule. It encompasses DNA as well as RNA molecules. The polynucleotide of the present invention shall be provided, preferably, either as an isolated polynucleotide (i.e. isolated from its natural context) or in genetically modified form. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides. The polynucleotide of the present invention is characterized in that it shall encode a polypeptide as referred to above. The polynucleotide, preferably, has a specific nucleotide sequence as mentioned above. Moreover, due to the degeneracy of the genetic code, polynucleotides are encompassed which encode a specific amino acid sequence as recited above.

Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence characterized in that the sequence can be derived from the aforementioned specific nucleic acid sequences by at least one nucleotide substitution, addition and/or deletion whereby the variant nucleic acid sequence shall still encode a polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 x sodium chloride/sodium citrate (= SSC) at approximately 45°C, followed by one or more wash steps in 0.2 x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 x SSC (pH 7.2). If organic solvent is present in the abovementioned buffer, for example 50% formamide, the temperature under standard conditions is approximately 42°C. The hybridization conditions for DNA:DNA hybrids are preferably for example 0.1 x SSC and 20°C to 45°C, preferably between 30°C and 45°C. The hybridization conditions for DNA:RNA hybrids are preferably, for example, 0.1 x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer-based amplification of DNA, i.e. using degenerated primers against conserved domains of the polypeptides of the present invention. Conserved domains of the polypeptide of the present invention may be identified by a sequence comparison of the nucleic acid sequence of the polynucleotide or the amino acid sequence of the polypeptide of the present invention with sequences of other members of the enzyme families referred to in accordance with this invention. Oligonucleotides suitable as PCR primers as well as suitable PCR conditions are described in the accompanying Examples. As a template, DNA or cDNA from bacteria, fungi, plants or animals may be used. Further, variants include polynucleotides comprising nucleic acid sequences which are, in increasing order of preference, at least 70%, at least 75%, at least 80%, at least 85%, at least 90%, at least 95%, at least 98% or at least 99% identical to the specific nucleic acid sequences. Moreover, also encompassed are polynucleotides which comprise nucleic acid sequences encoding amino acid sequences which are, in increasing order of preference, at least 70%, at least 75%, at least 80%, at least 85%, at least 90%, at least 95%, at least 98% or at least 99% identical to the specific amino acid sequences referred to herein. The percent identity values as set forth herein are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS, 5 1989: 151-153) or the programs Gap and BestFit (Needleman 1970, J. Mol. Biol. 48; 443-453 and Smith 198, Adv. Appl. Math. 2; 482-489), which are part of the GCG software packet from Genetics Computer Group, 575 Science Drive, Madison, Wisconsin, USA 53711, version 1991, are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the aforementioned nucleic acid sequences is also encompassed as a polynucleotide of the present invention. The fragment shall encode a polypeptide which still has the activity as specified above. Accordingly, the polypeptide may comprise or consist of the domains of the polypeptide of the present invention conferring the said biological activity. A fragment as meant herein, preferably, comprises at least 50, at least 100, at least 250 or at least 500 consecutive nucleotides of any one of the aforementioned nucleic acid sequences or encodes an amino acid sequence comprising at least 20, at least 30, at least 50, at least 80, at least 100 or at least 150 consecutive amino acids of any one of the aforementioned amino acid sequences.

Further preferred PEP carboxylases from C3 plants are derived from the following plants (indicated is the organism, the GenBank Accession Nummer, and the position of the conserved arginine residue. For some enzymes, only the sequences of fragments are known, the full length sequences can be determined by the skilled person without further ado):

| | |
|---|---|
| Oryza sativa (Rice) EMBL AAG00180.1 | R879 |
| Brassica napus (Rape) TrEMBL Q42634 | R883 |
| Triticum aestivum (Wheat) EMBL CAA07610.1 | R891 |
| Phaseolus vulgaris (Kidney bean) Q9AU12 | R887 |
| Hordeum vulgare (Barley) EMBL ABB01326.1 (Fragment) | R146 |
| Vicia faba (Broad bean) (Faba vulgaris) EMBL CAA09588.1 | R885 |
| Cucumis sativus (Cucumber) EMBL CAD10147.1 (Fragment) | R117 |
| Vitis vinifera (Grape) EMBL AAL83719.1 (Fragment) | R258 |
| Solanum tuberosum (Potato) P29196 | R885 |
| Gossypium hirsutum (Upland cotton) EMBL AAB80714.1 | R884 |
| Glycine max (Soybean) P51061 [UniParc] | R886 |
| Nicotiana tabacum (Common tobacco) P27154 [UniParc] | R884 |

Further preferred PEP carboxylases from C3 plants are derived from Hordeum vulgare (BAJ88050.1 GI:326516054) or from Arabidopsis thaliana (AAC24594.1 GI:3264805)

Further preferred PEP carboxylases from C4 plants are derived from the following plants (indicated is the organism, the GenBank Accession Nummer, and the position of the conserved glycine residue, alternatively of the serine, glutamine or isoleucine, see elsewhere herein. For some enyzmes, only the sequences of fragments are known, the full length sequences can be determined by the skilled person without further ado):

| | |
|---|---|
| Bothriochloa saccharoides (Fragment) TrEMBL A7DX44 | G429 |
| Bothriochloa ischaemum (Fragment) TrEMBL A7DX82 | G429 |
| Imperata cylindrica (Cogon grass) Fragment) TrEMBL A7DX63 | G429 |
| Panicum capillare (Witchgrass) (Fragment) EMBL CAM84110.1 | G429 |
| Panicum coloratum (Fragment) TrEMBL A7DXB1 | G429 |
| Panicum fluviicola TrEMBL (Fragment) D9UAH3 | G721 |
| Panicum miliaceum (Fragment) TrEMBL E1XUD1 | G721 |
| Panicum phragmitoides (Fragment) TrEMBL D9UAH8 | G721 |
| Panicum turgidum (Fragment) TrEMBL D9UAH5 | G721 |
| Saccharum officinarum (Wild sugarcane) EMBL CAC85930.1 | G881 |
| Sorghum bicolor Swiss-prot P15804 | G881 |
| Zea mays (Maize) EMBL CAA33317.1 | G890 |
| Flaveria trinervia Swiss-Prot P30694 | G884 |
| Panicum laetum (Fragment) EMBL CAM84117.1 | S429 |
| Setaria italica (Foxtail millet) TrEMBL Q8S2Z8 | Q884 |
| Setaria palmifolia (Fragment) TrEMBL A7DXC5 | Q429 |
| Setaria plicata (Fragment) TrEMBL A7DXC6 | Q429 |
| Paspalum conjugatum (Buffalo Gras) (Fragment) TrEMBL A7DXB6 | Q429 |
| Paspalum quadrifarium (Fragment) EMBL CAM84120.1 | Q429 |
| Paspalum dilatatum (Fragment) TrEMBL A7DXB7 | Q429 |
| Amaranthus hypochondriacus SwissProt Q43299 | I884 |

Preferably, the compound which is capable of binding to the malate binding site comprised by the phosphoenolpyruvate carboxylase from a C4 plant, but which is not capable of binding to the malate binding site comprised by the phosphoenolpyruvate carboxylase from a C3 plant does not bind to the malate binding site comprised by a phosphoenolpyruvate carboxylase from a C3 plant due to steric and/or electrostatic interactions with the conserved arginine residue comprised by said malate binding site. It is particularly envisaged that the said compound does not bind to the malate binding site due to steric interaction with the conserved arginine residue comprised by said malate binding site. Accordingly, binding of said compound is, preferably, inhibited by the presence of said conserved arginine residue.

The assessment whether a compound binds to a malate binding site can be carried out in vivo or in vitro. Methods for carrying out this assessment are well known in the art, and include Isothermal Titration Calorimetry (ITC) for measuring biomolecular interactions as disclosed by Chaires J. B. (2008) Calorimetry and thermodynamics in drug design. Annu Rev Biophys 37, 135-151. Further preferred methods are disclosed by Cooper M. A. (2003) Label-free screening of bio-molecular interactions. Anal Bioanal Chem 377, 834-842.

According to the disclosure, the assessment whether said compound binds to said malate binding site of phosphoenolpyruvate carboxylase from said C3 or C4 plant is done by determining the enzymatic activity of said (C3 or C4) phosphoenolpyruvate carboxylase after contacting the compound with the phosphoenolpyruvate carboxylase. Preferably, a compound binds to the malate binding site of said PEP carboxylase (in particular of a C4 PEP carboxylase) if the activity of said PEP carboxylase is reduced i) after contacting said compound with said PEP carboxylase and/or ii) in comparison of a PEP carboxylase that has not been contacted with the compound. Preferably, a compound does not bind to the malate binding site of said PEP carboxylase (in particular of a C3 PEP carboxylase) if the activity of said PEP carboxylase is unchanged, in particular, essentially unchanged i) after contacting said compound with said PEP carboxylase and/or ii) in comparison of a PEP carboxylase that has not been contacted with the compound.

How to determine the activity of a PEP carboxylase is well known in the art. E.g, the formation of oxaloacetate can be determined spectrophotometrically, e.g., in a malate dehydrogenase coupled system. The reaction velocity can be measured as a decrease in A₃₄₀ resulting from the oxidation of NADH.

The figures show:

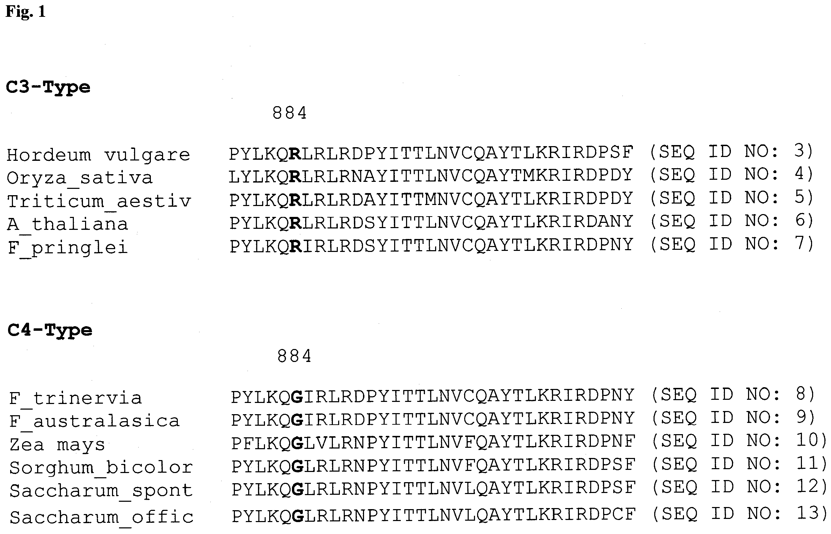

**Fig. 1****:** Partial alignment of PEP carboxlyases derived from various C3 and C4 plants. Indicated are the conserved arginine residues in C3 PEP carboxylases, and the conserved glycine residues in C4 PEP carboxylases.
**Fig.:2****:** Selective Inhibition of a C4-PEPC by (+)-catechin (measured at a concentration of 50 micromolar and 500 micromolar. In table 2 the IC50 value determined from inhibition studies at different concentrations of (+)-catechin is given. Experiments were done in triplicate with at least seven different concentrations of the inhibitor.)
**Fig.:3****:** Selective Inhibition of a C4-PEPC by folic acid (measured at a concentration of 0.025 mM)

The following Examples shall merely illustrate the invention. They shall not be construed, whatsoever, to limit the scope of the invention.

### Example 1:

The histidine-tagged PEP carboxylase from *Flaveria pringlei* or from *Flaveria trinervia* was heterologously expressed in *E*. *coli* and purified via immobilized metal affinity chromatography (IMAC). Crystals of the C3 (*F*. *pringlei*) and the C4 (*F. trinervia*) PEP carboxylase were obtained from the purified proteins by micro batch vapor diffusion. Crystals where analyzed by synchrotron radiation and PEPC structures were determined from the diffraction data by molecular replacement using XDS, coot and the CCP4 suite (Kabsch, 2010; Emsley *et al*. 2004; CCP4, 1994).

Crystal structures of PEP carboxylase from *Flaveria pringlei* or from *Flaveria trinervia* were superimposed by the align algorithm of PyMOL (DeLano, 2002) which performs a BLAST-like BLOSUM62-weighted dynamic programming sequence alignment followed by a series of refinement cycles intended to improve the fit by eliminating pairing with high relative variability. The malate binding site in the aligned crystal structures was visually inspected for structural differences in the region of 10-15 Å around the bound aspartate inhibitor. From this alignment the structural difference in position 884 was identified. The C3 type PEP carboxylase of *Flaveria pringlei* has a voluminous arginine side chain in this position, while the C4 type PEP carboxylase of *Flaveria trinervia* carries a small glycine residue in the corresponding position.

Potential selective inhibitors of C4 PEP carboxylase were selected from a Virtual Drug Screening (VDS) approach using the program PyRX (Wolf, 2009), standard compound libraries (ChemBank, NCI DataBase, KEGG Database), a library assembled from the Plant Metabolome Database and the high resolution crystal structures of PEP carboxylases from *F. pringlei* and *F. trinervia.*

In particular, the following potential selective inhibitors of C4 PEP carboxylase were identified:
1. (+)-Catechin [(2R,3S)-2-(3,4-dihydroxyphenyl)-3,4-dihydro-2H-chromene-3,5,7-triol]
2. Epigallocatechin gallate
3. 2-(4-methoxy-phenyl)-3-phenylquinoxaline
4. Folic Acid
5. 1-(3'-carboxy-4'-hydroxyphenyl)-2-(2,5-dihydroxyphenyl)ethane
6. 1(R),9(S)-hydrastine
7. 1(S),9(R)-beta-hydrastine
8. 2-(3,4-dihydroxyphenyl)-3,5,7-chromanetriol
9. 2-(3,4-Dihydroxyphenyl)-6,7-dimethylquinoxaline
10. 4-((12,20-dioxopregnan-3-yl)oxy)-4-oxobutanoic acid
11. Acacetin [5,7-dihydroxy-2-(4-methoxyphenyl)chromen-4-one]
12. Afrormosin
13. Apigenin [5,7-Dihydroxy-2-(4-hydroxyphenyl)-4H-1-benzopyran-4-one]
14. Chrysin [5,7-dihydroxy-2-phenyl-4H-chromen-4-one]
15. Daidzein [7-Hydroxy-3-(4-hydroxyphenyl) chromen-4-one]
16. Genistein [5,7-Dihydroxy-3-(4-hydroxyphenyl)chromen-4-one]
17. Hydrochlorothiazide
18. Malvidin [3,5,7-trihydroxy-2-(4-hydroxy- 3,5-dimethoxyphenyl)chromenium]
19. N-((1.3-dioxo-1.3-dihydro-2H-isoindol-2-yl)acetyl)aspartic acid
20. N-((4-(((2.4-diamino-6-pteridinyl)methyl)amino)phenyl)acetyl)aspartic acid
21. N-((4-(((2-amino-4-hydroxy-6-pteridinyl)methyl)amino)phenyl)acetyl)glutamic acid
22. N-((5-(((2-amino-4-hydroxy-6-pteridinyl)methyl)amino)-2-thienyl)carbonyl)glutamic acid
23. N-([1.1'-biphenyl]-4-ylcarbonyl)glutamic acid
24. N-(2-quinoxalinylcarbonyl)aspartic acid
25. N-(2-quinoxalinylcarbonyl)glutamic acid
26. N-(4-((((2.4-diamino-6-pteridinyl)methyl)(methyl)amino)methyl)benzoyl)glutamic acid
27. N-(4-(((2,4-diamino-6-pteridinyl)methyl)amino)-3-methylbenzoyl)glutamic acid
28. N-(4-(((2,4-diamino-6-pteridinyl)methyl)amino)benzoyl)glutamic acid
29. N-(4-(((2,4-diamino-6-quinazolinyl)methyl)(methyl)amino)benzoyl)glutamic acid
30. N-(4-(((2,6-diamino-9H-purin-8-yl)methyl)(methyl)amino)benzoyl)glutamic acid
31. N-(4-(((2.4-diamino-6-pteridinyl)methyl)(methyl)amino)-3-iodobenzoyl)glutamic acid
32. N-(4-(((2-amino-4-hydrox-6-pteridinyl)methyl)amino)-3-iodobenzoyl)glutamic acid
33. N-(4-((2-(2.4-diamino-6-pteridinyl)ethyl)(methyl)amino)benzoyl)glutamic acid
34. N-(4-((2-(2-amino-4-hydroxy-6-pteridinyl)ethyl)(methyl)amino)benzoyl)glutamic acid
35. Naringenin [5,7-dihydroxy-2-(4-hydroxyphenyl)chroman-4-one]
36. Tectorigenin [5,7-dihydroxy-3-(4-hydroxyphenyl)-6-methoxychromen-4-one]
37. α-Cyano-(3,4-dihydroxy)cinnamoyl-(3',4'-dihydroxyphenyl) ketone

### Example 2: Confirmation of selective inhibition by (+)-catechin

Inhibition of purified PEP carboxylases from *F. pringlei* and *F. trinervia* by compounds selected from the VDS was monitored by a spectrophotometric coupled assay with malate dehydrogenase which reduces the oxaloacetate formed by PEP carboxylase to malate. The simultaneous oxidation of NADH is followed at 340 nm with standard optical equipment. The concentration of the inhibitors selected from the VDS was varied in the coupled assay to determine the half maximal inhibitory concentration (IC50).

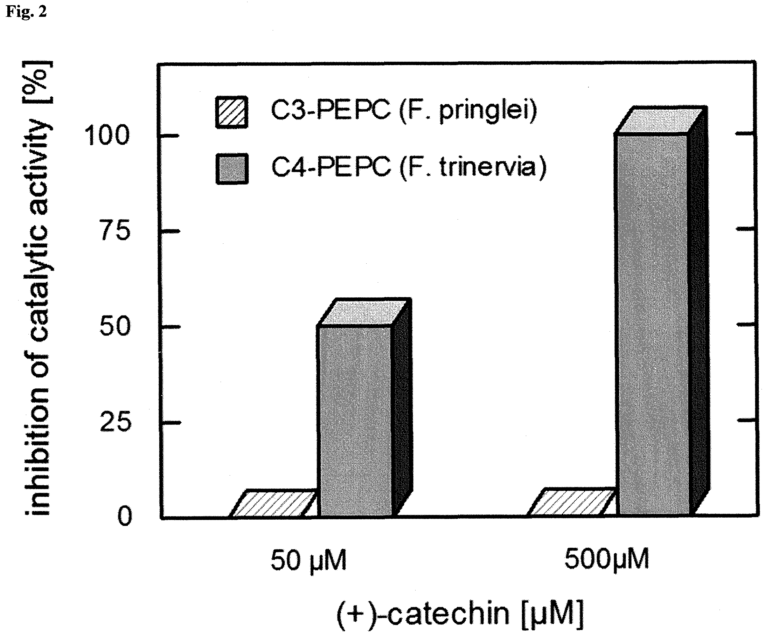

With (+)-catechin the inhibition shown in Fig. 2 of the PEP carboxylase activity of the C3 and the C4 enzyme was obtained. Further results are given in table 4.

### Example 3: Confirmation of selective inhibition by folic acid

Inhibition of purified PEP carboxylases from *F. pringlei* and *F. trinervia* by compounds selected from the VDS was monitored by a spectrophotometric coupled assay with malate dehydrogenase which reduces the oxaloacetate formed by PEP carboxylase to malate. The simultaneous oxidation of NADH is followed at 340 nm with standard optical equipment. The concentration of the inhibitors selected from the VDS was varied in the coupled assay to determine the half maximal inhibitory concentration (IC50).

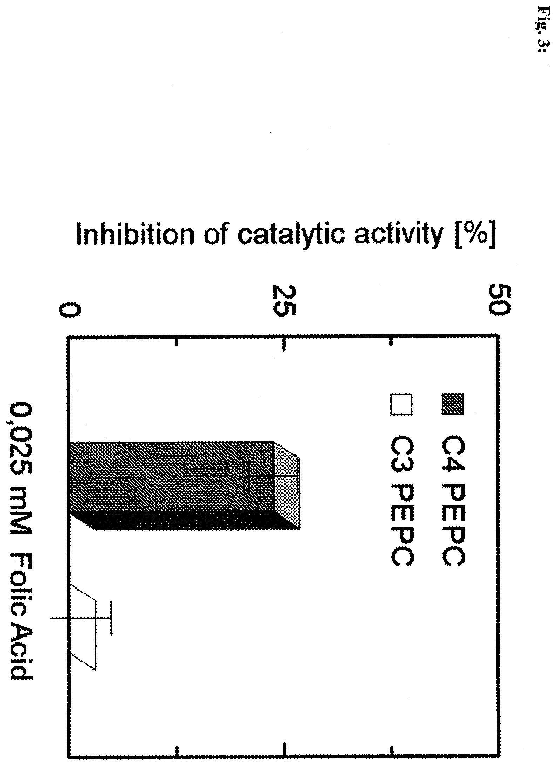

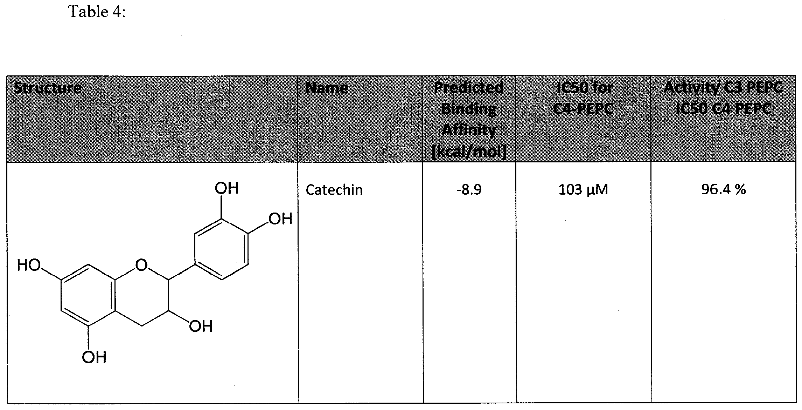

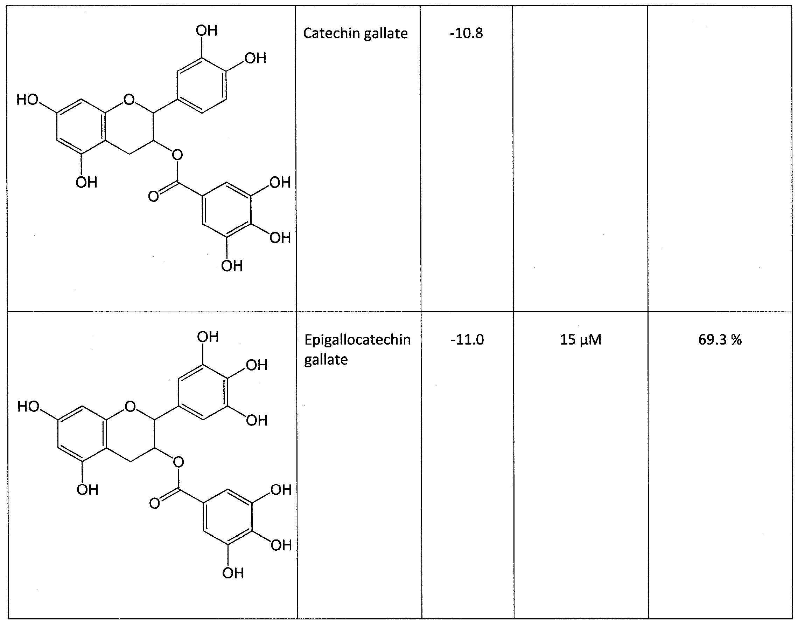

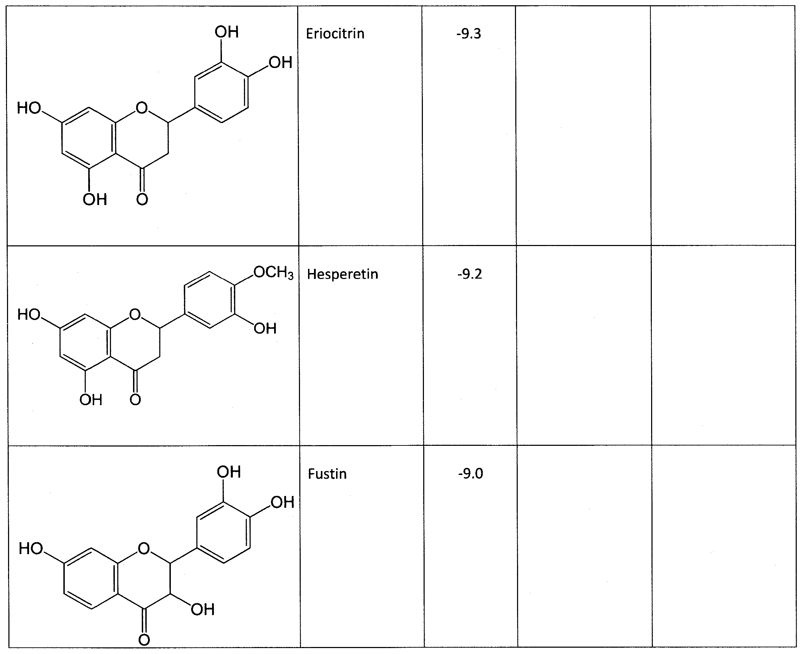

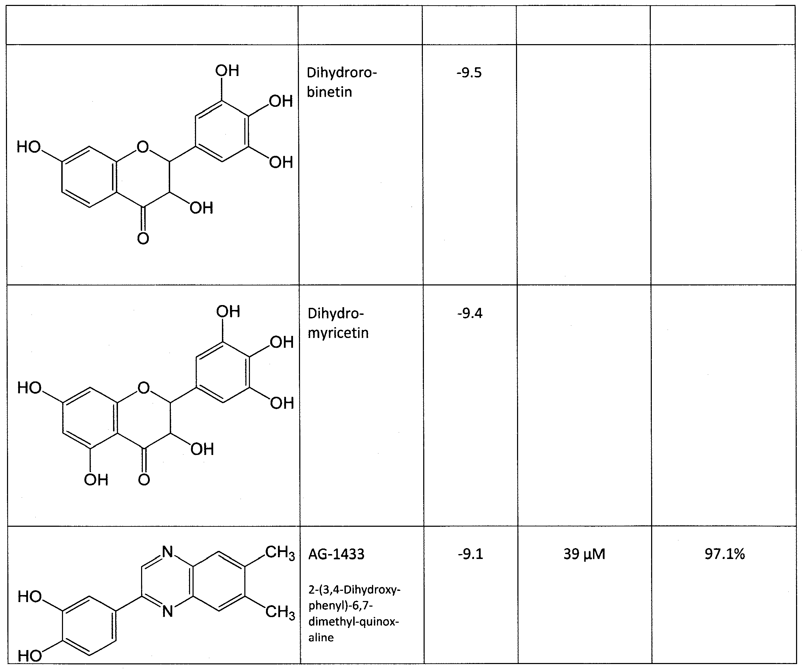

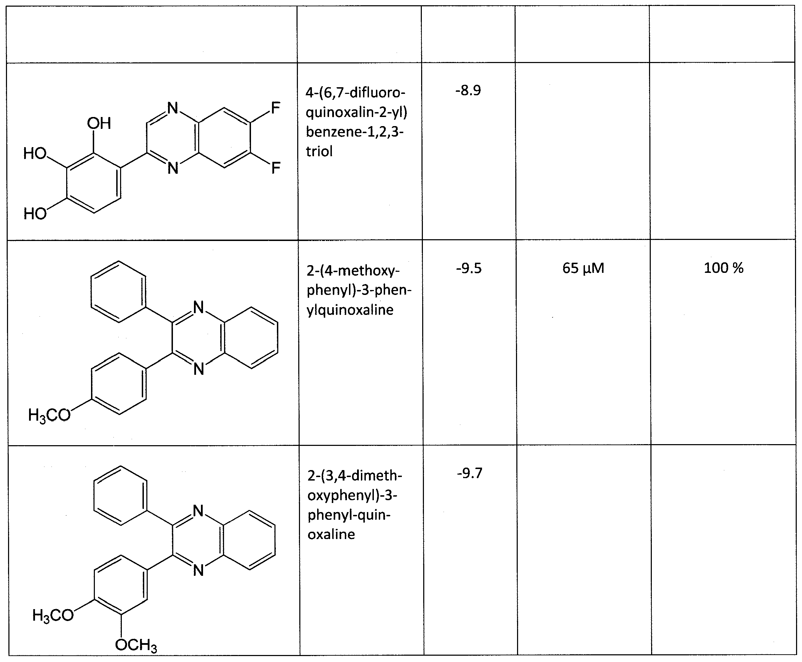

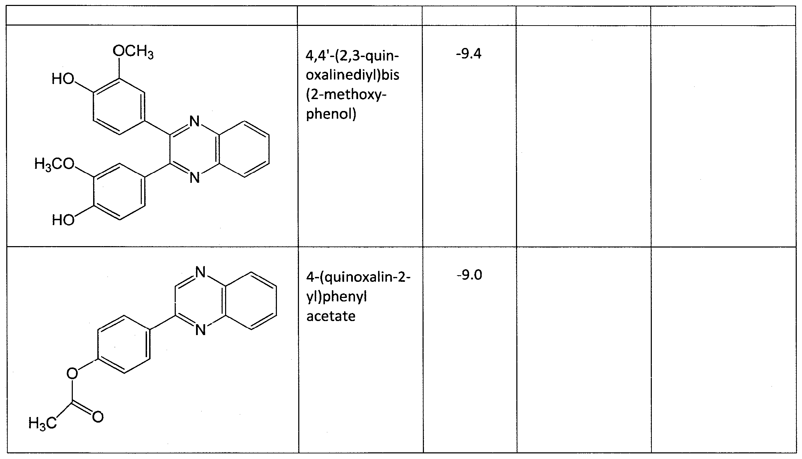

With folic acid the inhibition shown in Fig. 3 of the PEP carboxylase activity of the C3 and the C4 enzyme was obtained.

### References:

Collaborative Computational Project, Number 4 (1994) The CCP4 Suite: Programs for Protein Crystallography. Acta Cryst. D50, 760-763.
DeLano, W. L., (2002) The PyMOL Molecular Graphics System, DeLano Scientific, San Carlos, CA, USA
Doyle, JR, Burnell, JN, Haines, DS, Llewellyn, LE, Motti, CA und Tapiolas, DM (2005) A Rapid Screening Method to Detect Specific Inhibitors of Pyruvate Orthophosphate Dikinase as Leads for C4 Plant-Selective Herbicides. J Biomol Screen 10: 67-75.
Durrant, JD, Amaro, RE und McCammon, JA (2009) AutoGrow: A Novel Algorithm for Protein Inhibitor Design. Chemical Biology & Drug Design 73(2): 168-178
Emsley P, Cowtan K (2004)Coot: model-building tools for molecular graphics. Acta Crystallographica Section D-Biological Crystallography 60: 2126-2132 Part 12 Sp. Iss.
Jenkins CLD, Harris RLN und McFadden HG (1987) 3,3-Dichloro-2-dihydroxyphosphinoylmethyl-2-propenoate, a new, specific inhibitor of phosphoenolpyruvate carboxylase. Biochem Int 14:219-226.
Jenkins CLD (1989) Effects of the phosphoenolpyruvate carboxylase inhibitor3,3-dichloro-2-(dihydroxyphosphinoylmethyl)propenoate on photosynthesis. Plant Physiol 89:1231-1237.
Kabsch, W.(2010) XDS. Acta Cryst. D66, 125-132
Mancera RL, Gómez AG und Pisanty A. (1995) Quantitative structure-activity relationships of com-petitive inhibitors of phosphoenolpyruvate carboxylase. Bioorg Med Chem. 3(3):217-25.
Matsumura, H, Xie, Y, Shirakata, S, Inoue, T, Yoshinaga, T, Ueno, Y, Izui, K und Kai Y. (2002) Crystal structures of C4 form maize and quaternary complex of E. coli phosphoenolpyruvate car-boxylases. Structure 10(12):1721-30.
McFadden, HG, Harris, RLN and Jenkins, CLD (1989) Potential Inhibitors of Phosphoenolpyruvate Carboxylase. II. Phosphonic Acid Substrate Analogues Derived from Reaction of Trialkyl Phosphites with Halomethacrylates. Aust. J. Chem. 42:301-14.
Motti, CA, Bourne, DG, Burnell, JN, Doyle, JR, Haines, DS, Liptrot, CH, Llewellyn, LE, Ludke, S, Muirhead, A und Tapiolas DM (2007) Screening marine fungi for inhibitors of the C4 plant enzyme pyruvate phosphate dikinase: unguinol as a potential novel herbicide candidate. Appl Environ Mi-crobiol. 73(6):1921-7
Pairoba, CF, Colombo, SL and Andreo, CS (1996) Flavonoids as Inhibitors of NADP-Malic Enzyme and PEP Carboxylase from C4 Plants. Biosci. Biotech. Biochem. 60(5): 779-783.
Trott, O und Olson, AJ (2010) AutoDock Vina: improving the speed and accuracy of docking with a new scoring function, efficient optimization and multithreading. Journal of Computational Chemistry 31:455-461.
Wolf LK (2009) New software and Websites for the Chemical Enterprise, Chemical & Engineering News 87, 31

## Claims

1. Use of a compound, a salt or solvate thereof as C4 plant selective herbicide wherein said compound comprises a cyclic alkyl, aryl, heterocycloalkyl or heteroaryl group, and wherein said compound further comprises
(a) at least a functional group Z being -C(=Y^{Z})R¹, wherein Y^{Z} is selected from the group consisting of O, NH and S, preferably wherein Y^{Z} is O, and wherein R¹ is selected from the group consisting of H, OH and N(R²R³), - O(R²) and -S(R²), wherein R² and R³, are independently of each other, selected from the group consisting of H, Alkyl, cycloalkyl, aryl and heteroaryl and a functional group Q which is -C(=Y^{Q})R⁴, wherein Y^{Q} is selected from the group consisting of O, NH and S, preferably wherein Y^{Q} is O, and wherein R⁴ is selected from the group consisting of H, OH and NR^{4#}R^{5#}, OR^{4#}, SR^{4#} wherein R^{4#} and R^{5#}, are independently of each other, selected from the group consisting of H, alkyl, cycloalkyl, aryl and heteroaryl, wherein the compound has a structure according to the formula (I) 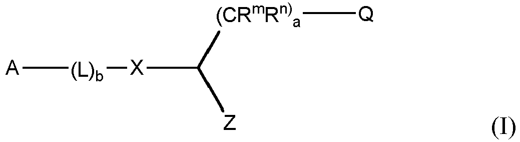
wherein A is selected from the group consisting of 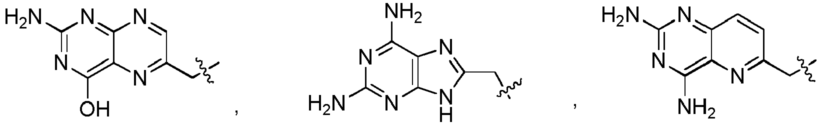 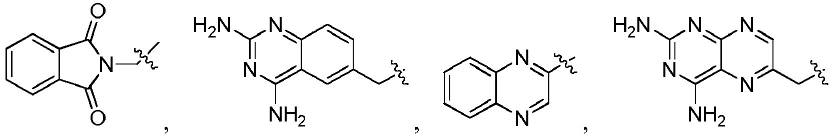 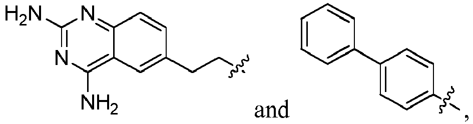
X is -Y^{X}-, or -C(=Y^{XX})-, wherein Y^{X} is selected from the group consisting of-S-, -O-, -NH-, -N(CH₃)- and NH-NH-,
and Y^{XX} is selected from the group consisting of S, O and NH,
in particular -NH- or -C(=Y)- with Y being S or O,
L is a linking moiety, preferably a linking moiety comprising an aryl group,
integer b is 0 or 1,
integer a is in the range of from 2 to 4
and wherein R^{m} and Rⁿ are, independently of each other, H or alkyl, preferably H or methyl, in particular H,
or
(b) at least one electron donating group selected from the group consisting of OH, -SH, -O-R^{p}, -S-R^{p}, and -NR^{p}R^{q}, wherein R^{p} and R^{q} are, independently of each other, selected from the group consisting of H, alkyl, cycloalkyl and heteroaryl, wherein the compound has a structure of the formula (IV') or (IV") 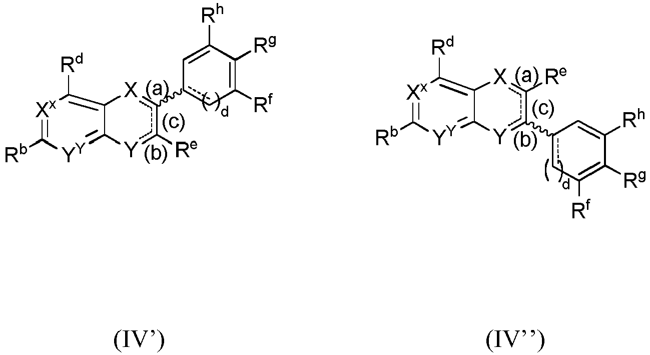 wherein
X is selected from the group consisting of -C(=O)-, -O- and -N-,
Y is selected from the group consisting of -C(H)ₚ-, -C(=O)-, -O- and -N-,
X^{x} is selected from the group consisting of -C(R^{a})- or N, Y^{y} is selected from the group consisting of -C(R^{c})- or N,
with p being 1 or 2,
the bond (a) is a single or double bond,
the bond (b) is a single or double bond,
the bond (c) is a single or double bond,
and wherein R^{a} and R^{b} are, independently of each other selected from the group consisting of H, Alkyl, halogene, -C(=Y^{p})R^{r}, and an electron donating group, wherein R^{r} is -OH, -NH₂, -NH-Alkyl, -O-Alkyl, or -S-Alkyl, and
wherein Y^{p} is selected from the group consisting of S, O and NH, or
wherein R^{a} and R^{b} form together a cycloalkyl, cycloheteroalkyl, aryl or heteroaryl, wherein the cycloalkyl, cycloheteroalkyl, aryl or heteroaryl is a single-ring group or a multicyclic group,
and wherein R^{c}, R^{d}, R^{f}, R^{g} and R^{h} are, independently of each other, selected from the group consisting of H, Alkyl, halogene, -C(=Y^{p})R^{r}, and an electron donating group, wherein R^{r} is -OH, -NH₂, -NH-Alkyl, -O-Alkyl, or -S-Alkyl, and wherein Y^{p} is selected from the group consisting of S, O and NH, and wherein R^{e} is selected from the group consisting of H, Alkyl, halogene, -C(=Y^{p})R^{r}, -O-C(=Y^{p})R^{r}, an electron donating group, aryl, heteroaryl group and -O-C(=O)-R*, wherein R* is an aryl or heteroaryl group, and wherein
R^{r} is -OH, -NH₂, -NH-Alkyl, -O-Alkyl, or -S-Alkyl, and wherein Y^{p} is selected from the group consisting of S, O and NH,
wherein at least one of R^{a}, R^{b}, R^{c}, R^{d}, R^{f}, R^{g}, R^{e} and R^{h} is the electron donating group selected from the group consisting of OH, -SH, -O-R^{p}, -S-R^{p}, and -NR^{p}R^{q}" with the proviso that in case the bond (c) is a double bond (a) and (b) are both single bonds, and in case X is -C(=O)-, the bond (a) is a single bond, and wherein in case Y is -C(=O)-, the bond (b) is a single bond, and wherein integer d is 1,
said compound being capable of binding to the malate binding site comprised by a phosphoenolpyruvate carboxylase from a C4 plant, thereby inhibiting said phosphoenolpyruvate carboxylase,
and wherein the cyclic alkyl, aryl, heterocycloalkyl or heteroaryl group inhibits binding to the malate binding site of a phosphoenolpyruvate carboxylase from a C3 plant.

2. Use according to claim 1, wherein in (a) Q is COOH, the compound having the structure (II):

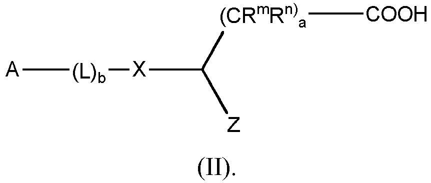

3. Use according to claims 1 and 2, wherein in (a) Z is COOH, the compound having the structure (III):

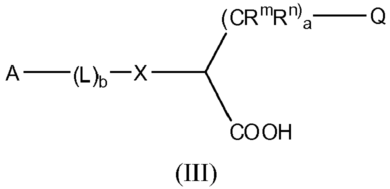

4. Use according to any of claims 1 to 3, wherein in (a) L has a structure according to the following formula
-[F²]_{c}-[L¹]_{d}-[F¹]ₑ-
wherein L¹ is a linking moiety, preferably selected from the group consisting of alkyl, alkenyl, alkylaryl, arylalkyl, aryl, heteroaryl, alkylheteroaryl and heteroarylalkyl,
and wherein integer d is 0 or 1,
and wherein
F¹ is a functional group consisting of -Y^{F1}-, -C(=Y^{F})-, -C(=Y^{F})-NR^{Y1}-,
wherein Y^{F1} is selected from the group consisting of -S-, -O-, -NH-, -N(CH₃)- and NH-NH-,
Y^{F} is selected from the group consisting of S, O and NH
and R^{Y1} is H or alkyl,
and wherein in case X is -Y^{X}-, F¹ is -C(=Y^{F})- or -C(=Y^{F})-NR^{Y1} and wherein in case X is -C(=Y^{X})-, -C(=Y^{XX})-NR^{X}-, F¹ is -Y^{F1}-,
integer e is 0 or 1,
F² is a functional group selected from the group consisting of -Y^{F2}-, -C(=Y^{FF})-, -C(=Y^{FF})-NR^{Y2}- and -NR^{Y2}-C(=Y^{FF})-
wherein Y^{F2} is selected from the group consisting of -S-, -O-, -NH-, -N(CH₃)- ,-NH-NH-,
Y^{FF} is selected from the group consisting of S, O and NH and R^{Y2} is H or alkyl,
and wherein integer c is 0 or 1.

5. Use according to claim 4, wherein in (a) X is -NH- and wherein L is
-Y^{F2}-L¹-C(=Y^{F})-
wherein L¹ is alkylaryl, arylalkyl, heteroaryl or aryl group, preferably a group having one of the following structures: 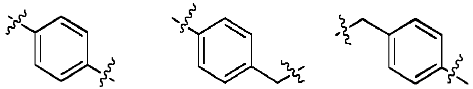 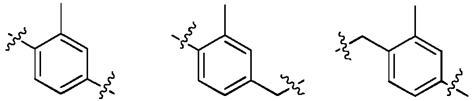 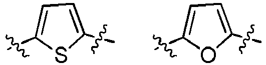 more preferably wherein L is selected from the following structures:

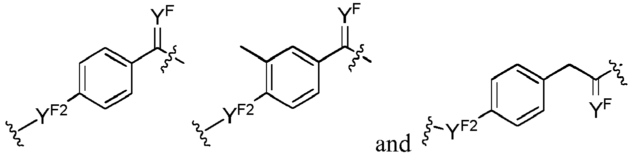

6. The use according to claim 1, wherein the compound in (b) has a structure of the formula (IVa) or (IVb) 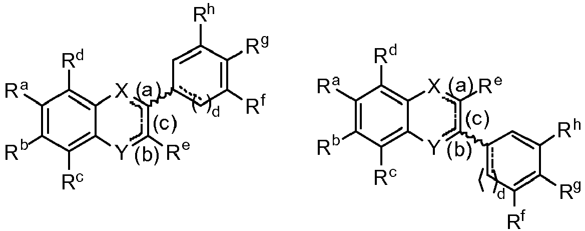 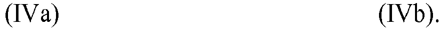
preferably wherein R^{a}, R^{b}, R^{c}, R^{d}, R^{e}, R^{f}, R^{g} and R^{h} independently of each other, selected from the group consisting of H, Alkyl, -C(=Y^{p})R^{r}, and an electron donating group, , said electron donating group being selected from the group consisting of -OH, -SH, -O-Alkyl, -S-Alkyl, -NR^{p}R^{q} and -C(=Y^{p})R^{r},
wherein R^{p} and R^{q} are, independently of each other H or alkyl,
and wherein R^{r} is -OH, -NH₂, -NH-Alkyl, -O-Alkyl or -NH-Alkyl,
and wherein Y^{p} is selected from the group consisting of S, O and NH,
wherein at least one of R^{a}, R^{b}, R^{c}, R^{d}, R^{e} and R^{f} is an electron donating group.

7. Use according to claim 1 or 6, wherein in (b) at least one of R^{a}, R^{b}, R^{c}, R^{d}, R^{e} and R^{f} is -OH.

## Patentansprüche

1. Verwendung einer Verbindung, eines Salzes oder eines Solvates davon als C4-Pflanzen-selektives Herbizid, wobei die Verbindung eine cyclische Alkyl-, Aryl-, Heterocycloalkyl- oder Heteroarylgruppe umfasst und wobei die Verbindung ferner Folgendes umfasst:
(a) mindestens eine funktionelle Gruppe Z, bei der es sich um -C(=Y^{Z})R¹ handelt, wobei Y^{Z} aus der Gruppe bestehend aus O, NH und S ausgewählt ist, vorzugsweise wobei Y^{Z} für O steht, und wobei R¹ aus der Gruppe bestehend aus H, OH und N (R²R³), -O(R²) und -S(R²) ausgewählt ist, wobei R² und R³ unabhängig voneinander aus der Gruppe bestehend aus H, Alkyl, Cycloalkyl, Aryl und Heteroaryl ausgewählt sind, und eine funktionelle Gruppe Q, bei der es sich um -C(=Y^{Q})R⁴ handelt, wobei Y^{Q} aus der Gruppe bestehend aus O, NH und S ausgewählt ist, vorzugsweise wobei Y^{Q} für O steht, und wobei R⁴ aus der Gruppe bestehend aus H, OH und NR^{4#}R^{5#}, OR^{4#} und SR^{4#} ausgewählt ist, wobei R^{4#} und R^{5#} unabhängig voneinander aus der Gruppe bestehend aus H, Alkyl, Cycloalkyl, Aryl und Heteroaryl ausgewählt sind, wobei die Verbindung eine Struktur gemäß der Formel (I) aufweist: 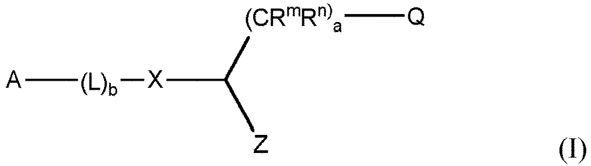
wobei A aus der Gruppe bestehend aus 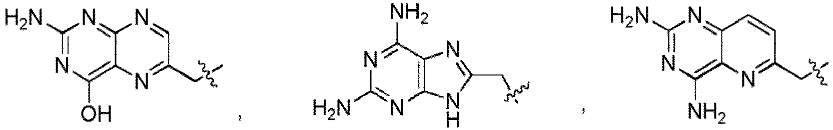 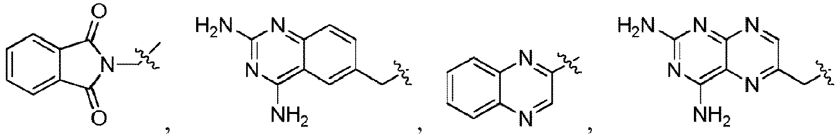 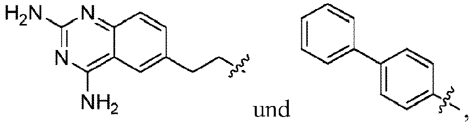 ausgewählt ist,
X für -Y^{X}- oder -C(=Y^{XX})- steht, wobei Y^{X} aus der Gruppe bestehend aus -S-, -O-, -NH-, -N(CH₃)- und NH-NH- ausgewählt ist
und Y^{XX} aus der Gruppe bestehend aus NH, O und S ausgewählt ist,
insbesondere -NH- oder -C(=Y)-, wobei Y für S oder O steht,
L für eine Verknüpfungsgruppierung, vorzugsweise eine Verknüpfungsgruppierung, die eine Arylgruppe umfasst, steht,
die ganze Zahl b für 0 oder 1 steht,
die ganze Zahl a im Bereich von 2 bis 4 liegt und wobei R^{m} und Rⁿ unabhängig voneinander für H oder Alkyl, vorzugsweise H oder Methyl, insbesondere H, stehen,
oder
(b) mindestens eine elektronenliefernde Gruppe aus der Gruppe bestehend aus OH, -SH, -O-R^{p}, -S-R^{p} und -NR^{p}R^{q}, wobei R^{p} und R^{q} unabhängig voneinander aus der Gruppe bestehend aus H, Alkyl, Cycloalkyl und Heteroaryl ausgewählt sind, wobei die Verbindung eine Struktur gemäß der Formel (IV') oder (IV'') aufweist: 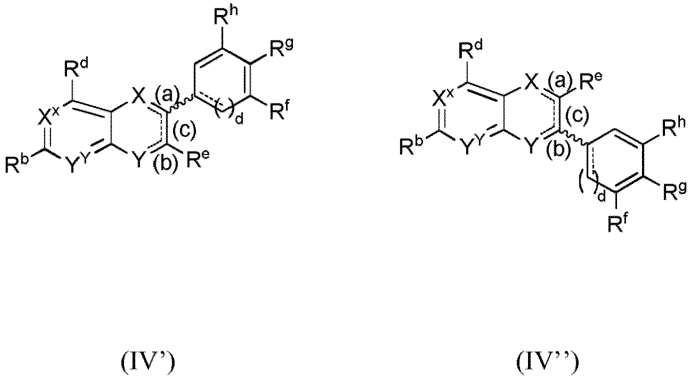 wobei
X aus der Gruppe bestehend aus -C(=O)-, -O- und - N- ausgewählt ist,
Y aus der Gruppe bestehend aus -C(H)ₚ, -C(=O)-, -O- und -N- ausgewählt ist,
X^{x} aus der Gruppe bestehend aus -C(R^{a})- oder N ausgewählt ist, Y^{y} aus der Gruppe bestehend aus -C(R^{c})- oder N ausgewählt ist,
wobei p für 1 oder 2 steht,
die Bindung (a) eine Einfach- oder Doppelbindung ist,
die Bindung (b) eine Einfach- oder Doppelbindung ist,
die Bindung (c) eine Einfach- oder Doppelbindung ist,
und wobei R^{a} und R^{b} unabhängig voneinander aus der Gruppe bestehend aus H, Alkyl, Halogen, -C(=Y^{p})R^{r} und einer elektronenliefernden Gruppe ausgewählt sind, wobei R^{r} für -OH, -NH₂, -NH-Alkyl, -O-Alkyl oder -S-Alkyl steht und wobei Y^{p} aus der Gruppe bestehend aus S, O und NH ausgewählt ist, oder
wobei R^{a} und R^{b} zusammen ein Cycloalkyl, Cycloheteroalkyl, Aryl oder Heteroaryl bilden, wobei das Cycloalkyl, Cycloheteroalkyl, Aryl oder Heteroaryl eine Gruppe mit einem Ring oder eine Gruppe mit mehreren Ringen ist,
und wobei R^{c}, R^{d}, R^{f}, R^{g} und R^{h} unabhängig voneinander aus der Gruppe bestehend aus H, Alkyl, Halogen, -C(=Y^{p})R^{r} und einer elektronenliefernden Gruppe ausgewählt sind, wobei R^{r} für -OH, -NH₂, - NH-Alkyl, -O-Alkyl oder -S-Alkyl steht und wobei Y^{p} aus der Gruppe bestehend aus S, O und NH ausgewählt ist,
und wobei R^{e} aus der Gruppe bestehend aus H, Alkyl, Halogen, -C(=Y^{p})R^{r}, -O-C(=Y^{p})R^{r}, eine elektronenliefernden Gruppe, eine Arylgruppe, einer Heteroarylgruppe und -O-C(O)-R* ausgewählt ist, wobei R* für eine Aryl- oder Heteroarylgruppe steht und wobei R^{r} für -OH, -NH₂, -NH-Alkyl, -O-Alkyl oder -S-Alkyl steht und wobei Y^{p} aus der Gruppe bestehend aus S, O und NH ausgewählt ist, wobei mindestens einer der Reste R^{a}, R^{b}, R^{c}, R^{d}, R^{f}, R^{g}, R^{e} und R^{h} für die elektronenliefernde Gruppe aus der Gruppe bestehend aus OH, -SH, -O-R^{p}, -S-R^{p} und -NR^{p}R^{q} steht, mit der Maßgabe, dass in dem Fall, dass die Bindung (c) eine Doppelbindung ist, (a) und (b) beide Einfachbindungen sind, und in dem Fall, dass X für -C(=O)- steht, die Bindung (a) eine Einfachbindung ist, und wobei in dem Fall, dass Y für -C(=O)- steht, die Bindung (b) eine Einfachbindung ist,
und wobei die ganze Zahl d für 1 steht,
wobei die Verbindung an die Malat-Bindungsstelle in einer Phosphoenolpyruvat-carboxylase aus einer C4-Pflanze binden kann, wodurch die Phosphoenolpyruvatcarboxylase inhibiert wird,
und wobei die cyclische Alkyl-, Aryl-, Heterocycloalkyl- oder Heteroarylgruppe die Bindung an die Malat-Bindungsstelle einer Phosphoenolpyruvat-carboxylase aus einer C3-Pflanze inhibiert.

2. Verwendung nach Anspruch 1, wobei in (a) Q für COOH steht, wobei die Verbindung die Struktur (II) aufweist:

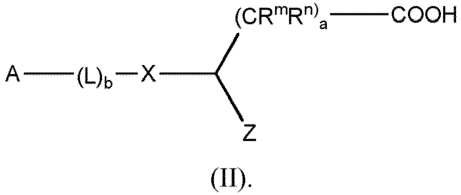

3. Verwendung nach den Ansprüchen 1 und 2, wobei in (a) Z für COOH steht, wobei die Verbindung die Struktur (III) aufweist:

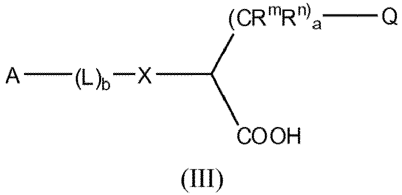

4. Verwendung nach einem der Ansprüche 1 bis 3, wobei in (a) L eine Struktur gemäß der folgenden Formel aufweist:
-[F²]_{c}-[L¹]_{d}-[F¹]ₑ-
wobei L¹ für eine Verknüpfungsgruppierung, vorzugsweise aus der Gruppe bestehend aus Alkyl, Alkenyl, Alkylaryl, Arylalkyl, Aryl, Heteroaryl, Alkylheteroaryl und Heteroarylalkyl, steht und wobei die ganze Zahl d für 0 oder 1 steht,
und wobei
F¹ für eine funktionelle Gruppe steht, die aus -Y^{F1}-, -C(=Y^{F})-, -C(=Y^{F})-NR^{Y1}- besteht,
wobei Y^{F1} aus der Gruppe bestehend aus -S, -O-, -NH-, -N(CH₃)- und -NH-NH- ausgewählt ist,
Y^{F} aus der Gruppe bestehend aus S, O und NH ausgewählt ist
und R^{Y1} für H oder Alkyl steht,
und wobei in dem Fall, dass X für -Y^{X}- steht, F¹ für -C(=Y^{F})- oder -C(=Y^{F})-NR^{Y1} steht, und wobei in dem Fall, dass X für -C(=Y^{X})-, -C(=Y^{XX})-NR^{X}- steht, F¹ für -Y^{F1}- steht,
die ganze Zahl e für 0 oder 1 steht,
F² für eine funktionelle Gruppe aus der Gruppe bestehend aus -Y^{F2}-, -C(=Y^{FF})-, -C(=Y^{FF})-NR^{Y2}- und -NR^{Y2}-C(=Y^{FF})- steht,
wobei Y^{F2} aus der Gruppe bestehend aus -S-, -O-, -NH-, -N(CH₃)- und -NH-NH- ausgewählt ist,
Y^{FF} aus der Gruppe bestehend aus S, O und NH ausgewählt ist
und R^{Y2} für H oder Alkyl steht,
und wobei die ganze Zahl c für 0 oder 1 steht.

5. Verwendung nach Anspruch 4, wobei in (a) X -NHsteht und wobei L für -Y^{F2}-L¹-C(=Y^{F})- steht,
wobei L¹ für eine Alkylaryl-, Arylalkyl-, Heteroaryl- oder Arylgruppe, vorzugsweise eine Gruppe mit einer der folgenden Strukturen: 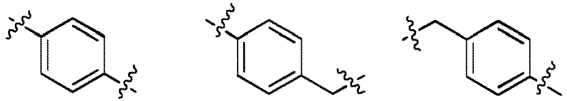 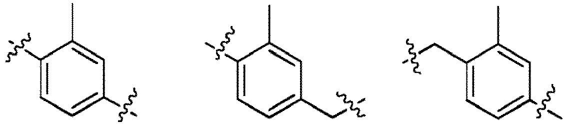 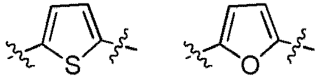 steht, weiter bevorzugt wobei L aus den folgenden Strukturen ausgewählt ist:

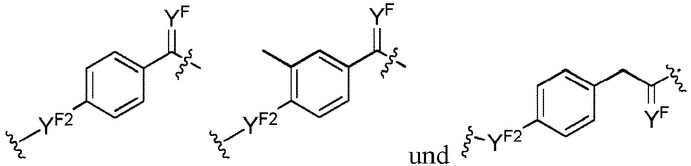

6. Verwendung nach Anspruch 1, wobei die Verbindung in (b) eine Struktur der Formel (IVa) oder (IVb) aufweist: 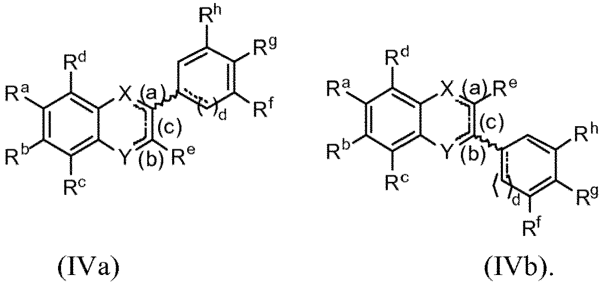 vorzugsweise wobei R^{a}, R^{b}, R^{c}, R^{d}, R^{e}, R^{f}, R^{g} und R^{h} unabhängig voneinander aus der Gruppe bestehend aus H, Alkyl, -C(=Y^{p})R^{r} und einer elektronenliefernden Gruppe ausgewählt sind, wobei die elektronenliefernde Gruppe aus der Gruppe bestehend aus -OH, -SH, -O-Alkyl, -S-Alkyl, -NR^{p}R^{q} und -C(=Y^{p})R^{r} ausgewählt ist, wobei R^{p} und R^{q} unabhängig voneinander für H oder Alkyl stehen und wobei R^{r} für -OH, -NH₂, -NH-Alkyl, -O-Alkyl oder -NH-Alkyl steht
und wobei Y^{p} aus der Gruppe bestehend aus S, O und NHR ausgewählt ist,
wobei mindestens einer der Reste R^{a}, R^{b}, R^{c}, R^{d}, R^{e} und R^{f} für eine elektronenliefernde Gruppe steht.

7. Verwendung nach Anspruch 1 oder 6, wobei in (b) einer der Reste R^{a}, R^{b}, R^{c}, R^{d}, R^{e} und R^{f} für -OH steht.

## Revendications

1. Utilisation d'un composé, d'un sel ou solvate de celui-ci comme herbicide sélectif des plantes en C₄, dans laquelle ledit composé comprend un groupement alkyle cyclique, aryle, hétérocycloalkyle ou hétéroaryle, et où ledit composé comprend en outre
(a) au moins un groupement fonctionnel Z qui est - C(=Y^{Z})R¹, où Y^{Z} est choisi dans le groupe constitué par O, NH et S, préférablement où Y^{Z} est O, et où R¹ est choisi dans le groupe constitué par H, OH et N(R²R³), - O(R²) et -S(R²), où R² et R³ sont choisis, indépendamment l'un de l'autre, dans le groupe constitué par H, alkyle, cycloalkyle, aryle et hétéroaryle et un groupement fonctionnel Q qui est -C(=Y^{Q})R⁴, où Y^{Q} est choisi dans le groupe constitué par O, NH et S, préférablement où Y^{Q} est O, et où R⁴ est choisi dans le groupe constitué par H, OH et NR^{4#}R^{5#}, OR^{4#}, et SR^{4#}, où R^{4#} et R^{5#} sont choisis, indépendamment l'un de l'autre, dans le groupe constitué par H, alkyle, cycloalkyle, aryle et hétéroaryle, où le composé répond à une structure selon la formule (I) 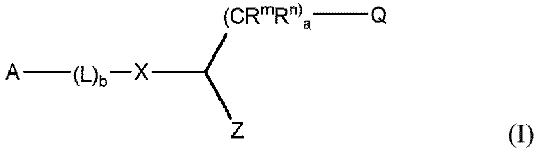
où A est choisi dans le groupe constitué par 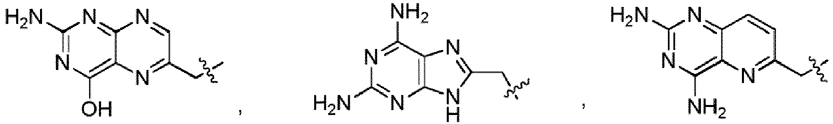 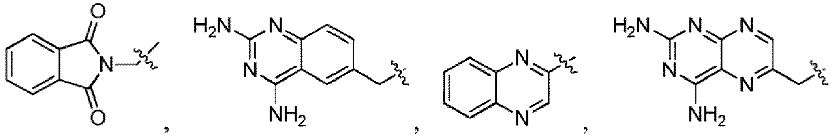 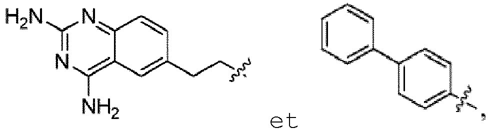
X est -Y^{X-}, ou -C(=Y^{XX})-, où Y^{X} est choisi dans le groupe constitué par -S-, -O-, -NH-, -N(CH₃)- et NH-NH-,
et Y^{XX} est choisi dans le groupe constitué par S, O, et NH,
en particulier -NH- ou -C(=Y)-, Y étant S ou O,
L est un motif de liaison, préférablement un motif de liaison comprenant un groupement aryle,
le nombre entier b vaut 0 ou 1,
le nombre entier a se trouve dans la plage allant de 2 à 4,
et où R^{m} et Rⁿ sont, indépendamment l'un de l'autre, H ou alkyle, préférablement H ou méthyle, en particulier H,
ou
(b) au moins un groupement donneur d'électrons choisi dans le groupe constitué par OH, -SH, -O-R^{p}, -S-R^{p}, et - NR^{p}R^{q}, où R^{p} et R^{q} sont, indépendamment l'un de l'autre, choisis dans le groupe constitué par H, alkyle, cycloalkyle et hétéroaryle, où le composé répond à une structure selon la formule (IV') ou (IV") 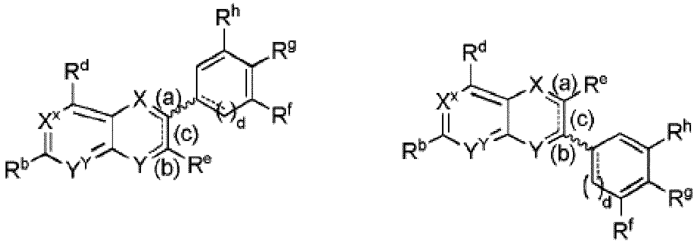 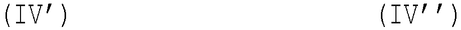 où
X est choisi dans le groupe constitué par -C(=O)-, -O-et -N-,
Y est choisi dans le groupe constitué par -C(H)ₚ-, -C(=O)-, -O- et -N-,
X^{x} est choisi dans le groupe constitué par -C(R^{a})- ou N,
Y^{y} est choisi dans le groupe constitué par -C(R^{c}) - ou N,
p valant 1 ou 2,
la liaison (a) est une liaison simple ou double,
la liaison (b) est une liaison simple ou double,
la liaison (c) est une liaison simple ou double,
et où R^{a} et R^{b} sont, indépendamment l'un de l'autre, choisis dans le groupe constitué par H, alkyle, halogène, -C(=Y^{p})R^{r}, et un groupement donneur d'électrons, où R^{r} est -OH, -NH₂, -NH-alkyle, -O-alkyle, ou -S-alkyle, et où Y^{p} est choisi dans le groupe constitué par S, O, et NH, ou où R^{a} et R^{b} forment ensemble un groupement cycloalkyle, cyclohétéroalkyle, aryle ou hétéroaryle, où le groupement cycloalkyle, cyclohétéroalkyle, aryle ou hétéroaryle est un groupement à cycle unique ou un groupement à cycles multiples,
et où R^{c}, R^{d}, R^{f}, R^{g} et R^{h} sont, indépendamment les uns des autres, choisis dans le groupe constitué par H, alkyle, halogène, -C(=Y^{p})R^{r}, et un groupement donneur d'électrons, où R^{r} est -OH, -NH₂, -NH-alkyle, -O-alkyle, ou -S-alkyle, et où Y^{p} est choisi dans le groupe constitué par S, O, et NH, et où R^{e} est choisi dans le groupe constitué par H, alkyle, halogène, -C(=Y^{p})R^{r}, -O-C(=Y^{p})^{Rr}, un groupement donneur d'électrons, un groupement aryle, hétéroaryle et -O-C(=O)-R*, où R* est un groupement aryle ou hétéroaryle, et où R^{r} est -OH, -NH₂, -NH-alkyle, -O-alkyle, ou -S-alkyle, et où Y^{p} est choisi dans le groupe constitué par S, O, et NH,
où au moins l'un parmi R^{a}, R^{b}, R^{c}, R^{d}, R^{f}, R^{g}, R^{e} et R^{h} est le groupement donneur d'électrons choisi dans le groupe constitué par OH, -SH, -O-R^{p}, -S-R^{p}, et -NR^{p}R^{q}, à condition que dans le cas où la liaison (c) est une double liaison, (a) et (b) soient tous deux des liaisons simples, et dans le cas où X est -C(=O)-, la liaison (a) soit une liaison simple, et où dans le cas où Y est -C(=O)-, la liaison (b) soit une liaison simple,
et où le nombre entier d vaut 1,
ledit composé étant capable de se fixer au site de fixation de malate compris par une phosphoénolpyruvate carboxylase issue d'une plante en C₄, inhibant ainsi ladite phosphoénolpyruvate carboxylase,
et où le groupement alkyle cyclique, aryle, hétérocycloalkyle ou hétéroaryle inhibe la fixation au site de fixation de malate d'une phosphoénolpyruvate carboxylase issue d'une plante en C₃.

2. Utilisation selon la revendication 1, dans laquelle, dans (a), Q est COOH, le composé répondant à la structure (II) :

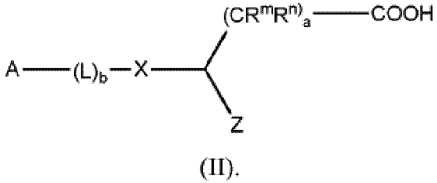

3. Utilisation selon les revendications 1 et 2, dans laquelle, dans (a), Z est COOH, le composé répondant à la structure (III) :

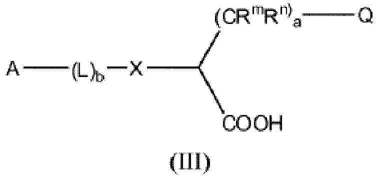

4. Utilisation selon l'une quelconque des revendications 1 à 3, dans laquelle, dans (a), L répond à une structure selon la formule suivante
-[F²]_{c}-[L¹]_{d}-[F¹]ₑ-
où L¹ est un motif de liaison, choisi préférablement dans le groupe constitué par alkyle, alcényle, alkylaryle, arylalkyle, aryle, hétéroaryle, alkylhétéroaryle et hétéroarylalkyle,
et où le nombre entier d vaut 0 ou 1,
et où
F¹ est un groupement fonctionnel constitué par -Y^{F1}-, - C(=Y^{F})-, -C(=Y^{F})-NR^{y1}-,
où Y^{F1} est choisi dans le groupe constitué par -S-, -O-, -NH-, -N(CH₃)- et NH-NH-,
Y^{F} est choisi dans le groupe constitué par S, O, et NH, et R^{Y1} est H ou alkyle,
et où dans le cas où X est -Y^{X}-, F¹ est -C(=Y^{F})- ou - C(=Y^{F})-NR^{Y1} et où dans le cas où X est -C(=Y^{X})-, -C(=Y^{XX})-NR^{X}-, F¹ est -Y^{F1}-,
le nombre entier e vaut 0 ou 1,
F² est un groupement fonctionnel choisi dans le groupe constitué par -Y^{F2}-, -C(=Y^{FF})-, -C(=Y^{FF})-NR^{Y2}- et -NR^{Y2}-C(=Y^{FF})-,
où Y^{F2} est choisi dans le groupe constitué par -S-, -O-, -NH-, -N(CH₃)-, et -NH-NH-,
Y^{FF} est choisi dans le groupe constitué par S, O, et NH, et R^{Y2} est H ou alkyle,
et où le nombre entier c vaut 0 ou 1.

5. Utilisation selon la revendication 4, dans laquelle, dans (a), X est -NH- et où L est -Y^{F2}-L¹-C(=Y^{F})-
où L¹ est un groupement alkylaryle, arylalkyle, hétéroaryle ou aryle, préférablement un groupement répondant à l'une des structures suivantes : 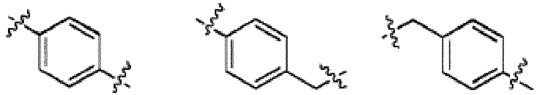 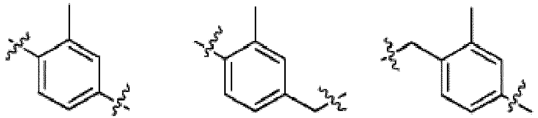 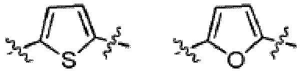 plus préférablement où L est choisi parmi les structures suivantes :

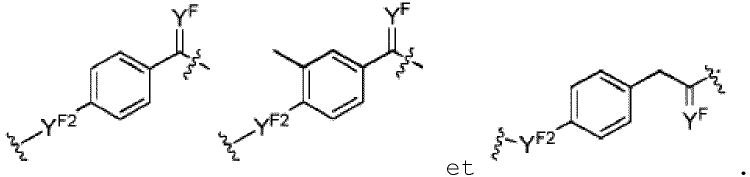

6. Utilisation selon la revendication 1, dans laquelle le composé dans (b) répond à une structure de formule (IVa) ou (IVb) 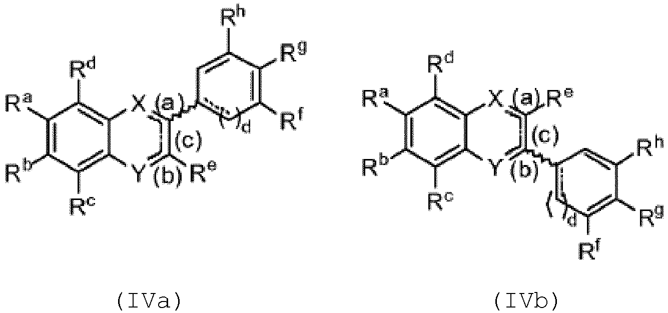 préférablement où R^{a}, R^{h}, R^{c}, R^{d}, R^{e}, R^{f}, R^{g} et R^{h}, indépendamment les uns des autres, sont choisis dans le groupe constitué par H, alkyle, -C(=Y^{p})R^{r}, et un groupement donneur d'électrons, ledit groupement donneur d'électrons étant choisi dans le groupe constitué par - OH, -SH, -O-alkyle, -S-alkyle, -NR^{p}R^{q} et -C(=Y^{p})R^{r}, où R^{p} et R^{q} sont, indépendamment l'un de l'autre, H ou alkyle, et où R^{r} est -OH, -NH₂, -NH-alkyle, -O-alkyle ou -NH-alkyle, et où Y^{p} est choisi dans le groupe constitué par S, O, et NH, où au moins l'un parmi R^{a}, R^{b}, R^{c}, R^{d}, R^{e} et R^{f} est un groupement donneur d'électrons.

7. Utilisation selon la revendication 1 ou 6, dans laquelle, dans (b), au moins l'un parmi R^{a}, R^{b}, R^{c}, R^{d}, R^{e} et R^{f} est -OH.


### SEQUENCE LISTING

<110> Heinrich-Heine-Universitaet Düsseldorf
<120> Selective Inhibition of C4-PEP carboxylases
<130> P10681PC
<160> 13
<170> PatentIn version 3.5
<210> 1
   <211> 966
   <212> PRT
   <213> Flaveria trinervia
<400> 1
   Met Ala Asn Arg Asn Val Glu Lys Leu Ala Ser Ile Asp Ala Gln Leu
   1               5                   10                  15
   Arg Leu Leu Val Pro Gly Lys Val Ser Glu Asp Asp Lys Leu Val Glu
               20                  25                  30
   Tyr Asp Ala Leu Leu Leu Asp Lys Phe Leu Asp Ile Leu Gln Asp Leu
           35                  40                  45
   His Gly Glu Asp Leu Lys Glu Ala Val Gln Gln Cys Tyr Glu Leu Ser
       50                  55                  60
   Ala Glu Tyr Glu Gly Lys His Asp Pro Lys Lys Leu Glu Glu Leu Gly
   65                  70                  75                  80
   Ser Leu Leu Thr Ser Leu Asp Thr Gly Asp Ser Ile Val Ile Ala Lys
                   85                  90                  95
   Ala Phe Ser His Met Leu Asn Leu Ala Asn Leu Ala Glu Glu Leu Gln
               100                 105                 110
   Ile Ala Tyr Arg Arg Arg Ile Lys Leu Lys Ser Gly Asp Phe Ala Asp
           115                 120                 125
   Glu Ala Asn Ala Thr Thr Glu Ser Asp Ile Glu Glu Thr Phe Lys Arg
       130                 135                 140
   Leu Val His Lys Leu Asn Lys Ser Pro Glu Glu Val Phe Asp Ala Leu
   145                 150                 155                 160
   Lys Asn Gln Thr Val Glu Leu Val Leu Thr Ala His Pro Thr Gln Ser
                   165                 170                 175
   Val Arg Arg Ser Leu Leu Gln Lys His Gly Arg Ile Arg Asn Cys Leu
   180 185 190
   Ala Gln Leu Tyr Ala Lys Asp Ile Thr Pro Asp Asp Lys Gln Glu Leu
   195 200 205
   Asp Glu Ala Leu His Arg Glu Ile Gln Ala Ala Phe Arg Thr Asp Glu
   210 215 220
   Ile Arg Arg Thr Pro Pro Thr Pro Gln Asp Glu Met Arg Ala Gly Met
   225 230 235 240
   Ser Tyr Phe His Glu Thr Ile Trp Lys Gly Val Pro Lys Phe Leu Arg
   245 250 255
   Arg Val Asp Thr Ala Leu Lys Asn Ile Gly Ile Asn Glu Arg Phe Pro
   260 265 270
   Tyr Asn Ala Pro Leu Ile Gln Phe Ser Ser Trp Met Gly Gly Asp Arg
   275 280 285
   Asp Gly Asn Pro Arg Val Thr Pro Glu Val Thr Arg Asp Val Cys Leu
   290 295 300
   Leu Ala Arg Met Met Thr Ser Asn Met Tyr Phe Ser Gln Ile Glu Asp
   305 310 315 320
   Leu Met Ile Glu Met Ser Met Trp Arg Cys Asn Ser Glu Leu Arg Val
   325 330 335
   Arg Ala Glu Glu Leu Tyr Arg Thr Ala Arg Lys Asp Val Lys His Tyr
   340 345 350
   Ile Glu Phe Trp Lys Arg Ile Pro Pro Asn Gln Pro Tyr Arg Val Ile
   355 360 365
   Leu Gly Asp Val Arg Asp Lys Leu Tyr Asn Thr Arg Glu Arg Ser Arg
   370 375 380
   His Leu Leu Val Asp Gly Lys Ser Asp Ile Pro Asp Glu Ala Val Tyr
   385 390 395 400
   Thr Asn Val Glu Gln Leu Leu Glu Pro Leu Glu Leu Cys Tyr Arg Ser
   405 410 415
   Leu Cys Asp Cys Gly Asp His Val Ile Ala Asp Gly Ser Leu Leu Asp
   420 425 430
   Phe Leu Arg Gln Val Ser Thr Phe Gly Leu Ser Leu Val Lys Leu Asp
           435                 440                 445
   Ile Arg Gln Glu Ser Asp Arg His Thr Glu Val Leu Asp Ala Ile Thr
       450                 455                 460
   Gln His Leu Gly Ile Gly Ser Tyr Arg Glu Trp Ser Glu Glu Lys Arg
   465                 470                 475                 480
   Gln Glu Trp Leu Leu Ala Glu Leu Ser Gly Lys Arg Pro Leu Ile Gly
                   485                 490                 495
   Pro Asp Leu Pro Lys Thr Glu Glu Val Lys Asp Cys Leu Asp Thr Phe
               500                 505                 510
   Lys Val Leu Ala Glu Leu Pro Ser Asp Cys Phe Gly Ala Tyr Ile Ile
           515                 520                 525
   Ser Met Ala Thr Ser Thr Ser Asp Val Leu Ala Val Glu Leu Leu Gln
       530                 535                 540
   Arg Glu Tyr His Ile Lys His Pro Leu Arg Val Val Pro Leu Phe Glu
   545                 550                 555                 560
   Lys Leu Ala Asp Leu Glu Ala Ala Pro Ala Ala Met Thr Arg Leu Phe
                   565                 570                 575
   Ser Met Asp Trp Tyr Arg Asn Arg Ile Asp Gly Lys Gln Glu Val Met
               580                 585                 590
   Ile Gly Tyr Ser Asp Ser Gly Lys Asp Ala Gly Arg Phe Ser Ala Ala
           595                 600                 605
   Trp Gln Leu Tyr Lys Thr Gln Glu Gln Ile Val Lys Ile Ala Lys Glu
       610                 615                 620
   Phe Gly Val Lys Leu Val Ile Phe His Gly Arg Gly Gly Thr Val Gly
   625                 630                 635                 640
   Arg Gly Gly Gly Pro Thr His Leu Ala Leu Leu Ser Gln Pro Pro Asp
                   645                 650                 655
   Thr Ile Asn Gly Ser Leu Arg Val Thr Val Gln Gly Glu Val Ile Glu
               660                 665                 670
   Gln Ser Phe Gly Glu Glu His Leu Cys Phe Arg Thr Leu Gln Arg Phe
           675                 680                 685
   Cys Ala Ala Thr Leu Glu His Gly Met Asn Pro Pro Ile Ser Pro Arg
       690                 695                 700
   Pro Glu Trp Arg Glu Leu Met Asp Gln Met Ala Val Val Ala Thr Glu
   705                 710                 715                 720
   Glu Tyr Arg Ser Val Val Phe Lys Glu Pro Arg Phe Val Glu Tyr Phe
                   725                 730                 735
   Arg Leu Ala Thr Pro Glu Leu Glu Phe Gly Arg Met Asn Ile Gly Ser
               740                 745                 750
   Arg Pro Ser Lys Arg Lys Pro Ser Gly Gly Ile Glu Ser Leu Arg Ala
           755                 760                 765
   Ile Pro Trp Ile Phe Ser Trp Thr Gln Thr Arg Phe His Leu Pro Val
       770                 775                 780
   Trp Leu Gly Phe Gly Ala Ala Phe Lys His Ala Ile Gln Lys Asp Ser
   785                 790                 795                 800
   Lys Asn Leu Gln Met Leu Gln Glu Met Tyr Lys Thr Trp Pro Phe Phe
                   805                 810                 815
   Arg Val Thr Ile Asp Leu Val Glu Met Val Phe Ala Lys Gly Asn Pro
               820                 825                 830
   Gly Ile Ala Ala Leu Asn Asp Lys Leu Leu Val Ser Glu Asp Leu Arg
           835                 840                 845
   Pro Phe Gly Glu Ser Leu Arg Ala Asn Tyr Glu Glu Thr Lys Asn Tyr
       850                 855                 860
   Leu Leu Lys Ile Ala Gly His Lys Asp Leu Leu Glu Gly Asp Pro Tyr
   865                 870                 875                 880
   Leu Lys Gln Gly Ile Arg Leu Arg Asp Pro Tyr Ile Thr Thr Leu Asn
                   885                 890                 895
   Val Cys Gln Ala Tyr Thr Leu Lys Arg Ile Arg Asp Pro Asn Tyr His
               900                 905                 910
   Val Thr Leu Arg Pro His Ile Ser Lys Glu Tyr Ala Ala Glu Pro Ser
           915                 920                 925
   Lys Pro Ala Asp Glu Leu Ile His Leu Asn Pro Thr Ser Glu Tyr Ala
   930 935 940
   Pro Gly Leu Glu Asp Thr Leu Ile Leu Thr Met Lys Gly Ile Ala Ala
   945 950 955 960
   Gly Met Gln Asn Thr Gly
   965
<210> 2
   <211> 967
   <212> PRT
   <213> Flaveria pringlei
<400> 2
   Met Ala Asn Arg Asn Leu Glu Lys Leu Ala Ser Ile Asp Ala Gln Leu
   1               5                   10                  15
   Arg Leu Leu Val Pro Gly Lys Val Ser Glu Asp Asp Lys Leu Ile Glu
               20                  25                  30
   Tyr Asp Ala Leu Leu Leu Asp Lys Phe Leu Asp Ile Leu Gln Asp Leu
           35                  40                  45
   His Gly Glu Asp Leu Lys Glu Ala Val Gln Glu Cys Tyr Glu Leu Ser
       50                  55                  60
   Ala Glu Tyr Glu Gly Lys His Asp Pro Lys Lys Leu Glu Glu Leu Gly
   65                  70                  75                  80
   Ser Val Leu Thr Ser Leu Asp Pro Gly Asp Ser Ile Val Ile Ala Lys
                   85                  90                  95
   Ala Phe Ser His Met Leu Asn Leu Ala Asn Leu Ala Glu Glu Val Gln
               100                 105                 110
   Ile Ala Tyr Arg Arg Arg Ile Lys Leu Lys Arg Gly Asp Phe Ala Asp
           115                 120                 125
   Glu Ala Asn Ala Thr Thr Glu Ser Asp Ile Glu Glu Thr Phe Lys Lys
       130                 135                 140
   Leu Val Leu Lys Leu Asn Lys Ser Pro Glu Glu Val Phe Asp Ala Leu
   145                 150                 155                 160
   Lys Asn Gln Thr Val Asp Leu Val Leu Thr Ala His Pro Thr Gln Ser
                   165                 170                 175
   Val Arg Arg Ser Leu Leu Gln Lys His Gly Arg Ile Arg Asn Cys Leu
               180                 185                 190
   Ala Gln Leu Tyr Ala Lys Asp Ile Thr Pro Asp Asp Lys Gln Glu Leu
           195                 200                 205
   Asp Glu Ala Leu His Arg Glu Ile Gln Ala Ala Phe Arg Thr Asp Glu
       210                 215                 220
   Ile Arg Arg Thr Pro Pro Thr Pro Gln Asp Glu Met Arg Ala Gly Met
   225                 230                 235                 240
   Ser Tyr Phe His Glu Thr Ile Trp Lys Gly Val Pro Lys Phe Leu Arg
                   245                 250                 255
   Arg Val Asp Thr Ala Leu Lys Asn Ile Gly Ile Asn Glu Arg Val Pro
               260                 265                 270
   Tyr Asn Ala Pro Leu Ile Gln Phe Ser Ser Trp Met Gly Gly Asp Arg
           275                 280                 285
   Asp Gly Lys His Pro Arg Val Thr Pro Glu Val Thr Arg Asp Val Cys
       290                 295                 300
   Leu Leu Ala Arg Met Met Ala Ser Asn Met Tyr Phe Ser Gln Ile Glu
   305                 310                 315                 320
   Asp Leu Met Phe Glu Met Ser Met Trp Arg Cys Asn Ser Glu Leu Arg
                   325                 330                 335
   Val Arg Ala Glu Glu Leu Tyr Arg Thr Ala Arg Arg Asp Val Lys His
               340                 345                 350
   Tyr Ile Glu Phe Trp Lys Gln Val Pro Pro Thr Glu Pro Tyr Arg Val
           355                 360                 365
   Ile Leu Gly Asp Val Arg Asp Lys Leu Tyr Asn Thr Arg Glu Arg Ser
       370                 375                 380
   Arg His Leu Leu Ala His Gly Ile Ser Asp Ile Pro Glu Glu Ala Val
   385                 390                 395                 400
   Tyr Thr Asn Val Glu Gln Phe Leu Glu Pro Leu Glu Leu Cys Tyr Arg
                   405                 410                 415
   Ser Leu Cys Asp Cys Gly Asp Arg Val Ile Ala Asp Gly Ser Leu Leu
               420                 425                 430
   Asp Phe Leu Arg Gln Val Ser Thr Phe Gly Leu Ser Leu Val Lys Leu
           435                 440                 445
   Asp Ile Arg Gln Glu Ser Asp Arg His Thr Asp Val Leu Asp Ala Ile
       450                 455                 460
   Thr Gln His Leu Glu Ile Gly Ser Tyr Arg Glu Trp Ser Glu Glu Lys
   465                 470                 475                 480
   Arg Gln Glu Trp Leu Leu Ala Glu Leu Ser Gly Lys Arg Pro Leu Phe
                   485                 490                 495
   Gly Ser Asp Leu Pro Lys Thr Glu Glu Val Lys Asp Val Leu Asp Thr
               500                 505                 510
   Phe Asn Val Leu Ala Glu Leu Pro Ser Asp Cys Phe Gly Ala Tyr Ile
           515                 520                 525
   Ile Ser Met Ala Thr Ser Pro Ser Asp Val Leu Ala Val Glu Leu Leu
       530                 535                 540
   Gln Arg Glu Cys His Val Lys His Pro Leu Arg Val Val Pro Leu Phe
   545                 550                 555                 560
   Glu Lys Leu Ala Asp Leu Glu Ala Ala Pro Ala Ala Met Ala Arg Leu
                   565                 570                 575
   Phe Ser Ile Asp Trp Tyr Arg Asn Arg Ile Asp Gly Lys Gln Glu Val
               580                 585                 590
   Met Ile Gly Tyr Ser Asp Ser Gly Lys Asp Ala Gly Arg Phe Ser Ala
           595                 600                 605
   Ala Trp Gln Leu Tyr Lys Ala Gln Glu Glu Ile Ile Lys Val Ala Lys
       610                 615                 620
   Glu Phe Gly Val Lys Leu Val Ile Phe His Gly Arg Gly Gly Thr Val
   625                 630                 635                 640
   Gly Arg Gly Gly Gly Pro Thr His Leu Ala Ile Leu Ser Gln Pro Pro
                   645                 650                 655
   Asp Thr Ile His Gly Ser Leu Arg Val Thr Val Gln Gly Glu Val Ile
               660                 665                 670
   Glu Gln Ser Phe Gly Glu Glu His Leu Cys Phe Arg Thr Leu Gln Arg
           675                 680                 685
   Phe Cys Ala Ala Thr Leu Glu His Gly Met Asn Pro Pro Ile Ser Pro
       690                 695                 700
   Arg Pro Glu Trp Arg Glu Leu Met Asp Gln Met Ala Val Val Ala Thr
   705                 710                 715                 720
   Glu Glu Tyr Arg Ser Ile Val Phe Lys Glu Pro Arg Phe Val Glu Tyr
                   725                 730                 735
   Phe Arg Leu Ala Thr Pro Glu Leu Glu Tyr Gly Arg Met Asn Ile Gly
               740                 745                 750
   Ser Arg Pro Ser Lys Arg Lys Pro Ser Gly Gly Ile Glu Ser Leu Arg
           755                 760                 765
   Ala Ile Pro Trp Ile Phe Ala Trp Thr Gln Thr Arg Phe His Leu Pro
       770                 775                 780
   Val Trp Leu Gly Phe Gly Ala Ala Phe Lys His Ala Ile Lys Lys Asp
   785                 790                 795                 800
   Ser Lys Asn Leu Gln Met Leu Gln Glu Met Tyr Lys Thr Trp Pro Phe
                   805                 810                 815
   Phe Arg Val Thr Ile Asp Leu Val Glu Met Val Phe Ala Lys Gly Asp
               820                 825                 830
   Pro Gly Ile Ala Ala Leu Asn Asp Lys Leu Leu Val Ser Glu Asp Leu
           835                 840                 845
   Trp Pro Phe Gly Glu Ser Leu Arg Ala Asn Tyr Glu Glu Thr Lys Asp
       850                 855                 860
   Tyr Leu Leu Lys Ile Ala Gly His Arg Asp Leu Leu Glu Gly Asp Pro
   865                 870                 875                 880
   Tyr Leu Lys Gln Arg Ile Arg Leu Arg Asp Ser Tyr Ile Thr Thr Leu
                   885                 890                 895
   Asn Val Cys Gln Ala Tyr Thr Leu Lys Arg Ile Arg Asp Pro Asn Tyr
               900                 905                 910
   His Val Thr Leu Arg Pro His Ile Ser Lys Glu Tyr Ala Ala Glu Pro
           915                 920                 925
   Ser Lys Pro Ala Asp Glu Leu Ile His Leu Asn Pro Thr Ser Glu Tyr
       930                 935                 940
   Ala Pro Gly Leu Glu Asp Thr Leu Ile Leu Thr Met Lys Gly Ile Ala
   945 950 955 960
   Ala Gly Met Gln Asn Thr Gly
   965
<210> 3
   <211> 33
   <212> PRT
   <213> Hordeum vulgare
<400> 3
   Pro Tyr Leu Lys Gln Arg Leu Arg Leu Arg Asp Pro Tyr Ile Thr Thr
   1 5 10 15
   Leu Asn Val Cys Gln Ala Tyr Thr Leu Lys Arg Ile Arg Asp Pro Ser
   20 25 30
   Phe
<210> 4
   <211> 33
   <212> PRT
   <213> Oryza sativa
<400> 4
   Leu Tyr Leu Lys Gln Arg Leu Arg Leu Arg Asn Ala Tyr Ile Thr Thr
   1 5 10 15
   Leu Asn Val Cys Gln Ala Tyr Thr Met Lys Arg Ile Arg Asp Pro Asp
   20 25 30
   Tyr
<210> 5
   <211> 33
   <212> **PRT**
   <213> Triticum aestivum
<400> 5
   Pro Tyr Leu Lys Gln Arg Leu Arg Leu Arg Asp Ala Tyr Ile Thr Thr
   1 5 10 15
   Met Asn Val Cys Gln Ala Tyr Thr Leu Lys Arg Ile Arg Asp Pro Asp
   20 25 30
   Tyr
<210> 6
   <211> 33
   <212> PRT
   <213> Arabidopsis thaliana
<400> 6
   Pro Tyr Leu Lys Gln Arg Leu Arg Leu Arg Asp Ser Tyr Ile Thr Thr
   1               5                   10                  15
   Leu Asn Val Cys Gln Ala Tyr Thr Leu Lys Arg Ile Arg Asp Ala Asn
               20                  25                  30
   Tyr
<210> 7
   <211> 33
   <212> PRT
   <213> Flaveria pringlei
<400> 7
   Pro Tyr Leu Lys Gln Arg Ile Arg Leu Arg Asp Ser Tyr Ile Thr Thr
   1               5                   10                  15
   Leu Asn Val Cys Gln Ala Tyr Thr Leu Lys Arg Ile Arg Asp Pro Asn
               20                  25                  30
   Tyr
<210> 8
   <211> 33
   <212> **PRT**
   <213> Flaveria trinervia
<400> 8
   Pro Tyr Leu Lys Gln Gly Ile Arg Leu Arg Asp Pro Tyr Ile Thr Thr
   1               5                   10                  15
   Leu Asn Val Cys Gln Ala Tyr Thr Leu Lys Arg Ile Arg Asp Pro Asn
               20                  25                  30
   Tyr
<210> 9
   <211> 33
   <212> PRT
   <213> Flaveria australasica
<400> 9
   Pro Tyr Leu Lys Gln Gly Ile Arg Leu Arg Asp Pro Tyr Ile Thr Thr
   1 5 10 15
   Leu Asn Val Cys Gln Ala Tyr Thr Leu Lys Arg Ile Arg Asp Pro Asn
   20 25 30
   Tyr
<210> 10
   <211> 33
   <212> PRT
   <213> Zea mays
<400> 10
   Pro Phe Leu Lys Gln Gly Leu Val Leu Arg Asn Pro Tyr Ile Thr Thr
   1 5 10 15
   Leu Asn Val Phe Gln Ala Tyr Thr Leu Lys Arg Ile Arg Asp Pro Asn
   20 25 30
   Phe
<210> 11
   <211> 33
   <212> PRT
   <213> Sorghum bicolor
<400> 11
   Pro Tyr Leu Lys Gln Gly Leu Arg Leu Arg Asn Pro Tyr Ile Thr Thr
   1 5 10 15
   Leu Asn Val Phe Gln Ala Tyr Thr Leu Lys Arg Ile Arg Asp Pro Ser
   20 25 30
   Phe
<210> 12
   <211> 33
   <212> PRT
   <213> Saccharum spontaneum
<400> 12
   Pro Tyr Leu Lys Gln Gly Leu Arg Leu Arg Asn Pro Tyr Ile Thr Thr
   1               5                   10                  15
   Leu Asn Val Leu Gln Ala Tyr Thr Leu Lys Arg Ile Arg Asp Pro Ser
               20                  25                  30
   Phe
<210> 13
   <211> 33
   <212> PRT
   <213> Saccharum officinarum
<400> 13
   Pro Tyr Leu Lys Gln Gly Leu Arg Leu Arg Asn Pro Tyr Ile Thr Thr
   1               5                   10                  15
   Leu Asn Val Leu Gln Ala Tyr Thr Leu Lys Arg Ile Arg Asp Pro Cys
               20                  25                  30
   Phe